(19) Europäisches Patentamt

European Patent Office

Office européen des brevets

(11) **EP 4 467 569 A1**

(12) **EUROPEAN PATENT APPLICATION**
published in accordance with Art. 153(4) EPC

(43) Date of publication:
27.11.2024 Bulletin 2024/48

(21) Application number: 23742894.1

(22) Date of filing: 17.01.2023

(51) International Patent Classification (IPC):
$C07K\ 16/28^{(2006.01)}$  $C07K\ 16/46^{(2006.01)}$
$C07K\ 19/00^{(2006.01)}$  $C12N\ 5/10^{(2006.01)}$
$C12N\ 15/13^{(2006.01)}$  $C12N\ 15/63^{(2006.01)}$
$A61K\ 39/395^{(2006.01)}$  $A61P\ 35/00^{(2006.01)}$
$G01N\ 33/50^{(2006.01)}$

(52) Cooperative Patent Classification (CPC):
A61K 39/395; A61P 35/00; C07K 16/00;
C07K 16/28; C07K 16/46; C07K 19/00; C12N 5/10;
C12N 15/63; G01N 33/50

(86) International application number:
PCT/CN2023/072622

(87) International publication number:
WO 2023/138579 (27.07.2023 Gazette 2023/30)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: 21.01.2022 CN 202210071861

(71) Applicant: Harbour Biomed (Shanghai) Co., Ltd
Shanghai 201203 (CN)

(72) Inventors:
• **WU, Xiaodong**
  **Shanghai 201203 (CN)**
• **WANG, Yongqiang**
  **Shanghai 201203 (CN)**

• **LV, Xiaocheng**
  **Shanghai 201203 (CN)**
• **CHEN, Fei**
  **Shanghai 201203 (CN)**
• **LI, Xuemei**
  **Shanghai 201203 (CN)**
• **ZHAO, Jiuqiao**
  **Shanghai 201203 (CN)**
• **RONG, Yiping**
  **Shanghai 201203 (CN)**

(74) Representative: **Maiwald GmbH
Elisenhof
Elisenstraße 3
80335 München (DE)**

Remarks:
The complete document including Reference
Table(s) and the Sequence Listing(s) can be
downloaded from the EPO website

(54) **ANTI-B7-H7 ANTIBODY OR ANTIGEN-BINDING FRAGMENT THEREOF, AND PREPARATION METHOD THEREFOR AND USE THEREOF**

(57) Disclosed are an anti-B7-H7 antibody or an antigen-binding fragment thereof, a preparation method therefor and the use thereof. The anti-B7-H7 antibody or antigen-binding fragment thereof of the present invention has high affinity and high biological activity for B7-H7, and can effectively block the binding of B7-H7 to a receptor CD28H thereof and the binding of B7-H7 to a receptor KIR3DL3 thereof; furthermore, the anti-B7-H7 antibody or antigen-binding fragment thereof only specifically binds to B7-H7 without cross-reacting with other member proteins of the B7 family, and has good stability; in addition, the anti-B7-H7 antibody or antigen-binding fragment thereof shows strong *in vivo* anti-tumor activity.

EP 4 467 569 A1

**Description**

The present international patent application claims the right of priority for the Chinese patent application 202210071861.3 filed on January 21, 2022, the content of which is incorporated herein by reference in its entirety.

Technical Field

The present invention relates to the field of biomedicine, in particular to an anti-B7-H7 antibody or an antigen-binding fragment thereof, a preparation method therefor and the use thereof.

Background Art

Immune checkpoint molecules are often highly expressed in the tumor microenvironment, which promote tumors to evade the attack from the immune system by inhibiting T cell activation and inducing T cell exhaustion. Inhibition of Immune checkpoints is the most effective and cutting edge treatment among current anti-tumor immunotherapies. For example, immune checkpoint inhibitors against PD1, PD-L1 and CTLA-4 have shown strong anti-tumor activity clinically. Thus the 2018 Nobel Prize in Physiology or Medicine was awarded to two scientists who discovered the negative immune regulation of PD1 and CTLA-4 and made outstanding contributions to the treatment of cancers.

Although treatment with anti-PD1, PD-L1 and CTLA-4 has achieved good efficacy clinically, the response rate of patients is not high, with most of the patients being resistant to the treatment of these targets, or the efficacy is not obvious. It has been proved that the tumor microenvironment is a very complex comprehensive mechanism. A variety of immunosuppressive molecules, suppressor cells and special tumor tissue structures together block the occurrence of normal immune responses. It is conceivable that a single blocking of a certain immune checkpoint is often difficult to achieve good efficacy. In addition to these targets PD1, PD-L1 and CTLA-4, other immune checkpoints such as Tim3, Lag3, TIGIT and other molecules are also highly expressed in the tumor microenvironment. Current studies on these immune checkpoints have shown that inhibition of these targets, or same in combination with anti-PD1/PD-L1 antibodies, can further enhance the anti-tumor efficacy.

The B7 family is an important family of immune system co-stimulatory/inhibitory molecules. There are currently 10 molecules in the B7 family, namely CD80 (B7.1), CD86 (B7.2), B7H1 (PD-L1/CD274), B7-DC (PD-L2/CD273), B7H2 (ICOSL), B7H3 (CD276), B7H4 (B7S1/B7x/Vtcn1), B7H5 (VISTA), B7H6 and B7-H7 (HHLA2). Multiple members of the B7 family or their receptors have been shown to be immune checkpoints, such as PD-L1/PD1, CTLA4, B7H4 and VISTA.

B7-H7 is currently one of the latest B7 family molecules, and unlike other molecules of this family, B7-H7 has 3 extracellular Ig domains. The in-vitro function of B7-H7 is somewhat controversial and may be related to the different receptors on which it acts. One of these receptors is CD28H, and it has been reported that B7-H7 acts on CD28H to promote the function of T cells or NK cells (Nat Commun. 2013; 4: 2043; Cancer Immunol Res. 2019; 7 (6): 939-951.). Additionally, it has been reported that B7-H7 can act on KIR3DL3, thereby inhibiting the function of T cells (Cancer Immunol Res. 2020 Nov 23; canimm.0315.2020.).

In normal tissues, B7-H7 is mainly highly expressed in tissues such as the gastrointestinal tract and bile duct. Studies of patient samples have found that the B7-H7 molecule is highly expressed in many tumor types, such as breast cancer, lung cancer, bowel cancer and pancreatic cancer, wherein its expression abundance is often negatively correlated with prognosis and survival (Clin Cancer Res. 2015 May 15; 21 (10): 2359-2366; Onko Targets Ther. 2018; 11: 1563-1570). Therefore, B7-H7 acts as an immune checkpoint *in vivo,* inhibiting immune responses. Blocking the action of B7-H7 by preparing neutralizing antibodies to reactivate the immune system is a promising means to treat tumors with high expression of B7-H7.

At present, there are few studies on B7-H7, or all of them are under preclinical development. Therefore, there is an urgent need for the development of human anti-B7-H7 antibodies having high affinity and high biological activity and showing strong *in vivo* anti-tumor activity.

Summary of the Invention

The technical problem to be solved by the present invention is to overcome the defect of lacking anti-B7-H7 antibodies in the prior art, and an anti-B7-H7 antibody or an antigen-binding fragment thereof, a preparation method therefor and the use thereof are provided.

In order to solve the above-mentioned technical problem, a first aspect of the present invention provides an anti-B7-H7 antibody or an antigen-binding fragment thereof comprising a light chain variable region (VL) and/or a heavy chain variable region (VH); wherein

the VL comprises complementarity determining regions (CDRs) as follows or mutations thereof: LCDR1 set forth in an amino acid sequence of SEQ ID NO: 82, LCDR2 set forth in any one of amino acid sequences of SEQ ID NOs: 94-100, and/or LCDR3 set forth in any one of amino acid sequences of SEQ ID NOs: 111, 112, 114-116, 120 and 121; the VH comprises CDRs as follows or mutations thereof: HCDR1 set forth in an amino acid sequence of SEQ ID NO: 7, HCDR2 set forth in an amino acid sequence of SEQ ID NO: 34 or 36, and/or HCDR3 set forth in any one of amino acid sequences of SEQ ID NOs: 57-68; wherein the mutation is 3, 2 or 1 amino acid insertion, deletion or substitution in the amino acid sequence of the CDR.

In the present application, in the sentence similar to the "with 3, 2 or 1 amino acid insertion, deletion or substitution", the "amino acid mutation" refers to amino acid mutations in the sequence of a variant comparing to an original amino acid sequence, including amino acid insertion, deletion or substitutions occurring on the basis of the original amino acid sequence. An exemplary explanation is that the mutation of CDRs can comprise 3, 2 or 1 amino acid mutation, and the same or a different number of amino acid residues can optionally be selected for mutation between these CDRs, for example, it can be 1 amino acid mutation for CDR1, but no amino acid mutation for CDR2 and CDR3.

In the present application, the mutations may include mutations as currently well known to those skilled in the art, such as some mutations that may be performed on antibodies during the production or use of the antibodies, such as mutations at potential sites, in particular the sites of post-translational modifications (PTMs) in the CDR region, including mutations at sites such as aggregation sites of antibodies, asparagine deamidation sensitive sites (NG, NS, NH, etc.), aspartate isomerization sensitive sites (DG, DP), N-glycosylation sensitive sites (N-{P}S/T), and oxidation sensitive sites.

Preferably, the mutation in LCDR1 is 3, 2 or 1 amino acid substitution of S5G, I6V/F, S7N, S8N/Y, W9N/Y and L10F in the amino acid sequence set forth in SEQ ID NO: 82; the amino acid sequence having the mutation in the LCDR1 is preferably set forth in any one of SEQ ID NOs: 81 and 83-88.

Preferably, the mutation in LCDR3 is 1 amino acid substitution of N4Q, S5A and Y8V in the amino acid sequence set forth in SEQ ID NO: 112, or the mutation in LCDR3 is 2 or 1 amino acid substitution of Q1H, N4Y, N5I/K and L8Y in the amino acid sequence set forth in SEQ ID NO: 115, or the mutation in LCDR3 is 1 amino acid substitution of N4Q and S5A in the amino acid sequence set forth in SEQ ID NO: 116; the amino acid sequence having the mutation in the LCDR3 is preferably set forth in any one of SEQ ID NOs: 113, 117-119 and 122-125.

Preferably, the mutation in HCDR1 is 2 or 1 amino acid substitution of F2G and D6R/N/S/Y/T in the amino acid sequence set forth in SEQ ID NO: 7; the amino acid sequence having the mutation in the HCDR1 is preferably set forth in any one of SEQ ID NOs: 8-13.

Preferably, the mutation in HCDR2 is 2 or 1 amino acid substitution of Y2F, D3N/E, G4E/A, S5N/T/R and N6E/D/K in the amino acid sequence set forth in SEQ ID NO: 36; the amino acid sequence having the mutation in the HCDR2 is preferably set forth in any one of SEQ ID NOs: 27-33, 35 and 37-40.

The S5G as described above generally refers to a mutation of amino acid S to G at position 5 of the amino acid sequence set forth in SEQ ID NO: 82, and other amino acid substitutions such as I6V/F, S7N, S8N/Y, W9N/Y and L10F, and so forth, the meaning of which should be understood by those skilled in the art.

Preferably, the LCDR1 comprises an amino acid sequence set forth in SEQ ID NO: 87, the LCDR2 comprises an amino acid sequence set forth in SEQ ID NO: 97, and the LCDR3 comprises an amino acid sequence set forth in SEQ ID NO: 120; or the LCDR1 comprises an amino acid sequence set forth in SEQ ID NO: 82, the LCDR2 comprises an amino acid sequence set forth in SEQ ID NO: 94, and the LCDR3 comprises an amino acid sequence set forth in SEQ ID NO: 112; or the LCDR1 comprises an amino acid sequence set forth in SEQ ID NO: 82, the LCDR2 comprises an amino acid sequence set forth in SEQ ID NO: 94, and the LCDR3 comprises an amino acid sequence set forth in SEQ ID NO: 122; or the LCDR1 comprises an amino acid sequence set forth in SEQ ID NO: 82, the LCDR2 comprises an amino acid sequence set forth in SEQ ID NO: 94, and the LCDR3 comprises an amino acid sequence set forth in SEQ ID NO: 123; or the LCDR1 comprises an amino acid sequence set forth in SEQ ID NO: 85, the LCDR2 comprises an amino acid sequence set forth in SEQ ID NO: 97, and the LCDR3 comprises an amino acid sequence set forth in SEQ ID NO: 116; or the LCDR1 comprises an amino acid sequence set forth in SEQ ID NO: 85, the LCDR2 comprises an amino acid sequence set forth in SEQ ID NO: 97, and the LCDR3 comprises an amino acid sequence set forth in SEQ ID NO: 124; or the LCDR1 comprises an amino acid sequence set forth in SEQ ID NO: 85, the LCDR2 comprises an amino acid sequence set forth in SEQ ID NO: 97, and the LCDR3 comprises an amino acid sequence set forth in SEQ ID NO: 125; or the LCDR 1 comprises an amino acid sequence set forth in SEQ ID NO: 81, the LCDR2 comprises an amino acid sequence set forth in SEQ ID NO: 94, and the LCDR3 comprises an amino acid sequence set forth in SEQ ID NO: 111; or the LCDR1 comprises an amino acid sequence set forth in SEQ ID NO: 82, the LCDR2 comprises an amino acid sequence set forth in SEQ ID NO: 94, and the LCDR3 comprises an amino acid sequence set forth in SEQ ID NO: 111; or the LCDR1 comprises an amino acid sequence set forth in SEQ ID NO: 82, the LCDR2 comprises an amino acid sequence set forth in SEQ ID NO: 94, and the LCDR3 comprises an amino acid sequence set forth in SEQ ID NO: 117; or the LCDR1 comprises an amino acid sequence set forth in SEQ ID NO: 82, the LCDR2 comprises an amino acid sequence set forth in SEQ ID NO: 96, and the LCDR3 comprises an amino acid sequence set forth in SEQ ID NO: 114; or the LCDR1 comprises an amino acid sequence set forth in SEQ ID NO: 83, the LCDR2 comprises an amino acid sequence set forth in SEQ ID NO: 95, and the LCDR3 comprises an amino acid sequence set forth in SEQ ID NO: 113; or the LCDR1 comprises an amino acid sequence set forth in SEQ ID NO: 83, the LCDR2 comprises an amino acid sequence set forth in SEQ ID NO: 98, and the LCDR3 comprises an amino acid sequence set forth in SEQ ID NO: 113; or the LCDR1 comprises an amino acid sequence set forth in SEQ ID NO: 83, the LCDR2 comprises an amino acid sequence set forth in SEQ ID NO: 99, and the LCDR3 comprises an amino acid sequence set forth in SEQ ID NO: 119; or the LCDR1 comprises an amino acid sequence set forth in SEQ ID NO: 84, the LCDR2 comprises an amino acid sequence set forth in SEQ ID NO: 95, and the LCDR3 comprises an amino acid sequence set forth in SEQ ID NO: 115; or the LCDR1 comprises an amino acid sequence set forth in SEQ ID NO: 86, the LCDR2 comprises an amino acid sequence set forth in SEQ ID NO: 95, and the LCDR3 comprises an amino acid sequence set forth in SEQ ID NO: 118; or the LCDR1 comprises an amino acid sequence set forth in SEQ ID NO: 88, the LCDR2 comprises an amino acid sequence set forth in SEQ ID NO: 100, and the LCDR3 comprises an amino acid sequence set forth in SEQ ID NO: 121.

Preferably, the HCDR1 comprises an amino acid sequence set forth in SEQ ID NO: 12, the HCDR2 comprises an amino acid sequence set forth in SEQ ID NO: 34, and the HCDR3 comprises an amino acid sequence set forth in SEQ ID NO: 66; or the HCDR1 comprises an amino acid sequence set forth in SEQ ID NO: 7, the HCDR2 comprises an amino acid sequence set forth in SEQ ID NO: 28, and the HCDR3 comprises an amino acid sequence set forth in SEQ ID NO: 58; or the HCDR1 comprises an amino acid sequence set forth in SEQ ID NO: 7, the HCDR2 comprises an amino acid sequence set forth in SEQ ID NO: 37, and the HCDR3 comprises an amino acid sequence set forth in SEQ ID NO: 58; or the HCDR1 comprises an amino acid sequence set forth in SEQ ID NO: 7, the HCDR2 comprises an amino acid sequence set forth in SEQ ID NO: 38, and the HCDR3 comprises an amino acid sequence set forth in SEQ ID NO: 58; or the HCDR1 comprises an amino acid sequence set forth in SEQ ID NO: 9, the HCDR2 comprises an amino acid sequence set forth in SEQ ID NO: 27, and the HCDR3 comprises an amino acid sequence set forth in SEQ ID NO: 63; or the HCDR1 comprises an amino acid sequence set forth in SEQ ID NO: 9, the HCDR2 comprises an amino acid sequence set forth in SEQ ID NO: 39, and the HCDR3 comprises an amino acid sequence set forth in SEQ ID NO: 63; or the HCDR1 comprises an amino acid sequence set forth in SEQ ID NO: 9, the HCDR2 comprises an amino acid sequence set forth in SEQ ID NO: 40, and the HCDR3 comprises an amino acid sequence set forth in SEQ ID NO: 63; or the HCDR1 comprises an amino acid sequence set forth in SEQ ID NO: 7, the HCDR2 comprises an amino acid sequence set forth in SEQ ID NO: 27, and the HCDR3 comprises an amino acid sequence set forth in SEQ ID NO: 57; or the HCDR1 comprises an amino acid sequence set forth in SEQ ID NO: 7, the HCDR2 comprises an amino acid sequence set forth in SEQ ID NO: 28, and the HCDR3 comprises an amino acid sequence set forth in SEQ ID NO: 59; or the HCDR1 comprises an amino acid sequence set forth in SEQ ID NO: 7, the HCDR2 comprises an amino acid sequence set forth in SEQ ID NO: 36, and the HCDR3 comprises an amino acid sequence set forth in SEQ ID NO: 68; or the HCDR1 comprises an amino acid sequence set forth in SEQ ID NO: 8, the HCDR2 comprises an amino acid sequence set forth in SEQ ID NO: 29, and the HCDR3 comprises an amino acid sequence set forth in SEQ ID NO: 60; or the HCDR1 comprises an amino acid sequence set forth in SEQ ID NO: 8, the HCDR2 comprises an amino acid sequence set forth in SEQ ID NO: 30, and the HCDR3 comprises an amino acid sequence set forth in SEQ ID NO: 62; or the HCDR1 comprises an amino acid sequence set forth in SEQ ID NO: 8, the HCDR2 comprises an amino acid sequence set forth in SEQ ID NO: 32, and the HCDR3 comprises an amino acid sequence set forth in SEQ ID NO: 62; or the HCDR1 comprises an amino acid sequence set forth in SEQ ID NO: 9, the HCDR2 comprises an amino acid sequence set forth in SEQ ID NO: 27, and the HCDR3 comprises an amino acid sequence set forth in SEQ ID NO: 61; or the HCDR1 comprises an amino acid sequence set forth in SEQ ID NO: 9, the HCDR2 comprises an amino acid sequence set forth in SEQ ID NO: 31, and the HCDR3 comprises an amino acid sequence set forth in SEQ ID NO: 64; or the HCDR1 comprises an amino acid sequence set forth in SEQ ID NO: 10, the HCDR2 comprises an amino acid sequence set forth in SEQ ID NO: 29, and the HCDR3 comprises an amino acid sequence set forth in SEQ ID NO: 62; or the HCDR1 comprises an amino acid sequence set forth in SEQ ID NO: 11, the HCDR2 comprises an amino acid sequence set forth in SEQ ID NO: 33, and the HCDR3 comprises an amino acid sequence set forth in SEQ ID NO: 65; or the HCDR1 comprises an amino acid sequence set forth in SEQ ID NO: 13, the HCDR2 comprises an amino acid sequence set forth in SEQ ID NO: 35, and the HCDR3 comprises an amino acid sequence set forth in SEQ ID NO: 67.

In a certain preferred embodiment, in the anti-B7-H7 antibody or the antigen-binding fragment thereof, the VL comprises LCDR1, LCDR2 and LCDR3 having amino acid sequences set forth in SEQ ID NOs: 87, 97 and 120, respectively, and the VH comprises HCDR1, HCDR2 and HCDR3 having amino acid sequences set forth in SEQ ID NOs: 12, 34 and 66, respectively.

In a certain preferred embodiment, in the anti-B7-H7 antibody or the antigen-binding fragment thereof, the VL comprises LCDR1, LCDR2 and LCDR3 having amino acid sequences set forth in SEQ ID NOs: 82, 94 and 112, respectively, and the VH comprises HCDR1, HCDR2 and HCDR3 having amino acid sequences set forth in SEQ ID NOs: 7, 28 and 58, respectively.

In a certain preferred embodiment, in the anti-B7-H7 antibody or the antigen-binding fragment thereof, the VL comprises LCDR1, LCDR2 and LCDR3 having amino acid sequences set forth in SEQ ID NOs: 82, 94 and 112, respectively, and the VH comprises HCDR1, HCDR2 and HCDR3 having amino acid sequences set forth in SEQ ID NOs: 7, 37 and 58, respectively.

In a certain preferred embodiment, in the anti-B7-H7 antibody or the antigen-binding fragment thereof, the VL comprises LCDR1, LCDR2 and LCDR3 having amino acid sequences set forth in SEQ ID NOs: 82, 94 and 122, respectively, and the VH comprises HCDR1, HCDR2 and HCDR3 having amino acid sequences set forth in SEQ ID NOs: 7, 37 and 58, respectively.

In a certain preferred embodiment, in the anti-B7-H7 antibody or the antigen-binding fragment thereof, the VL comprises LCDR1, LCDR2 and LCDR3 having amino acid sequences set forth in SEQ ID NOs: 82, 94 and 123, respectively, and the VH comprises HCDR1, HCDR2 and HCDR3 having amino acid sequences set forth in SEQ ID NOs: 7, 37 and 58, respectively.

In a certain preferred embodiment, in the anti-B7-H7 antibody or the antigen-binding fragment thereof, the VL comprises LCDR1, LCDR2 and LCDR3 having amino acid sequences set forth in SEQ ID NOs: 82, 94 and 112, respectively, and the VH comprises HCDR1, HCDR2 and HCDR3 having amino acid sequences set forth in SEQ ID NOs: 7, 38 and 58, respectively.

In a certain preferred embodiment, in the anti-B7-H7 antibody or the antigen-binding fragment thereof, the VL comprises LCDR1, LCDR2 and LCDR3 having amino acid sequences set forth in SEQ ID NOs: 82, 94 and 122, respectively, and the VH comprises HCDR1, HCDR2 and HCDR3 having amino acid sequences set forth in SEQ ID NOs: 7, 38 and 58, respectively.

In a certain preferred embodiment, in the anti-B7-H7 antibody or the antigen-binding fragment thereof, the VL comprises LCDR1, LCDR2 and LCDR3 having amino acid sequences set forth in SEQ ID NOs: 82, 94 and 123, respectively, and the VH comprises HCDR1, HCDR2 and HCDR3 having amino acid sequences set forth in SEQ ID NOs: 7, 38 and 58, respectively.

In a certain preferred embodiment, in the anti-B7-H7 antibody or the antigen-binding fragment thereof, the VL comprises LCDR1, LCDR2 and LCDR3 having amino acid sequences set forth in SEQ ID NOs: 82, 94 and 122, respectively, and the VH comprises HCDR1, HCDR2 and HCDR3 having amino acid sequences set forth in SEQ ID NOs: 7, 28 and 58, respectively.

In a certain preferred embodiment, in the anti-B7-H7 antibody or the antigen-binding fragment thereof, the VL comprises LCDR1, LCDR2 and LCDR3 having amino acid sequences set forth in SEQ ID NOs: 82, 94 and 123, respectively, and the VH comprises HCDR1, HCDR2 and HCDR3 having amino acid sequences set forth in SEQ ID NOs: 7, 28 and 58, respectively.

In a certain preferred embodiment, in the anti-B7-H7 antibody or the antigen-binding fragment thereof, the VL comprises LCDR1, LCDR2 and LCDR3 having amino acid sequences set forth in SEQ ID NOs: 85, 97 and 116, respectively, and the VH comprises HCDR1, HCDR2 and HCDR3 having amino acid sequences set forth in SEQ ID NOs: 9, 27 and 63, respectively.

In a certain preferred embodiment, in the anti-B7-H7 antibody or the antigen-binding fragment thereof, the VL comprises LCDR1, LCDR2 and LCDR3 having amino acid sequences set forth in SEQ ID NOs: 85, 97 and 116, respectively, and the VH comprises HCDR1, HCDR2 and HCDR3 having amino acid sequences set forth in SEQ ID NOs: 9, 39 and 63, respectively.

In a certain preferred embodiment, in the anti-B7-H7 antibody or the antigen-binding fragment thereof, the VL comprises LCDR1, LCDR2 and LCDR3 having amino acid sequences set forth in SEQ ID NOs: 85, 97 and 124, respectively, and the VH comprises HCDR1, HCDR2 and HCDR3 having amino acid sequences set forth in SEQ ID NOs: 9, 39 and 63, respectively.

In a certain preferred embodiment, in the anti-B7-H7 antibody or the antigen-binding fragment thereof, the VL comprises LCDR1, LCDR2 and LCDR3 having amino acid sequences set forth in SEQ ID NOs: 85, 97 and 125, respectively, and the VH comprises HCDR1, HCDR2 and HCDR3 having amino acid sequences set forth in SEQ ID NOs: 9, 39 and 63, respectively.

In a certain preferred embodiment, in the anti-B7-H7 antibody or the antigen-binding fragment thereof, the VL comprises LCDR1, LCDR2 and LCDR3 having amino acid sequences set forth in SEQ ID NOs: 85, 97 and 116, respectively, and the VH comprises HCDR1, HCDR2 and HCDR3 having amino acid sequences set forth in SEQ ID NOs: 9, 40 and 63, respectively.

In a certain preferred embodiment, in the anti-B7-H7 antibody or the antigen-binding fragment thereof, the VL comprises LCDR1, LCDR2 and LCDR3 having amino acid sequences set forth in SEQ ID NOs: 85, 97 and 124, respectively, and the VH comprises HCDR1, HCDR2 and HCDR3 having amino acid sequences set forth in SEQ ID NOs: 9, 40 and 63, respectively.

In a certain preferred embodiment, in the anti-B7-H7 antibody or the antigen-binding fragment thereof, the VL comprises LCDR1, LCDR2 and LCDR3 having amino acid sequences set forth in SEQ ID NOs: 85, 97 and 125, respectively, and the VH comprises HCDR1, HCDR2 and HCDR3 having amino acid sequences set forth in SEQ ID NOs: 9, 40 and 63, respectively.

In a certain preferred embodiment, in the anti-B7-H7 antibody or the antigen-binding fragment thereof, the VL comprises LCDR1, LCDR2 and LCDR3 having amino acid sequences set forth in SEQ ID NOs: 85, 97 and 124, respectively, and the VH comprises HCDR1, HCDR2 and HCDR3 having amino acid sequences set forth in SEQ ID NOs: 9, 27 and 63, respectively.

In a certain preferred embodiment, in the anti-B7-H7 antibody or the antigen-binding fragment thereof, the VL comprises LCDR1, LCDR2 and LCDR3 having amino acid sequences set forth in SEQ ID NOs: 85, 97 and 125, respectively, and the VH comprises HCDR1, HCDR2 and HCDR3 having amino acid sequences set forth in SEQ ID NOs: 9, 27 and 63, respectively.

In a certain preferred embodiment, in the anti-B7-H7 antibody or the antigen-binding fragment thereof, the VL comprises LCDR1, LCDR2 and LCDR3 having amino acid sequences set forth in SEQ ID NOs: 81, 94 and 111, respectively, and the VH comprises HCDR1, HCDR2 and HCDR3 having amino acid sequences set forth in SEQ ID NOs: 7, 27 and 57, respectively.

In a certain preferred embodiment, in the anti-B7-H7 antibody or the antigen-binding fragment thereof, the VL comprises LCDR1, LCDR2 and LCDR3 having amino acid sequences set forth in SEQ ID NOs: 82, 94 and 112, respectively, and the VH comprises HCDR1, HCDR2 and HCDR3 having amino acid sequences set forth in SEQ ID NOs: 7, 28 and 59, respectively.

In a certain preferred embodiment, in the anti-B7-H7 antibody or the antigen-binding fragment thereof, the VL comprises LCDR1, LCDR2 and LCDR3 having amino acid sequences set forth in SEQ ID NOs: 83, 95 and 113, respectively, and the VH comprises HCDR1, HCDR2 and HCDR3 having amino acid sequences set forth in SEQ ID NOs: 8, 29 and 60, respectively.

In a certain preferred embodiment, in the anti-B7-H7 antibody or the antigen-binding fragment thereof, the VL comprises LCDR1, LCDR2 and LCDR3 having amino acid sequences set forth in SEQ ID NOs: 82, 96 and 114, respectively, and the VH comprises HCDR1, HCDR2 and HCDR3 having amino acid sequences set forth in SEQ ID NOs: 9, 27 and 61, respectively.

In a certain preferred embodiment, in the anti-B7-H7 antibody or the antigen-binding fragment thereof, the VL comprises LCDR1, LCDR2 and LCDR3 having amino acid sequences set forth in SEQ ID NOs: 84, 95 and 115, respectively, and the VH comprises HCDR1, HCDR2 and HCDR3 having amino acid sequences set forth in SEQ ID NOs: 10, 29 and 62, respectively.

In a certain preferred embodiment, in the anti-B7-H7 antibody or the antigen-binding fragment thereof, the VL comprises LCDR1, LCDR2 and LCDR3 having amino acid sequences set forth in SEQ ID NOs: 83, 98 and 113, respectively, and the VH comprises HCDR1, HCDR2 and HCDR3 having amino acid sequences set forth in SEQ ID NOs: 8, 30 and 62, respectively.

In a certain preferred embodiment, in the anti-B7-H7 antibody or the antigen-binding fragment thereof, the VL comprises LCDR1, LCDR2 and LCDR3 having amino acid sequences set forth in SEQ ID NOs: 82, 94 and 117, respectively, and the VH comprises HCDR1, HCDR2 and HCDR3 having amino acid sequences set forth in SEQ ID NOs: 9, 31 and 64, respectively.

In a certain preferred embodiment, in the anti-B7-H7 antibody or the antigen-binding fragment thereof, the VL comprises LCDR1, LCDR2 and LCDR3 having amino acid sequences set forth in SEQ ID NOs: 86, 95 and 118, respectively, and the VH comprises HCDR1, HCDR2 and HCDR3 having amino acid sequences set forth in SEQ ID NOs: 8, 32 and 62, respectively.

In a certain preferred embodiment, in the anti-B7-H7 antibody or the antigen-binding fragment thereof, the VL comprises LCDR1, LCDR2 and LCDR3 having amino acid sequences set forth in SEQ ID NOs: 83, 99 and 119, respectively, and the VH comprises HCDR1, HCDR2 and HCDR3 having amino acid sequences set forth in SEQ ID NOs: 11, 33 and 65, respectively.

In a certain preferred embodiment, in the anti-B7-H7 antibody or the antigen-binding fragment thereof, the VL comprises LCDR1, LCDR2 and LCDR3 having amino acid sequences set forth in SEQ ID NOs: 88, 100 and 121, respectively, and the VH comprises HCDR1, HCDR2 and HCDR3 having amino acid sequences set forth in SEQ ID NOs: 13, 35 and 67, respectively.

In a certain preferred embodiment, in the anti-B7-H7 antibody or the antigen-binding fragment thereof, the VL comprises LCDR1, LCDR2 and LCDR3 having amino acid sequences set forth in SEQ ID NOs: 82, 94 and 111, respectively, and the VH comprises HCDR1, HCDR2 and HCDR3 having amino acid sequences set forth in SEQ ID NOs: 7, 36 and 68, respectively.

In the present invention, the amino acid sequences of the CDRs listed above are shown according to the rules of Chothia definition. However, it is well known to those skilled in the art that the CDRs of an antibody can be defined in the art by a variety of methods, such as the rules of Kabat definition based on sequence variability (see, Kabat et al., Sequences of Proteins of Immunological Interest, fifth edition, National Institutes of Health, Bethesda, Maryland (1991)), and the rules of Chothia definition based on the location of a structural loop region (see JMol Biol 273: 927-48, 1997). In the technical solutions of the present invention, amino acid residues in variable domain sequences can also be determined according to the rules of Combined definition that incorporates both Kabat definition and Chothia definition. The rules of Combined definition refers to the combination of the ranges of Kabat definition and Chothia definition, based on which a larger scope is taken, see Table 1-6 in the examples for details. It should be understood by those skilled in the art that unless otherwise specified, the terms "CDR" and "complementarity determining region" of a given antibody or region thereof (for example, a variable region) should be understood to encompass the complementarity determining region as defined by any of the above-mentioned known schemes as described in the present invention. Although the scope of protection as claimed in the present invention is based on the sequences shown according to the rules of Chothia definition, corresponding amino acid sequences according to the rules of other CDR definitions shall also fall within the scope of protection of the present invention.

Preferably, the light chain variable region (VL) further comprises a light chain variable region framework region (VL FWR), the VL FWR is a light chain variable region framework region of a human antibody, and the gene encoding the VL FWR is preferably derived from a germline V gene IGKV1-5*03, IGKV3-15*01, IGKV1-27*01 or IGKV1-9*01. More preferably, the VL FWR comprises VL FWR1 having an amino acid sequence set forth in any one of sequences of SEQ ID NOs: 74-79 or mutations thereof, VL FWR2 having an amino acid sequence set forth in any one of sequences of SEQ ID NOs: 90-92 or mutations thereof, VL FWR3 having an amino acid sequence set forth in any one of sequences of SEQ ID NOs: 102-109 or mutations thereof, and VL FWR4 having an amino acid sequence set forth in any one of sequences of SEQ ID NOs: 127-130 or mutations thereof. The mutation is 3, 2 or 1 amino acid insertion, deletion, substitution or duplication in the amino acid sequence of the VL FWR.

Preferably, the heavy chain variable region (VH) further comprises a heavy chain variable region framework region (VH FWR), the VH FWR is a heavy chain variable region framework region of a human antibody, and the gene encoding the VH FWR is preferably derived from a germline V gene IGHV3-33*01, IGHV3-33*06 or IGHV1-69*01. More preferably, the VH FWR comprises VH FWR1 having an amino acid sequence set forth in any one of sequences of SEQ ID NOs: 2-5 or mutations thereof, VH FWR2 having an amino acid sequence set forth in any one of sequences of SEQ ID NOs: 15-25 or mutations thereof, VH FWR3 having an amino acid sequence set forth in any one of sequences of SEQ ID NOs: 42-55 or mutations thereof, and VH FWR4 having an amino acid sequence set forth in any one of sequences of SEQ ID NOs: 70-72 or mutations thereof. The mutation is 3, 2 or 1 amino acid insertion, deletion, substitution or duplication in the amino acid sequence of the VH FWR.

In a certain embodiment of the present invention, the VL comprises an amino acid sequence set forth in any one of SEQ ID NOs: 151-167 or mutations thereof. In a certain embodiment of the present invention, the VH comprises an amino acid sequence set forth in any one of SEQ ID NOs: 132-149 or mutations thereof.

In a certain preferred embodiment, the VL comprises an amino acid sequence set forth in SEQ ID NO: 161, and the VH comprises an amino acid sequence set forth in SEQ ID NO: 143. In a certain preferred embodiment, the VL comprises an amino acid sequence set forth in SEQ ID NO: 152, and the VH comprises an amino acid sequence set forth in SEQ ID NO: 133. In a certain preferred embodiment, the VL comprises an amino acid sequence set forth in SEQ ID NO: 152, and the VH comprises an amino acid sequence set forth in SEQ ID NO: 146. In a certain preferred embodiment, the VL comprises an amino acid sequence set forth in SEQ ID NO: 164, and the VH comprises an amino acid sequence set forth in SEQ ID NO: 146. In a certain preferred embodiment, the VL comprises an amino acid sequence set forth in SEQ ID NO: 165, and the VH comprises an amino acid sequence set forth in SEQ ID NO: 146. In a certain preferred embodiment, the VL comprises an amino acid sequence set forth in SEQ ID NO: 152, and the VH comprises an amino acid sequence set forth in SEQ ID NO: 147. In a certain preferred embodiment, the VL comprises an amino acid sequence set forth in SEQ ID NO: 164, and the VH comprises an amino acid sequence set forth in SEQ ID NO: 147. In a certain preferred embodiment, the VL comprises an amino acid sequence set forth in SEQ ID NO: 165, and the VH comprises an amino acid sequence set forth in SEQ ID NO: 147. In a certain preferred embodiment, the VL comprises an amino acid sequence set forth in SEQ ID NO: 164, and the VH comprises an amino acid sequence set forth in SEQ ID NO: 133. In a certain preferred embodiment, the VL comprises an amino acid sequence set forth in SEQ ID NO: 165, and the VH comprises an amino acid sequence set forth in SEQ ID NO: 133. In a certain preferred embodiment, the VL comprises an amino acid sequence set forth in SEQ ID NO: 156, and the VH comprises an amino acid sequence set forth in SEQ ID NO: 138. In a certain preferred embodiment, the VL comprises an amino acid sequence set forth in SEQ ID NO: 156, and the VH comprises an amino acid sequence set forth in SEQ ID NO: 148. In a certain preferred embodiment, the VL comprises an amino acid sequence set forth in SEQ ID NO: 166, and the VH comprises an amino acid sequence set forth in SEQ ID NO: 148. In a certain preferred embodiment, the VL comprises an amino acid sequence set forth in SEQ ID NO: 167, and the VH comprises an amino acid sequence set forth in SEQ ID NO: 148. In a certain preferred embodiment, the VL comprises an amino acid sequence set forth in SEQ ID NO: 156, and the VH comprises an amino acid sequence set forth in SEQ ID NO: 149. In a certain preferred embodiment, the VL comprises an amino acid sequence set forth in SEQ ID NO: 166, and the VH comprises an amino acid sequence set forth in SEQ ID NO: 149. In a certain preferred embodiment, the VL comprises an amino acid sequence set forth in SEQ ID NO: 167, and the VH comprises an amino acid sequence set forth in SEQ ID NO: 149. In a certain preferred embodiment, the VL comprises an amino acid sequence set forth in SEQ ID NO: 166, and the VH comprises an amino acid sequence set forth in SEQ ID NO: 138. In a certain preferred embodiment, the VL comprises an amino acid sequence set forth in SEQ ID NO: 167, and the VH comprises an amino acid sequence set forth in SEQ ID NO: 138. In a certain preferred embodiment, the VL comprises an amino acid sequence set forth in SEQ ID NO: 151, and the VH comprises an amino acid sequence set forth in SEQ ID NO: 132. In a certain preferred embodiment, the VL comprises an amino acid sequence set forth in SEQ ID NO: 152, and the VH comprises an amino acid sequence set forth in SEQ ID NO: 134. In a certain preferred embodiment, the VL comprises an amino acid sequence set forth in SEQ ID NO: 153, and the VH comprises an amino acid sequence set forth in SEQ ID NO: 135. In a certain preferred embodiment, the VL comprises an amino acid sequence set forth in SEQ ID NO: 154, and the VH comprises an amino acid sequence set forth in SEQ ID NO: 136. In a certain preferred embodiment, the VL comprises an amino acid sequence set forth in SEQ ID NO: 155, and the VH comprises an amino acid sequence set forth in SEQ ID NO: 137. In a certain preferred embodiment, the VL comprises an amino acid sequence set forth in SEQ ID NO: 157, and the VH comprises an amino acid sequence set forth in SEQ ID NO: 139. In a certain preferred embodiment, the VL comprises an amino acid sequence set forth in SEQ ID NO: 158, and the VH comprises an amino acid sequence set forth in SEQ ID NO: 140. In a certain preferred embodiment, the VL comprises an amino acid sequence set forth in SEQ ID NO: 159, and the VH comprises an amino acid sequence set forth in SEQ ID NO: 141. In a certain preferred embodiment, the VL comprises an amino acid sequence set forth in SEQ ID NO: 160, and the VH comprises an amino acid sequence set forth in SEQ ID NO: 142. In a certain preferred embodiment, the VL comprises an amino acid sequence set forth in SEQ ID NO: 162, and the VH comprises an amino acid sequence set forth in SEQ ID NO: 144. In a certain preferred embodiment, the VL comprises an amino acid sequence set forth in SEQ ID NO: 163, and the VH comprises an amino acid sequence set forth in SEQ ID NO: 145. The above-mentioned mutation is one or more amino acid residue deletions, substitutions or additions occurring in the amino acid sequence of the VH and/or VL, and the amino acid sequence having the mutation has at least 85% sequence identity to the amino acid sequence of the VH and/or VL, and maintains or improves the binding of the antibody to B7-H7; the at least 85% sequence identity is preferably at least 90% sequence identity, more preferably at least 95%, 96%, 97% or 98% sequence identity, most preferably at least 99% sequence identity.

Preferably, the anti-B7-H7 antibody or the antigen-binding fragment thereof of the present invention can be a full-length antibody, Fab, Fab', F(ab')$_2$, Fv, preferably scFv, a bispecific antibody, a multispecific antibody, a heavy chain antibody or a single domain antibody, or a monoclonal antibody or a polyclonal antibody prepared from the above-mentioned antibodies.

In the present application, the "Fab fragment" consists of one light chain and the CH1 and variable region of one heavy chain. The heavy chain of a Fab molecule cannot form a disulfide bond with another heavy chain molecule. The "Fc" region contains two heavy chain fragments comprising the CH1 and CH2 domains of an antibody. The two heavy chain fragments are held together by two or more disulfide bonds and by hydrophobic interactions of the CH3 domains. The "Fab fragment" contains one light chain and a portion of a heavy chain that comprises the VH domain and the CH1 domain and the region between the CH1 and CH2 domains, whereby an interchain disulfide bond can be formed between the two heavy chains of two Fab' fragments to form a F(ab')$_2$ molecule. The "F(ab')$_2$ fragment" contains two light chains and two heavy chains that comprise a portion of the constant region between the CH1 and CH2 domains, whereby an interchain disulfide bond is formed between the two heavy chains. Therefore, the F(ab')$_2$ fragment consists of two Fab' fragments held together via a disulfide bond between the two heavy chains. The term "Fv" refers to an antibody fragment consisting of VL and VH domains of a single arm of an antibody, but lacking constant regions.

In the present application, the scFv (single chain antibody fragment) can be a conventional single chain antibody in the art, which comprises a heavy chain variable region, a light chain variable region, and a short peptide of 15 to 20 amino acids. The VL and VH domains are paired via a linker which enables them to be produced as a single polypeptide chain, thereby forming a monovalent molecule [see, e.g., Bird et al., Science 242: 423-426 (1988) and Huston et al., Proc. Natl. Acad. Sci. USA 85: 5879-5883 (1988)]. Such scFv molecules may have a general structure of NH2-VL-linker-VH-COOH or NH2-VH-linker-VL-COOH. Suitable linkers in the prior art consist of a repetitive G$_4$S amino acid sequence or a variant thereof. For example, a linker having an amino acid sequence (G$_4$S)$_4$ or (G$_4$S)$_3$ can be used, but variants thereof may also be used.

In the present application, the term "multispecific antibody" is used in its broadest sense to encompass antibodies with multi-epitope specificities. These multispecific antibodies include, but are not limited to: antibodies comprising a heavy chain variable region (VH) and a light chain variable region (VL), wherein the VH-VL unit has multi-epitope specificity; antibodies having two or more VL and VH regions, wherein each VH-VL unit binds to a different target or a different epitope of the same target; antibodies having two or more single variable regions, wherein each single variable region binds to a different target or a different epitope of the same target; and full-length antibodies, antibody fragments, bispecific antibodies (diabodies), triabodies, antibody fragments that are covalently or non-covalently linked together, etc.

In the present application, the monoclonal antibody or mAb or Ab refers to an antibody obtained from a single clonal cell strain, wherein the cell strain is not limited to eukaryotic, prokaryotic or phage clonal cell strains.

In the present application, the "heavy chain antibody" refers to an antibody comprising only one heavy chain variable region (VHH) and two conventional CH2 and CH3 regions, also known as HCAb.

The "single domain antibody", also known as "nanobody", refers to a VHH structure cloned from a heavy chain antibody, which is the smallest unit known that binds to an antigen of interest.

In a certain embodiment of the present invention, the anti-B7-H7 antibody or the antigen-binding fragment thereof is a full-length antibody comprising an antibody heavy chain constant region and an antibody light chain constant region. Preferably, the heavy chain constant region is selected from hIgG1, hIgG2, hIgG3 or hIgG4, and the light chain constant region is selected from a kappa chain or a lamda chain of a human antibody. More preferably, the heavy chain constant region is hIgG1, and the light chain constant region is a kappa chain of a human antibody.

In a certain preferred embodiment, the full-length antibody comprises a heavy chain and a light chain; wherein the heavy chain comprises an amino acid sequence set forth in any one of SEQ ID NOs: 169-190 or mutations thereof; and/or the light chain comprises an amino acid sequence set forth in any one of SEQ ID NOs: 192-208 or mutations thereof.

In a certain preferred embodiment, the full-length antibody comprises a heavy chain and a light chain; wherein the heavy chain comprises an amino acid sequence set forth in SEQ ID NO: 186, and the light chain comprises an amino acid sequence set forth in SEQ ID NO: 202. In a certain preferred embodiment, the full-length antibody comprises a heavy chain and a light chain; wherein the heavy chain comprises an amino acid sequence set forth in SEQ ID NO: 180, and the light chain comprises an amino acid sequence set forth in SEQ ID NO: 202. In a certain preferred embodiment, the full-length antibody comprises a heavy chain and a light chain; wherein the heavy chain comprises an amino acid sequence set forth in SEQ ID NO: 184, and the light chain comprises an amino acid sequence set forth in SEQ ID NO: 193. In a certain preferred embodiment, the full-length antibody comprises a heavy chain and a light chain; wherein the heavy chain comprises an amino acid sequence set forth in SEQ ID NO: 170, and the light chain comprises an amino acid sequence set forth in SEQ ID NO: 193. In a certain preferred embodiment, the full-length antibody comprises a heavy chain and a light chain; wherein the heavy chain comprises an amino acid sequence set forth in SEQ ID NO: 187, and the light chain comprises an amino acid sequence set forth in SEQ ID NO: 193. In a certain preferred embodiment, the full-length antibody comprises a heavy chain and a light chain; wherein the heavy chain comprises an amino acid sequence set forth in SEQ ID NO: 187, and the light chain comprises an amino acid sequence set forth in SEQ ID NO: 205. In a certain preferred embodiment, the full-length antibody comprises a heavy chain and a light chain; wherein the heavy chain comprises an amino acid sequence set forth in SEQ ID NO: 187, and the light chain comprises an amino acid sequence set forth in SEQ ID NO: 206. In a certain preferred embodiment, the full-length antibody comprises a heavy chain and a light chain; wherein the heavy chain comprises an amino acid sequence set forth in SEQ ID NO: 188, and the light chain comprises an amino acid sequence set forth in SEQ ID NO: 193. In a certain preferred embodiment, the full-length antibody comprises a heavy chain and a light chain; wherein the heavy chain comprises an amino acid sequence set forth in SEQ ID NO: 188, and the light chain comprises an amino acid sequence set forth in SEQ ID NO: 205. In a certain preferred embodiment, the full-length antibody comprises a heavy chain and a light chain; wherein the heavy chain comprises an amino acid sequence set forth in SEQ ID NO: 188, and the light chain comprises an amino acid sequence set forth in SEQ ID NO: 206. In a certain preferred embodiment, the full-length antibody comprises a heavy chain and a light chain; wherein the heavy chain comprises an amino acid sequence set forth in SEQ ID NO: 184, and the light chain comprises an amino acid sequence set forth in SEQ ID NO: 205. In a certain preferred embodiment, the full-length antibody comprises a heavy chain and a light chain; wherein the heavy chain comprises an amino acid sequence set forth in SEQ ID NO: 184, and the light chain comprises an amino acid sequence set forth in SEQ ID NO: 206. In a certain preferred embodiment, the full-length antibody comprises a heavy chain and a light chain; wherein the heavy chain comprises an amino acid sequence set forth in SEQ ID NO: 185, and the light chain comprises an amino acid sequence set forth in SEQ ID NO: 197. In a certain preferred embodiment, the full-length antibody comprises a heavy chain and a light chain; wherein the heavy chain comprises an amino acid sequence set forth in SEQ ID NO: 175, and the light chain comprises an amino acid sequence set forth in SEQ ID NO: 197. In a certain preferred embodiment, the full-length antibody comprises a heavy chain and a light chain; wherein the heavy chain comprises an amino acid sequence set forth in SEQ ID NO: 189, and the light chain comprises an amino acid sequence set forth in SEQ ID NO: 197. In a certain preferred embodiment, the full-length antibody comprises a heavy chain and a light chain; wherein the heavy chain comprises an amino acid sequence set forth in SEQ ID NO: 189, and the light chain comprises an amino acid sequence set forth in SEQ ID NO: 207. In a certain preferred embodiment, the full-length antibody comprises a heavy chain and a light chain; wherein the heavy chain comprises an amino acid sequence set forth in SEQ ID NO: 189, and the light chain comprises an amino acid sequence set forth in SEQ ID NO: 208. In a certain preferred embodiment, the full-length antibody comprises a heavy chain and a light chain; wherein the heavy chain comprises an amino acid sequence set forth in SEQ ID NO: 190, and the light chain comprises an amino acid sequence set forth in SEQ ID NO: 197. In a certain preferred embodiment, the full-length antibody comprises a heavy chain and a light chain; wherein the heavy chain comprises an amino acid sequence set forth in SEQ ID NO: 190, and the light chain comprises an amino acid sequence set forth in SEQ ID NO: 207. In a certain preferred embodiment, the full-length antibody comprises a heavy chain and a light chain; wherein the heavy chain comprises an amino acid sequence set forth in SEQ ID NO: 190, and the light chain comprises an amino acid sequence set forth in SEQ ID NO: 208. In a certain preferred embodiment, the full-length antibody comprises a heavy chain and a light chain; wherein the heavy chain comprises an amino acid sequence set forth in SEQ ID NO: 185, and the light chain comprises an amino acid sequence set forth in SEQ ID NO: 207. In a certain preferred embodiment, the full-length antibody comprises a heavy chain and a light chain; wherein the heavy chain comprises an amino acid sequence set forth in SEQ ID NO: 185, and the light chain comprises an amino acid sequence set forth in SEQ ID NO: 208. In a certain preferred embodiment, the full-length antibody comprises a heavy chain and a light chain; wherein the heavy chain comprises an amino acid sequence set forth in SEQ ID NO: 169, and the light chain comprises an amino acid sequence set forth in SEQ ID NO: 192. In a certain preferred embodiment, the full-length antibody comprises a heavy chain and a light chain; wherein the heavy chain comprises an amino acid sequence set forth in SEQ ID NO: 171, and the light chain comprises an amino acid sequence set forth in SEQ ID NO: 193. In a certain preferred embodiment, the full-length antibody comprises a heavy chain and a light chain; wherein the heavy chain comprises an amino acid sequence set forth in SEQ ID NO: 172, and the light chain comprises an amino acid sequence set forth in SEQ ID NO: 194. In a certain preferred embodiment, the full-length antibody comprises a heavy chain and a light chain; wherein the heavy chain comprises an amino acid sequence set forth in SEQ ID NO: 173, and the light chain comprises an amino acid sequence set forth in SEQ ID NO: 195. In a certain preferred embodiment, the full-length antibody comprises a heavy chain and a light chain; wherein the heavy chain comprises an amino acid sequence set forth in SEQ ID NO: 174, and the light chain comprises an amino acid sequence set forth in SEQ ID NO: 196. In a certain preferred embodiment, the full-length antibody comprises a heavy chain and a light chain; wherein the heavy chain comprises an amino acid sequence set forth in SEQ ID NO: 176, and the light chain comprises an amino acid sequence set forth in SEQ ID NO: 198. In a certain preferred embodiment, the full-length antibody comprises a heavy chain and a light chain; wherein the heavy chain comprises an amino acid sequence set forth in SEQ ID NO: 177, and the light chain comprises an amino acid sequence set forth in SEQ ID NO: 199. In a certain preferred embodiment, the full-length antibody comprises a heavy chain and a light chain; wherein the heavy chain comprises an amino acid sequence set forth in SEQ ID NO: 178, and the light chain comprises an amino acid sequence set forth in SEQ ID NO: 200. In a certain preferred embodiment, the full-length antibody comprises a heavy chain and a light chain; wherein the heavy chain comprises an amino acid sequence set forth in SEQ ID NO: 179, and the light chain comprises an amino acid sequence set forth in SEQ ID NO: 201. In a certain preferred embodiment, the full-length antibody comprises a heavy chain and a light chain; wherein the heavy chain comprises an amino acid sequence set forth in SEQ ID NO: 181, and the light chain comprises an amino acid sequence set forth in SEQ ID NO: 203. In a certain preferred embodiment, the full-length antibody comprises a heavy chain and a light chain; wherein the heavy chain comprises an amino acid sequence set forth in SEQ ID NO: 182, and the light chain comprises an amino acid sequence set forth in SEQ ID NO: 204. The above-mentioned mutation is one or more amino acid residue deletions, substitutions or additions occurring in the amino acid sequence of a heavy chain and/or a light chain, and the amino acid sequence having the mutation has at least 85% sequence identity to the amino acid sequence of the heavy chain and/or the light chain, and maintains or improves the binding of the antibody to B7-H7; the at least 85% sequence identity is preferably at least 90% sequence identity; more preferably at least 95% sequence identity; most preferably at least 99% sequence identity.

In order to solve the above-mentioned technical problem, a second aspect of the present invention provides an isolated nucleic acid encoding the anti-B7-H7 antibody or the antigen-binding fragment thereof according to the first aspect of the present invention.

The method for preparing the nucleic acid is a conventional preparation method in the art, which method preferably comprises the following steps: obtaining the nucleic acid molecule encoding the above-mentioned antibody by gene cloning technique, or obtaining the nucleic acid molecule encoding the above-mentioned antibody by artificial complete sequence synthesis. It is known to those skilled in the art that a substitution, deletion, alteration, insertion or addition can be appropriately introduced into a base sequence encoding the amino acid sequence of the above-mentioned antibody to provide a homolog of a polynucleotide. The homolog of the polynucleotide of the present invention can be prepared by the substitution, deletion or addition of one or more bases of the gene encoding the antibody sequence within a range of maintaining the activity of the antibody.

In order to solve the above-mentioned technical problem, a third aspect of the present invention provides a recombinant expression vector comprising the isolated nucleic acid according to the second aspect of the present invention.

The recombinant expression vector can be obtained by conventional methods in the art, namely, the recombinant expression vector is constructed by linking the nucleic acid molecule of the present application to various expression vectors. The expression vectors are various conventional vectors in the art, as long as they are capable of carrying the aforementioned nucleic acid molecules. Preferably, the expression vector comprises a eukaryotic cell expression vector and/or a prokaryotic cell expression vector.

In order to solve the above-mentioned technical problem, a fourth aspect of the present invention provides a transformant comprising the recombinant expression vector according to the third aspect of the present invention.

The method for preparing the transformant can be a conventional preparation method in the art, for example, the transformant is prepared by transforming the above-mentioned expression vector into a host cell. The host cell of the transformant is various conventional host cells in the art, as long as it meets the following requirements: in the host cell, the above-mentioned recombinant expression vector can stably replicate itself and the nucleic acid carried thereby can be efficiently expressed. Preferably, the host cell is a prokaryotic cell and/or a eukaryotic cell, the prokaryotic cell is preferably an *E. coli* cell such as a TG1 cell or a BL21 cell (expressing a single chain antibody or a Fab antibody), and the eukaryotic cell is preferably a HEK293 cell or a CHO cell (expressing a full-length IgG antibody). A preferred recombinant expression transformant of the present invention can be obtained by transforming the aforementioned recombinant expression plasmid into a host cell. The transformation method is a conventional transformation method in the art, preferably a chemical transformation method, a heat shock method or an electroporation method.

In order to solve the above-mentioned technical problem, a fifth aspect of the present invention provides a chimeric antigen receptor comprising the anti-B7-H7 antibody or the antigen-binding fragment thereof according to the first aspect of the present invention.

In order to solve the above-mentioned technical problem, a sixth aspect of the present invention provides a genetically modified cell comprising the chimeric antigen receptor according to the fifth aspect of the present invention.

Preferably, the genetically modified cell is a eukaryotic cell, preferably an isolated human cell; more preferably, the genetically modified cell is an immune cell such as a T cell, or an NK cell.

In order to solve the above-mentioned technical problem, a seventh aspect of the present invention provides a method for preparing an anti-B7-H7 antibody or an antigen-binding fragment thereof, which method comprises the following steps: culturing the transformant according to the fourth aspect of the present invention, and obtaining the anti-B7-H7 antibody or the antigen-binding fragment thereof from the culture.

In order to solve the above-mentioned technical problem, an eighth aspect of the present invention provides an antibody-drug conjugate comprising an antibody moiety and a conjugated moiety, wherein the antibody moiety comprises the anti-B7-H7 antibody or the antigen-binding fragment thereof according to the first aspect of the present invention, the conjugated moiety includes, but is not limited to, a detectable marker, a drug, a toxin, a cytokine, a radionuclide, an enzyme, or a combination thereof, and the antibody moiety can be conjugated to the conjugated moiety, for example, via a chemical bond or a linker.

In order to solve the above-mentioned technical problem, a ninth aspect of the present invention provides a pharmaceutical composition comprising the anti-B7-H7 antibody or the antigen-binding fragment thereof according to the first aspect of the present invention, the chimeric antigen receptor according to the fifth aspect of the present invention, the genetically modified cell according to the sixth aspect of the present invention, and/or the antibody-drug conjugate according to the eighth aspect of the present invention.

The pharmaceutical composition of the present invention can be made into various dosage forms as needed, and can be administered by a physician at a dose that is beneficial to a patient based on the type, age, body weight and general disease condition of the patient, the mode of administration, etc. Preferably, the pharmaceutical composition is in a liquid dosage form, a gaseous dosage form, a solid dosage form and a semi-solid dosage form.

Preferably, the pharmaceutical composition can be administered via oral administration, injection administration, nasal administration, transdermal administration or mucosal administration.

Preferably, the pharmaceutical composition further comprises other anti-tumor antibodies as active ingredients.

Preferably, the pharmaceutical composition further comprises a pharmaceutically acceptable carrier. More preferably, the pharmaceutical composition comprises 0.01% to 99.99% of the above-mentioned anti-B7-H7 antibody or the antigen-binding fragment thereof, the chimeric antigen receptor, the genetically modified cell or the antibody-drug conjugate, and 0.01% to 99.99% of a pharmaceutical carrier, and the percentage is mass percentage of the pharmaceutical composition.

The dose level for administration of the pharmaceutical composition of the present invention can be adjusted according to the amount of the composition that can effect the desired diagnostic or therapeutic effect. The administration regimen may also be a single injection or multiple injections, or adjusted. The dose level and regimen selected are reasonably adjusted depending on a variety of factors including the activity, stability (i.e., half-life), preparation and route of administration of the pharmaceutical composition, combinations with other drugs or treatments, a disease or disorder to be detected and/or treated, the health status, prior medical history of a subject to be treated.

The therapeutically effective dose of the pharmaceutical composition of the present invention may initially be estimated in cell culture experiments or animal models such as rodents, rabbits, dogs, pigs and/or primates. Animal models can also be used to determine appropriate concentration ranges and routes of administration. The resulting concentration ranges and routes of administration in the animal models can subsequently be used to determine useful doses and route of administration in humans. In general, the determination and adjustment of effective amount or dose to be administered and the evaluation of when and how such adjustment is to be made are known to those skilled in the art.

For additional guidance on preparations, doses, administration regimens and measurable therapeutic effects, see Berkow et al., (2000), The Merck Manual of Medical Information and Merck&Co.Inc., Whitehouse Station, New Jersey; Ebadi (1998) CRC Desk Reference of Clinical Pharmacology, etc.

In addition, it should be understood by those skilled in the art that, the pharmaceutical composition may further comprise a combined therapeutic agent (i.e., used in combination therapy), the combined therapeutic agent including, but not limited to, a chemotherapeutic agent, a radiotherapeutic agent, an immunosuppressant, and a cytotoxic drug. For a combination therapy with a combined therapeutic agent, the above-mentioned antibody or the antigen-binding fragment thereof, the chimeric antigen receptor, the genetically modified cell, the antibody-drug conjugate, etc., and additional therapeutic or diagnostic agents can each be used as a single agent over any period suitable for performing the intended treatment or diagnosis. Therefore, these single agents can be administered substantially simultaneously (i.e., either as a single preparation or within minutes or hours) or sequentially in succession, depending on the actual requirements at the time of the particular use.

In order to solve the above-mentioned technical problem, a tenth aspect of the present invention provides a kit comprising the antibody or the antigen-binding fragment thereof according to the first aspect of the present invention, the chimeric antigen receptor according to the fifth aspect of the present invention, the genetically modified cell according to the sixth aspect of the present invention, the antibody-drug conjugate according to the eighth aspect of the present invention, and/or the pharmaceutical composition according to the ninth aspect of the present invention.

Preferably, the kit further comprises (i) a device for administering the antibody or the antigen-binding fragment thereof, or the chimeric antigen receptor, or the genetically modified cell, or the antibody-drug conjugate, or the pharmaceutical composition; and/or (ii) instructions for use.

In order to solve the above-mentioned technical problem, an eleventh aspect of the present invention provides the use of the antibody or the antigen-binding fragment thereof according to the first aspect of the present invention, the chimeric antigen receptor according to the fifth aspect of the present invention, the genetically modified cell according to the sixth aspect of the present invention, the antibody-drug conjugate according to the eighth aspect of the present invention, the pharmaceutical composition according to the ninth aspect of the present invention, and/or the kit according to the tenth aspect of the present invention in the preparation of a drug for the treatment and/or prevention of a cancer.

Preferably, the cancer is selected from one or more of the group consisting of lung cancer, breast cancer, gastric cancer, small intestine cancer, colon cancer, rectal cancer, pancreatic cancer, kidney cancer, bladder cancer, osteosarcoma, etc.

In order to solve the above-mentioned technical problem, a twelfth aspect of the present invention provides an administration device comprising the antibody or the antigen-binding fragment thereof according to the first aspect of the present invention, the chimeric antigen receptor according to the fifth aspect of the present invention, the genetically modified cell according to the sixth aspect of the present invention, the antibody-drug conjugate according to the eighth aspect of the present invention, the pharmaceutical composition according to the ninth aspect of the present invention, and/or the kit according to the tenth aspect of the present invention.

Preferably, the administration device further comprises a component, such as a syringe, an infusion device or an implantable administration device, that accommodates the anti-B7-H7 antibody or the antigen-binding fragment thereof, the chimeric antigen receptor, the genetically modified cell, the antibody-drug conjugate, the pharmaceutical composition, and/or the kit, or that administers the anti-B7-H7 antibody or the antigen-binding fragment thereof, the chimeric antigen receptor, the genetically modified cell, the antibody-drug conjugate, the pharmaceutical composition, and/or the kit to a subject.

In order to solve the above-mentioned technical problem, a thirteenth aspect of the present invention provides a method for detecting B7-H7, which method comprises the step of performing detection using the antibody or the antigen-binding fragment thereof according to the first aspect of the present invention, the chimeric antigen receptor according to the fifth aspect of the present invention, the genetically modified cell according to the sixth aspect of the present invention, the antibody-drug conjugate according to the eighth aspect of the present invention, the pharmaceutical composition according to the ninth aspect of the present invention, and/or the kit according to the tenth aspect of the present invention.

Preferably, the detection is for non-diagnostic and/or therapeutic purposes, such as for the detection of B7-H7, etc. in basic research and development.

In order to solve the above-mentioned technical problem, the present invention further provides the use of the antibody or the antigen-binding fragment thereof according to the first aspect of the present invention, the chimeric antigen receptor according to the fifth aspect of the present invention, the genetically modified cell according to the sixth aspect of the present invention, the antibody-drug conjugate according to the eighth aspect of the present invention, the pharmaceutical composition according to the ninth aspect of the present invention, and/or the kit according to the tenth aspect of the present invention in the prevention and/or treatment of a cancer. Preferably, the cancer is as described in the eleventh aspect of the present invention.

In order to solve the above-mentioned technical problem, the present invention further provides a method for treating and/or preventing a B7-H7-related disease or disorder (such as a cancer, preferably a cancer according to the eleventh aspect of the present invention), which method comprises treating a patient in need thereof by administering to the patient in need thereof a therapeutically effective amount of the antibody or the antigen-binding fragment thereof according to the first aspect of the present invention, the chimeric antigen receptor according to the fifth aspect of the present invention, the genetically modified cell according to the sixth aspect of the present invention, the antibody-drug conjugate according to the eighth aspect of the present invention, the pharmaceutical composition according to the ninth aspect of the present invention, and/or the kit according to the tenth aspect of the present invention.

In the present application, unless otherwise defined, the scientific and technical terms used in the present application have the meanings commonly understood by those skilled in the art. Moreover, laboratory operation steps of cell culture, molecular genetics, nucleic acid chemistry, and immunology used in the present application are all conventional steps widely used in the corresponding art. Meanwhile, for better understanding of the present invention, definitions and explanations of related terms are provided below.

Three-letter codes and single-letter codes of amino acids used in the present invention are as known to those skilled in the art, or as described in J. Biol. Chem, 243, p 3558 (1968).

As used in the present application, the term "include" or "comprise" is intended to indicate that compositions and methods include the elements described but do not exclude other elements, so is the term "consisting of" as understood according to the context.

The term "antibody" of the present application can include an immunoglobulin, which is a tetrapeptide chain structure composed of two identical heavy chains and two identical light chains linked via inter-chain disulfide bonds. Since the amino acid composition and arrangement sequence of the heavy chain constant region of an immunoglobulin are different, the antigenicity of the immunoglobulin is also different. Accordingly, immunoglobulin can be divided into five classes, or isotypes of immunoglobulins, namely IgM, IgD, IgG, IgA and IgE, and the corresponding heavy chains thereof are a $\mu$ chain, a $\delta$ chain, a $\gamma$ chain, an $\alpha$ chain and an $\varepsilon$ chain, respectively. The same class of Ig can also be divided into different subclasses according to the differences in the amino acid composition of the hinge region thereof and the number and position of the disulfide bonds of the heavy chain. For example, IgG can be divided into IgG1, IgG2, IgG3, and IgG4. The light chains are divided into a kappa chain or a lamda chain by the difference in the constant region. Each Ig class of the five Ig classes can have either a kappa chain or a lamda chain.

In the present invention, the antibody light chain variable region of the present application may further comprise a light chain constant region, and the light chain constant region comprises a human kappa chain and a human lamda chain or variants thereof. In the present invention, the antibody heavy chain variable region of the present application may further comprise a heavy chain constant region, and the heavy chain constant region comprises human IgG1, IgG2, IgG3, and IgG4, or variants thereof.

Within light and heavy chains, variable regions and constant regions are linked by a "J" region having about 12 or more amino acids, and the heavy chain further comprises a "D" region having about 3 or more amino acids. Each heavy chain consists of a heavy chain variable region (VH) and a heavy chain constant region (CH). The heavy chain constant region consists of 3 domains (CH 1, CH2 and CH3). Each light chain consists of a light chain variable region (VL) and a light chain constant region (CL). The light chain constant region consists of one domain, CL. The constant region of an antibody can mediate the binding of an immunoglobulin to a host tissue or factor, including various cells (e.g., effector cells) of the immune system and the first component (C1q) of the classical complement system. The sequences of about 110 amino acids near the N-terminus of the antibody heavy chain and light chain vary greatly and are the variable regions (V regions); the sequences of other amino acids near the C-terminus are relatively stable and are the constant regions (C regions). The variable region includes 3 hypervariable regions (HVR) and 4 framework regions (FWR) with relatively conserved sequences. Three hypervariable regions, also known as complementarity determining regions (CDRs), determine the specificity of the antibody. Each light chain variable region (VL) and heavy chain variable region (VH) consists of 3 CDRs and 4 FWRs, which are arranged in the following order from amino end to carboxyl end: FWR1, CDR1, FWR2, CDR2, FWR3, CDR3, FWR4. The 3 CDRs of the light chain refer to VL CDR1, VL CDR2 and VL CDR3; and the 3 CDRs of the heavy chain refer to VH CDR1, VH CDR2 and VH CDR3.

The term "human antibody" includes antibodies having variable and constant regions of human germline immunoglobulin sequences. The human antibodies of the present invention may include amino acid residues that are not encoded by human germline immunoglobulin sequences (for example, mutations introduced by random or site-specific mutagenesis *in vitro* or by somatic mutation *in vivo*). However, the term "human antibody" does not include antibodies in which CDR sequences derived from the germline of another mammalian species (such as mouse) have been grafted onto human backbone sequences (i.e., "humanized antibodies") .

As used in the present application, the term "specific" with respect to an antibody means an antibody that recognizes a specific antigen but does not substantially recognize or bind to other molecules in a sample. For example, an antibody that specifically binds to an antigen from one species can also bind to that antigen from one or more species. However, this interspecies cross-reactivity itself does not affect the classification of antibodies according to specificity. In another example, an antibody that specifically binds to an antigen can also bind to different allelic forms of the antigen. However, this cross-reactivity itself does not affect the classification of antibodies according to specificity. In some cases, the term "specific" or "specific binding" can be used to refer to the interaction of an antibody, a protein or a peptide with a second chemical substance, meaning that the interaction depends on the presence of a particular structure (e.g., an antigenic determinant or epitope) on the chemical substance; for example, antibodies generally recognize and bind to particular protein structures, rather than proteins. If an antibody is specific for epitope "A", the presence of a molecule containing epitope A (or free, unlabeled A) will reduce the amount of labeled A bound to the antibody in the reaction between a molecule containing the labeled "A" and the antibody.

In the present application, the term "antigen-binding fragment" refers to an antigen-binding fragment of an antibody and an antibody analog, which generally include at least a portion of the antigen-binding region or variable region (for example, one or more CDRs) of a parental antibody. An antibody fragment retains at least some of the binding specificity of the parental antibody. Typically, the antibody fragment retains at least 10% of the binding activity of the parental antibody when the activity is expressed on a molar basis. Preferably, the antibody fragment retains at least 50%, 60%, 70%, 80%, 90%, 95%, 99% or 100% or more of the binding affinity of the parental antibody for the target. Examples of the antigen-binding fragment include, but are not limited to: Fab, Fab', F(ab')2, a Fv fragment, a linear antibody, a single (heavy) chain antibody, a nanobody, a domain antibody, and a multispecific antibody. Engineered antibody variants are reviewed in Holliger and Hudson (2005) Nat. Biotechnol. 23: 1126-1136.

The term "chimeric antigen receptor" or "CAR" as used herein refers to one comprising an extracellular domain capable of binding to an antigen (extracellular binding domain), a hinge domain, a transmembrane domain (transmembrane region), and a polypeptide that enables the cytoplasmic signaling to a domain (i.e., intracellular signaling domain). The hinge domain can be considered as part of a system for providing flexibility to the extracellular antigen-binding region. The intracellular signaling domain refers to a protein that transmits information into a cell via a defined signaling pathway by generating second messengers to regulate cell activity, or a protein that acts as an effector in response to such messengers, generating signals that can promote immune effector functions of CAR cells (e.g., CAR-T cells). The intracellular signaling domain comprises a signaling domain and may also comprise a co-stimulatory intracellular domain derived from a co-stimulatory molecule.

The "identity" and "mutation" refer to sequence similarity between two polynucleotide sequences or between two polypeptides. When a position in two compared sequences is occupied by the same base or amino acid monomer subunit, for example each position in two DNA molecules is occupied by adenine, the molecules are homologous at that position. The percent identity between two sequences is a function of the number of matching or homologous positions shared by the two sequences divided by the number of positions compared $\times$ 100. For example, when sequences are optimally aligned, if 6 out of 10 positions in two sequences match or are homologous, the two sequences are 60% homologous. In general, comparisons are made when aligning two sequences yields the greatest percent identity.

The terms "polypeptide", "peptide" and "protein" (if single chain) are used interchangeably in the present application. The terms "nucleic acid", "nucleic acid sequence", "nucleotide sequence" or "polynucleotide sequence" and "polynucleotide" are used interchangeably.

The term "vector" refers to a nucleic acid delivery vehicle into which a polynucleotide may be inserted. When the vector allows for the expression of the protein encoded by the inserted polynucleotide, the vector is called an expression vector. A vector can be introduced into a host cell by transformation, transduction or transfection, and the genetic substance elements carried thereby can be expressed in the host cell. Vectors are well known to those skilled in the art, and include, but are not limited to: plasmids; phagemids; cosmids; artificial chromosomes, such as yeast artificial chromosomes (YACs), bacterial artificial chromosomes (BACs) or P1-derived artificial chromosomes (PACs); phages such as a lamda phage or M13 phage and animal viruses. The animal viruses that can be used as vectors include, but are not limited to, retroviruses (including lentiviruses), adenoviruses, adeno-associated viruses, herpes viruses (e.g., herpes simplex virus), poxviruses, baculoviruses, papilloma viruses and papovaviruses (such as SV40). A vector can contain a variety of elements that control expression, including, but not limited to: a promoter sequence, a transcription initiation sequence, an enhancer sequence, a selection element, and a reporter gene. In addition, the vector may further contain a replication initiation site.

The expressions "cell" and "cell line" used in the present application are used interchangeably, and all such designations include progeny. The term "host cell" refers to a cell that can be used to introduce a vector, including but not limited to, a prokaryotic cell such as Escherichia coli, a fungal cell such as a yeast cell, or an animal cell such as a fibroblast, a CHO cell, a COS cell, a NSO cell, an HeLa cell, a BHK cell, a HEK 293 cell or a human cell.

The term "transfection" refers to the introduction of an exogenous nucleic acid into a eukaryotic cell. Transfection can be achieved by a variety of means known in the art, including calcium phosphate-DNA co-precipitation, DEAE-dextran-mediated transfection, polybrene-mediated transfection, electroporation, microinjection, liposome fusion, lipofection, protoplast fusion, retroviral infection and biolistics.

The term "immune cell" refers to a cell that can trigger an immune response, and the "immune cell" and other grammatical forms thereof can refer to an immune cell of any origin. The "immune cells" include, for example, white blood cells (leukocytes), lymphocytes (T cells, B cells, natural killer (NK) cells), and bone marrow-derived cells (neutrophils, eosinophils, basophils, monocytes, macrophages, and dendritic cells) derived from hematopoietic stem cells (HSC) produced in the bone marrow. The term "immune cell" can also be human or non-human. For example, the immune cells can be derived from blood, such as autologous T cells, allogeneic T cells, autologous NK cells and allogeneic NK cells, or from cell lines, such as NK cell lines prepared using EBV infection, NK cells induced and differentiated from embryonic stem cells and iPSC, and NK92 cell lines.

As used in the present application, the term "T cell" refers to a type of lymphocyte that matures in the thymus. T cells play an important role in cell-mediated immunity and differ from other lymphocytes (e.g., B cells) in the presence of T cell receptors on the cell surface. "T cells" include all types of immune cells that express CD3, including helper T cells (CD4+ cells), cytotoxic T cells (CD8+ cells), natural killer T cells, regulatory T cells (Treg) and γ-δ T cells. "Cytotoxic cells" include CD8+T cells, natural killer (NK) cells and neutrophils, which are capable of mediating cytotoxic responses. As used herein, the term "NK cells" refer to a type of lymphocytes that originate from the bone marrow and play an important role in the innate immune system. NK cells provide a rapid immune response against virally infected cells, tumor cells or other stressed cells, even in the absence of antibodies and major histocompatibility complexes on the cell surface.

The "optional", "any one", "any" or "any one of" means that the event or circumstance subsequently described may, but need not occur, and the description includes the occasions where the events or circumstances occur or do not occur. For example, "optionally comprising 1 antibody heavy chain variable region" means that an antibody heavy chain variable region of a particular sequence may, but need not be present. As used in the present invention, the "a/an" is used in the present invention to refer to one or more grammatical objects. Unless clearly indicated otherwise in the content, the term "or" is used in the present invention to mean the term "and/or" and is used interchangeably therewith. The "about" and "approximately" should generally mean the degree of an acceptable error of the measured amount in view of the property or accuracy of the measurement. Exemplary degrees of error are generally within 10% thereof and more generally within 5% thereof. The methods and compositions disclosed in the present invention encompass polypeptides and nucleic acids having a specified sequence, a variant sequence, or a sequence substantially identical or similar thereto, e.g., a sequence that is at least 85%, 90%, 95%, 99% or more identical to the sequence specified. In the case of an amino acid sequence, the term "substantially identical" is used in the present invention to refer to a first amino acid sequence.

As used herein, the term "pharmaceutical composition" generally refers to a preparation that is present in a form which allows active ingredients to be biologically effective and that does not contain additional ingredients which would be unacceptably toxic to a subject to which the pharmaceutical composition is administered. The composition is sterile.

As used herein, the term "pharmaceutically acceptable carrier" refers to a carrier that is pharmacologically and/or physiologically compatible with a subject and active ingredients and that is well known in the art (see, for example, Remington's Pharmaceutical Sciences. Edited by Gennaro AR, 19th ed. Pennsylvania: Mack Publishing Company, 1995), including but not limited to: a pH adjusting agent, a surfactant, an adjuvant, an ion strength enhancer, a diluent, a reagent for maintaining the osmotic pressure, an absorption delaying agent, and a preservative. For example, the pH adjusting agent includes, but is not limited to, a phosphate buffer. The surfactant includes, but is not limited to, a cationic, anionic or nonionic surfactant, such as Tween-80. The ion strength enhancer includes, but is not limited to, sodium chloride. The preservative includes, but is not limited to, various antibacterial reagents and antifungal reagents, such as parabens, chlorobutanol, phenol, and sorbic acid. The reagent for maintaining the osmotic pressure includes, but is not limited to, a saccharide, NaCl, and an analog thereof. The absorption delaying agent includes, but is not limited to, monostearate and gelatin. The diluent includes, but is not limited to, water, aqueous buffers (such as buffered saline), alcohols, polyols (such as glycerol), etc. The preservative includes, but is not limited to, various antibacterial reagents and antifungal reagents, such as thiomersal, 2-phenoxyethanol, parabens, chlorobutanol, phenol, and sorbic acid. The "stabilizer" has the meaning that is commonly understood by those skilled in the art and can stabilize the desired activity of active ingredients in a drug. The stabilizer includes, but is not limited to, sodium glutamate, gelatin, SPGA, saccharides (such as sorbitol, mannitol, starch, sucrose, lactose, dextran or glucose), amino acids (such as glutamic acid or glycine), proteins (such as dry whey, albumin or casein), or degradation products thereof (such as lactalbumin hydrolysate).

As used herein, the term $EC_{50}$ refers to the concentration for 50% of maximal effect, i.e., the concentration that causes 50% of the maximal effect.

As used herein, the terms "cancer" and "cancer patient" are intended to include all types of cancerous neoplasms or tumorigenic processes, metastatic tissues or malignant transformed cells, tissues or organs, regardless of their histopathological type or aggressive phase. Examples include, but are not limited to, solid tumors, hematological cancers, soft tissue tumors, and metastatic lesions.

It should be understood by those skilled in the art that products, such as CAR-T, TCR-T, double antibodies, polyclonal antibodies, ADCs and other product forms, comprising the anti-B7-H7 antibody or the antigen-binding fragment thereof of the present invention should fall within the scope of protection of the present invention.

On the basis of meeting common knowledge in the art, the above-mentioned various preferred conditions can be combined in any form, such that various preferred examples of the present invention are obtained.

Reagents and raw materials used in the present invention are all commercially available.

The present invention has the positive improvement effects as follows. the anti-B7-H7 antibody or the antigen-binding fragment thereof of the present invention has high affinity and high biological activity for B7-H7, and can effectively block the binding of B7-H7 protein to a receptor CD28H thereof and the binding of B7-H7 protein to a receptor KIR3DL3 thereof; furthermore, the anti-B7-H7 antibody or the antigen-binding fragment thereof of the present invention only specifically binds to B7-H7 protein without cross-reacting with other member proteins of the B7 family, and has good stability. In addition, the anti-B7-H7 antibody or the antigen-binding fragment thereof of the present invention shows strong *in vivo* anti-tumor activity.

Brief Description of the Drawings

FIG. 1 shows the binding of anti-B7-H7 initial antibodies to human and cynomolgus monkey B7-H7 on the cell surface and to B7-H7 on the surface of tumor cells HCC827.

FIG. 2 shows the binding of anti-B7-H7 LALA mutation antibodies to tumor cell LS180.

FIG. 3 shows the binding of anti-B7-H7 PTM mutation/LALA mutation antibodies to tumor cell LS180.

FIG. 4 shows the blocking effects of anti-B7-H7 antibodies against the binding of B7-H7 protein to CD28H on the surface of HEK-293 cells.

FIG. 5 shows the blocking effects of anti-B7-H7 antibodies and anti-B7-H7 LALA mutation antibodies against the binding of B7-H7 protein to KIR3DL3 on the surface of CHO-K1 cells.

FIG. 6 shows the blocking effects of anti-B7-H7 antibodies and anti-B7-H7 LALA mutation antibodies against the binding of B7-H7 protein to KIR3DL3 protein.

FIG. 7 shows that anti-B7-H7 antibodies block the immunosuppressive signaling of B7-H7 and thus activate T cells.

FIG. 8 shows that anti-B7-H7 antibodies do not act on B7-H7 negative cells and thus cannot activate T cells.

FIG. 9 shows that anti-B7-H7 antibody stimulates NK cells to kill target cells.

FIG. 10 shows the cross-reactivity of anti-B7-H7 LALA mutation antibodies with other member proteins of the B7 family.

FIG. 11 shows the stability of anti-B7-H7 LALA mutation antibodies in sera.

FIG. 12 shows the anti-tumor effects of anti-B7-H7 antibodies in mouse tumor models.

Detailed Description of Embodiments

The present invention is further described below by way of examples; however, the present invention is not limited to the scope of the described examples. For the experimental methods in which no specific conditions are specified in the following examples, selections are made according to conventional methods and conditions or according to the product instructions.

**Example 1. Obtaining of anti-B7-H7 antibody molecules**

Experimental animals, which could be mice, rats, rabbits, sheep, camels, etc., were immunized with the B7-H7 recombinant protein to obtain antibody molecules that specifically bind to B7-H7. Usually, the resulting antibody molecules are of non-human origin. After obtaining non-human antibodies, these molecules need to be humanized using antibody engineering techniques to reduce immunogenicity and improve druggability. However, the process of humanizing antibodies has its technical complexity, and molecules that have been humanized tend to have reduced affinity for antigens. On the other hand, advances in transgenic technology allows the breeding of genetically engineered mice that carry the human immunoglobulin repertoire and in which their endogenous murine immunoglobulin repertoires are deleted. Harbour H2L2 mice (Harbour antibodies BV) are transgenic mice carrying the human immunoglobulin repertoire, and the antibodies produced by the transgenic mice have a fully human sequence, so there is no need for further humanization.

**Example 1.1. Mouse immunization**

Harbour H2L2 mice were immunized in multiple rounds with a soluble recombinant human B7-H7-ECD-his fusion protein (Sino Biological, # 16139-H08H) as an antigen. The antigen protein was mixed with an immunoadjuvant to form an immunogen reagent, and the immunogen reagent was then injected subcutaneously in the inguinal region or injected intraperitoneally. In each round of immunization, each mouse received a total injection dose of 100 microliters. In the first round of immunization, each mouse was immunized with an immunogen reagent prepared by mixing 50 micrograms of the antigen protein with a complete Freund's adjuvant (Sigma, # F5881) at a volume ratio of 1:1. In each subsequent round of booster immunization, each mouse was immunized with an immunogen reagent prepared by mixing 25 micrograms of the antigen protein with Sigma Adjuvant System adjuvant (Sigma, # S6322). The interval between two rounds of booster immunization was at least two weeks, typically with 4 to 5 rounds of booster immunization. The immunization time was on day 0, 14, 28, 42, 56, 70, 84, and 96; and on days 49 and 77, the antibody titer in the sera of mice was measured. Five days before the isolation of splenic B cells in H2L2 mice, the final booster immunization was performed at a dose of 25 micrograms of the antigen protein per mouse.

**Example 1.2. Measurement of titer in sera**

At specific time points, mouse sera were harvested, the titer of the antibody that binds to B7-H7 protein in the sera was measured by ELISA, and the titer of the antibody that binds to cells overexpressing B7-H7 in the sera was measured by FACS.

In the ELISA, an ELISA plate (corning, 9018) was coated with 1 μg/mL hB7-H7-ECD-hFc protein (Sino Biological, # 16139-H02H) at 100 μL/well and incubated overnight at 4°C; after the plate was rinsed twice, blocking was performed with PBST containing 1% BSA for 2 hours at 37°C; gradiently diluted sera were added at 100 μL/well, and incubation was performed for 1 hour at 37°C; and after the plate was rinsed 3 times, anti-rat-HRP (sigma, # A5795) at 1 : 5000 dilution was added at 100 μL/well, and incubation was performed for 30 minutes at 37°C. After the plate was rinsed 3 times, a TMB substrate was added at 100 μL/well, incubation was performed for about 10 minutes, 1 N HCl was added at 50 μL/well to stop color development, and the absorbance at 450 nm was read (Molecular Devices, Plus 384).

In the FACS, gradiently diluted murine sera were incubated with HEK293-B7-H7 cells for 1 hour at 4°C; and after the cells were washed twice, a secondary antibody anti-rat IgG (H+L) (Life technologies, A11006) was added, incubation was performed for 1 hour at 4°C, and after the cells were washed twice, the cells were resuspended and then detected by a flow cytometer (BD, Flibur). HEK293 cells were used as a background control.

### Example 1.3. Screening of anti-B7-H7 antibodies by hybridoma technique

Immunized mice with high valency in the sera were selected for one final immunization and the mice were sacrificed, and the splenocytes and SP2/0 myeloma cells (ATCC, CRL-1581) were taken for electrofusion at a cell ratio of 4 : 1, wherein the electrofusion parameters were V1: 50 V, t1: 15 s, V2: 600 V, t2: 20 μs, t3: 0.5 s, n: 1, t4: 7 s, V+/-: +, fade: on. The cells were resuspended in a DMEM medium containing 20% FBS and HT, and plated at $1 \times 10^5$ cells/100 μL/well, and after 24 hours, a DMEM medium containing 20% FBS and $2 \times$ HT was added at 100 μL/well for further culture. The supernatant was subsequently taken, and the titer of the antibody was detected. Generally, 9-15 days after the fusion, the supernatants from the mice immunized with the protein were initially screened using Acumen to detect the binding to HEK-293/huB7-H7 cells; and the positive clones were then further confirmed by ELISA and FACS to detect the ability for binding to the HEK-293 cell strain overexpressing human B7-H7 (HEK-293/huB7-H7) and HEK-293 cell strain overexpressing cynomolgus monkey B7-H7 (HEK-293/cynoB7-H7). Subcloning was further performed in the positive wells by limiting dilution, and screening was then performed by ELISA and FACS. The clones having good binding to human B7-H7 and monkey B7-H7 were selected for sequencing.

### Example 1.4. Sequencing and sequence optimization of anti-B7-H7 antibodies

The nucleotide sequences encoding the variable domains of the antibody molecules and the corresponding amino acid sequences were obtained by conventional sequencing means. The sequences of 14 monoclonal antibodies were obtained. In this example, the sequences of the variable domains of the anti-B7-H7 monoclonal antibody molecules obtained from the immunized Harbour H2L2 mice were human antibody sequences. The germline gene analysis and post-translational modification (PTM) site analysis of the anti-B7-H7 monoclonal antibody are listed in Table 1-1.

After the translation synthesis of amino acid chains of proteins or polypeptides in cells, chemical modifications are sometimes introduced, called post-translational modifications (PTMs). For antibodies, some of the PTM sites are very conserved. For example, the conserved amino acid asparagine Asn at position 297 (EU numbering) of the constant domain of human IgG1 antibodies is usually glycosylated to form a saccharide chain, and this saccharide chain structure is critical for the antibody structure and related effector functions. However, if a PTM is present in a variable domain of an antibody, particularly in an antigen-binding region (e.g., CDR), the presence of such a PTM can have a greater impact on the binding to an antigen, and can also cause a change in the physicochemical properties of the antibody. For example, glycosylation, deamidation, isomerization, oxidation, etc. may increase the instability or heterogeneity of antibody molecules, thereby increasing the difficulty and risk of antibody development. Avoiding some potential PTMs is thus very important for the development of therapeutic antibodies. With the accumulation of experience, it has been found that some PTMs are highly correlated with the "pattern" of the composition of amino acid sequences, particularly of the composition of adjacent amino acids, which makes it possible to predict potential PTMs from the primary amino acid sequences of proteins. For example, an N-linked glycosylation site can be predicted according the sequence pattern N-x-S/T (asparagine at the first position, any amino acid other than proline at the second position, and serine or threonine at the third position). Amino acid sequence patterns that cause PTMs may be derived from germline gene sequences, for example, pattern NST where human germline gene fragment IGHV3-33 naturally has a glycosylation modification in the FR3 region; and they may also be derived from somatic hypermutation.

The amino acid sequence patterns of PTMs can be disrupted by amino acid mutations, thereby reducing or removing the formation of particular PTMs. Depending on different antibody sequences and different PTM sequence patterns, there are different mutation design methods. One method is to replace the "hot spot" amino acid (such as N or S in the NS pattern) with an amino acid having similar physicochemical properties (e.g., mutation of N to Q). If the PTM sequence pattern is derived from somatic hypermutation and is not present in the germline gene sequences, another method may be to replace the sequence pattern with the corresponding germline gene sequence. In actual operation, multiple mutation design methods may be used for the same PTM sequence pattern.

The sequences of the new antibody molecules resulting from amino acid mutations in the sequences of antibodies PR003249, PR003259 and PR003264 are listed in Table 1-2, wherein PR003181 is a positive control, from patent WO 2014190356.

Table 1-1 Germline gene analysis and PTM site analysis of anti-B7-H7 antibodies

| Clone No. | VH germline V gene | VL germline V gene | VH PTM | VL PTM | Recombinant antibody | Recombinant antibody subtype |
|---|---|---|---|---|---|---|
| 13D8D2 | IGHV3-33*01 | IGKV1-5*03 | DG (HCDR2), NS (HCDR3) | NS (LCDR3) | PR003248 | Human IgG1 |
| 25H3F2 | IGHV3-33*01 | IGKV1-5*03 | DG (HCDR2) | NS (LCDR3) | PR003249 | Human IgG1 |
| 34G2E6 | IGHV3-33*01 | IGKV1-5*03 | DG (HCDR2) | NS (LCDR3) | PR003250 | Human IgG1 |
| 29F2C8 | IGHV3-33*01 | IGKV3-15*01 | NG (HCDR2), DG (HCDR3) | | PR003251 | Human IgG1 |
| 53C12D4 | IGHV3-33*01 | IGKV1-5*03 | DG (HCDR2) | NS (LCDR3) | PR003252 | Human IgG1 |
| 59D6D2 | IGHV3-33*01 | IGKV3-15*01 | NG (HCDR2), DG (HCDR3) | | PR003254 | Human IgG1 |
| 68F3A2 | IGHV3-33*06 | IGKV1-27*01 | DG (HCDR2) | NS (LCDR3) | PR003259 | Human IgG1 |
| 70F3B1 | IGHV3-33*01 | IGKV3-15*01 | DG (HCDR2), DG (HCDR3) | | PR003260 | Human IgG1 |
| 74A11E4 | IGHV3-33*01 | IGKV1-5*03 | DG (HCDR2) | NS (LCDR3) | PR003261 | Human IgG1 |
| 77D5A4 | IGHV3-33*01 | IGKV3-15*01 | NG (HCDR2), DG (HCDR3) | | PR003262 | Human IgG1 |
| 77E6C4 | IGHV3-33*01 | IGKV3-15*01 | DG (HCDR2) | | PR003263 | Human IgG1 |
| 77G10D3 | IGHV1-69*01 | IGKV1-9*01 | | | PR003264 | Human IgG1 |
| 78G8B3 | IGHV3-33*01 | IGKV3-15*01 | | | PR003265 | Human IgG1 |
| 7D3D2 | IGHV3-33*01 | IGKV1-5*03 | DG (HCDR2) | NS (LCDR3) | PR003266 | Human IgG1 |

Table 1-2 Mutation site design of antigen-binding protein sequences

| Initial antibody | Variant | Mutation in variable region | Recombinant antibody subtype | Mutation in Fc |
|---|---|---|---|---|
| PR003181 | PR004260 | - | Human IgG1 | LALA mutation |
| PR003249 | PR004261 | - | Human IgG1 | LALA mutation |
| PR003259 | PR004262 | - | Human IgG1 | LALA mutation |
| PR003264 | PR004263 | - | Human IgG1 | LALA mutation |
| PR003249 | PR004479 | H:D54E | Human IgG1 | LALA mutation |
| PR003249 | PR004480 | H:D54E; L:N92Q | Human IgG1 | LALA mutation |
| PR003249 | PR004481 | H:D54E; L:S93A | Human IgG1 | LALA mutation |
| PR003249 | PR004482 | H:G55A | Human IgG1 | LALA mutation |
| PR003249 | PR004483 | H:G55A; L:N92Q | Human IgG1 | LALA mutation |
| PR003249 | PR004484 | H:G55A; L:S93A | Human IgG1 | LALA mutation |

18

(continued)

| Initial antibody | Variant | Mutation in variable region | Recombinant antibody subtype | Mutation in Fc |
|---|---|---|---|---|
| PR003249 | PR004485 | L:N92Q | Human IgG1 | LALA mutation |
| PR003249 | PR004486 | L:S93A | Human IgG1 | LALA mutation |
| PR003259 | PR004487 | H:D54E | Human IgG1 | LALA mutation |
| PR003259 | PR004488 | H:D54E; L:N92Q | Human IgG1 | LALA mutation |
| PR003259 | PR004489 | H:D54E; L:S93A | Human IgG1 | LALA mutation |
| PR003259 | PR004490 | H:G55A | Human IgG1 | LALA mutation |
| PR003259 | PR004491 | H:G55A; L:N92Q | Human IgG1 | LALA mutation |
| PR003259 | PR004492 | H:G55A; L:S93A | Human IgG1 | LALA mutation |
| PR003259 | PR004493 | L:N92Q | Human IgG1 | LALA mutation |
| PR003259 | PR004494 | L:S93A | Human IgG1 | LALA mutation |

**Example 1.5. Preparation and physicochemical property characterization analysis of recombinant antibodies**

**Example 1.5.1. Expression and purification of antibodies**

In this example, the general method for preparing antibodies using techniques such as mammalian host cells (e.g., human embryonic kidney cells HEK293 or Chinese hamster ovary cells CHO and derivatives thereof), transient transfection expression and affinity capture isolation was described. The present method is suitable for an antibody of interest containing an Fc region; the antibody of interest may be composed of one or more protein polypeptide chains; and the antibody of interest may be derived from one or more expression plasmids.

The amino acid sequences of antibody polypeptide chains are converted into nucleotide sequences by codon optimization; and the nucleotide sequences for encoding are respectively synthesized and cloned onto expression vectors compatible with a host cell. The plasmids encoding the antibody polypeptide chains are simultaneously transfected into a mammalian host cell according to a particular ratio, and the recombinant antibodies having correct folding and polypeptide chain assembly can be obtained using conventional recombinant protein expression and purification techniques. Specifically, FreeStyle™ 293-F cells (Thermo, # R79007) were subjected to scale-up culture in a FreeStyle™ F17 Expression Medium (Thermo, # A1383504). Before transient transfection, the cells were adjusted to a cell concentration of $6\text{-}8 \times 10^5$ cells/mL, and cultured in a shaker at 37°C and 8% $CO_2$ for 24 hours at a cell concentration of $1.2 \times 10^6$ cells/mL. 30 mL of the cultured cells was prepared. The plasmids encoding the antibody polypeptide chains were mixed according to a certain ratio, a total of 30 $\mu$g of plasmids (the ratio of the plasmids to the cells being 1 $\mu$g:1 mL) were dissolved in 1.5 mL of Opti-MEM reduced serum medium (Thermo, # 31985088), and the resulting mixture was filtered with a 0.22 $\mu$m filter membrane for sterilization. Another 1.5 mL of Opti-MEM was taken and dissolved in 120 $\mu$! of 1 mg/mL PEI (Polysciences, # 23966-2), and the resulting mixture was left to stand for 5 minutes. The PE! was slowly added to the plasmids, the resulting mixture was incubated at room temperature for 10 minutes, the plasmid and PEI mixed solution was slowly dripped into the culture flask while shaking the culture flask, and cultured in a shaker at 37°C and 8% $CO_2$ for 5 days. The cell viability was observed after 5 days. The culture was collected, and centrifuged at a rotary speed of 3300 g for 10 minutes, and then the supernatant was taken; and the supernatant was then centrifuged at a high speed to remove impurities. A gravity column (Bio-Rad, #7311550) containing MabSelect™ (GE Healthcare, #71-5020-91) was equilibrated with a PBS buffer with pH 7.4, that is, the column was rinsed with 2-5 times the column volume of the buffer. The supernatant sample was loaded onto the column; and the column was rinsed with 5-10 times the column volume of PBS buffer, the protein of interest was eluted with 0.1 M glycine with pH 3.5, then adjusted with Tris-HCl with pH 8.0 until neutrality, and finally transferred to a PBS buffer or a buffer containing other components through concentration and liquid exchange by an ultrafiltration tube (Millipore, # UFC901024) to obtain a purified recombinant antibody solution. Finally, the concentration was determined using NanoDrop (Thermo, NanoDrop™ One), and the purified recombinant antibody solution was subpackaged and stored for later use.

**Example 1.5.2. Analysis of protein purity and aggregate by SEC-HPLC**

In this example, analytical size exclusion chromatography (SEC) was used to analyze the purity and aggregate form of the protein samples. An analytical chromatographic column TSKgel G3000SWxl (Tosoh Bioscience, #08541, 5 $\mu$m, 7.8 mm $\times$ 30 cm) was connected to a high pressure liquid chromatograph HPLC (Agilent Technologies, Agilent 1260 Infinity II), and equilibrated with a PBS buffer for at least 1 hour at room temperature. An appropriate amount of protein sample (at least 10 μg) was filtered with a 0.22 μm filter membrane and then injected into the system, and the HPLC procedure was set: a PBS buffer was used to make the sample flow through the column at a flow rate of 1.0 mL/min for up to 25 minutes. HPLC would generate an analytical report where the retention times of components having different molecular sizes in the sample were reported.

### Example 1.5.3. Analysis of protein purity and hydrophobicity by HIC-HPLC

Analytical hydrophobic interaction chromatography (HIC) was used to analyze the purity and hydrophobicity of the protein samples. An analytical chromatographic column TSKge1 Buty1-NPR (Tosoh Bioscience, 14947, 4.6 mm × 3.5 cm) was connected to a high pressure liquid chromatograph HPLC (Agilent Technologies, Agilent 1260 Infinity II), and equilibrated with a PBS buffer for at least 1 hour at room temperature. The conditions were set up as follows: a linear gradient from 100% mobile phase A (20 mM histidine, 1.8 M ammonium sulfate, pH 6.0) to 100% mobile phase B (20 mM histidine, pH 6.0) over 16 minutes, a flow rate set at 0.7 mL/min, a protein sample concentration of 1 mg/mL, an injection volume of 20 μl, and a detection wavelength of 280 nm. After acquisition, ChemStation software was used to integrate the chromatogram and calculate the relevant data to generate an analytical report where the retention times of components having different molecular sizes in the sample were reported.

### Example 1.5.4. Determination of thermal stability of protein molecules by DSF

Differential scanning fluorimetry (DSF) is a common high-throughput method for determine the thermal stability of proteins. In the method, a real-time fluorescent quantitative PCR instrument is used to monitor the change in the fluorescence intensity of the dye bound to the unfolded protein molecule to reflect the process of protein denaturation, thereby reflecting the thermal stability of the protein molecule. In this example, the thermal denaturation temperature (Tm) of the protein molecule was determined by DSF. 10 μg of protein was added to a 96-well PCR plate (Thermo, # AB-0700/W), then 2 μl of 100 × diluted dye SYPROTM (Invitrogen, # 2008138) was added, and then a buffer was added to achieve a final volume of 40 μl per well. The PCR plate was sealed and placed in a real-time fluorescent quantitative PCR instrument (Bio-Rad CFX96 PCR System), incubation was first performed at 25°C for 5 minutes, then the temperature gradually increased from 25°C to 95°C at a gradient of 0.2°C/0.2 min, and the temperature decreased to 25°C at the end of the test. The FRET scanning mode was used, Bio-Rad CFX Maestro software was used to perform data analysis, and the Tm of the sample was calculated.

### Example 1.6. Preparation of fully human recombinant antibodies against B7-H7

The fully human IgG antibodies against B7-H7 obtained in example 1.6 were prepared and analyzed using the methods of example 1.5. Table 1-3 and Table 1-4 list the transient expression and purification results of small volume expression systems and large volume expression systems, respectively. In addition, the anti-B7-H7 antibody sequence which was obtained from the existing literature (Table 1-5), was used as the control in subsequent experiments.

Table 1-3 Expression of anti-B7-H7 antibodies

| Antibody | Expression system and volume | Yield after one-step purification (mg/L) | SEC-HPLC purity (%) |
|---|---|---|---|
| PR003248 | HEK293; 30 ml | 49.9 | 99.2 |
| PR003249 | HEK293; 30 ml | 43.8 | 99.2 |
| PR003250 | HEK293; 30 ml | 41.1 | 98.2 |
| PR003251 | HEK293; 30 ml | 46.8 | 99.9 |
| PR003252 | HEK293; 30 ml | 53.2 | 97.9 |
| PR003254 | HEK293; 30 ml | 61.5 | 99.4 |
| PR003259 | HEK293; 30 ml | 67.8 | 99.6 |
| PR003260 | HEK293; 30 ml | 54.0 | 93.1 |
| PR003261 | HEK293; 30 ml | 43.2 | 94.4 |
| PR003262 | HEK293; 30 ml | 24.3 | 77.7 |
| PR003263 | HEK293; 30 ml | 58.0 | 99.0 |
| PR003264 | HEK293; 30 ml | 59.7 | 100.0 |

(continued)

| Antibody | Expression system and volume | Yield after one-step purification (mg/L) | SEC-HPLC purity (%) |
|---|---|---|---|
| PR003265 | HEK293; 30 ml | 69.9 | 100.0 |
| PR003266 | HEK293; 30 ml | 44.1 | 99.6 |
| PR003181 | Expi-CHOs; 50 ml | 327.66 | 99.54 |
| PR004479 | HEK293; 10 ml | Expressed supernatant only | |
| PR004480 | HEK293; 10 ml | Expressed supernatant only | |
| PR004481 | HEK293; 10 ml | Expressed supernatant only | |
| PR004482 | HEK293; 10 ml | Expressed supernatant only | |
| PR004483 | HEK293; 10 ml | 11.00 | ND |
| PR004484 | HEK293; 10 ml | 14.93 | 96.44% |
| PR004485 | HEK293; 10 ml | Expressed supernatant only | |
| PR004486 | HEK293; 10 ml | Expressed supernatant only | |
| PR004487 | HEK293; 10 ml | Expressed supernatant only | |
| PR004488 | HEK293; 10 ml | Expressed supernatant only | |
| PR004489 | HEK293; 10 ml | Expressed supernatant only | |
| PR004490 | HEK293; 10 ml | Expressed supernatant only | |
| PR004491 | HEK293; 10 ml | 12.77 | 93.06% |
| PR004492 | HEK293; 10 ml | 14.68 | 94.1% |
| PR004493 | HEK293; 10 ml | Expressed supernatant only | |
| PR004494 | HEK293; 10 ml | Expressed supernatant only | |

Table 1-4 Expression of anti-B7-H7 antibodies

| Antibody | Expression system and volume | Yield after two-step purification (mg/L) | SEC-HPLC purity (%) |
|---|---|---|---|
| PR004260 | Expi-CHO/1000 ml | 230.18 | 98.09% |
| PR004261 | Expi-CHO/1000 ml | 266.5 | 97.75% |
| PR004262 | Expi-CHO/1000 ml | 256.3 | 100.00% |
| PR004263 | Expi-CHO/1000 ml | 158.3 | 98.80% |

Table 1-5 Information about control antibody

| Control antibody | Antibody number | Description |
|---|---|---|
| Control antibody 1 | PR003181 | From patent WO 2014190356 |

**Example 1.7. Sequence and numbering of anti-B7-H7 antibodies**

The amino acid sequences of the CDRs of the antibodies obtained in the present invention are as shown in the table below, and these CDRs are all shown according to the rules of Chothia definition.

Table 1-6 Definition method for CDRs of antibodies of the present application

| | Kabat | Chothia | Combined |
|---|---|---|---|
| LCDR1 | L24--L34 | L24--L34 | L24-L34 |
| LCDR2 | L50--L56 | L50--L56 | L50-L56 |
| LCDR3 | L89--L97 | L89--L97 | L89-L97 |
| HCDR1 | H31--H35 | H26--H32 | H26-H35 |

(continued)

| | Kabat | Chothia | Combined |
|---|---|---|---|
| HCDR2 | H50--H65 | H52--H56 | H50-H65 |
| HCDR3 | H95--H102 | H95--H102 | H95-H102 |

Laa-Lbb may refer to an amino acid sequence from position aa (Chothia numbering) to position bb (Chothia numbering), starting from the N-terminus of an antibody light chain; and Haa-Hbb may refer to an amino acid sequence from position aa (Chothia numbering) to position bb (Chothia numbering), starting from the N-terminus of an antibody heavy chain. For example, L24-L34 may refer to an amino acid sequence from position 24 to position 34 according to Chothia numbering, starting from the N-terminus of an antibody light chain; and H26-H35 may refer to an amino acid sequence from position 26 to position 35 according to Chothia numbering, starting from the N-terminus of an antibody heavy chain. It should be known by those skilled in the art, when Chothia numbering scheme is used to number CDR, there may be cases where insertion sites are present at some locations (see http://bioinf.org.uk/abs/).

Table 1-7-1 and table 1-7-2 list the sequence numbers corresponding to the sequences of the anti-B7-H7 antibody molecules of the present invention and the control antibody molecule.

Table 1-7-1 Sequence numbers of anti-B7-H7 antibodies (rules of Chothia definition)

| Antibody number | Heavy chain HC (SEQ ID NO:) | VH (SEQ ID NO:) | HCDR1 (SEQ ID NO:) | Amino acid sequence | HCDR2 (SEQ ID NO:) | Amino acid sequence | HCDR3 (SEQ ID NO:) | Amino acid sequence |
|---|---|---|---|---|---|---|---|---|
| PR003181 | 168 | 131 | 6 | GYTFTSH | 26 | FPGDGS | 56 | NSFYSMDY |
| PR003248 | 169 | 132 | 7 | GFTFSDY | 27 | WYDGSE | 57 | DRNSGALGYFPH |
| PR003249 | 170 | 133 | 7 | GFTFSDY | 28 | WYDGSD | 58 | DRSSGAMGYFVY |
| PR003250 | 171 | 134 | 7 | GFTFSDY | 28 | WYDGSD | 59 | DRSSGAMGYFAY |
| PR003251 | 172 | 135 | 8 | GFTFSRY | 29 | WYNGNN | 60 | DGAYSYGPFDY |
| PR003252 | 173 | 136 | 9 | GFTFSNY | 27 | WYDGSE | 61 | EYSSGWGYFDY |
| PR003254 | 174 | 137 | 10 | G FTFSSY | 29 | WYNGNN | 62 | DGGYSYGPFDY |
| PR003259 | 175 | 138 | 9 | GFTFSNY | 27 | WYDGSE | 63 | DLISGIYYVDH |
| PR003260 | 176 | 139 | 8 | GFTFSRY | 30 | WYDGSK | 62 | DGGYSYGPFDY |
| PR003261 | 177 | 140 | 9 | GFTFSNY | 31 | WYDGTN | 64 | DRNTAMDYFVY |
| PR003262 | 178 | 141 | 8 | GFTFSRY | 32 | WYNGRN | 62 | DGGYSYGPFDY |
| PR003263 | 179 | 142 | 11 | G FTFSYY | 33 | WFDGTN | 65 | GYGSGSYYNYVM DA |
| PR003264 | 180 | 143 | 12 | GGTFSSY | 34 | IPLIGT | 66 | DTMVRGVRALD Y |
| PR003265 | 181 | 144 | 13 | GFTFSTY | 35 | WYDESN | 67 | LAHDYGGTYFDY |
| PR003266 | 182 | 145 | 7 | GFTFSDY | 36 | WYDGSN | 68 | DRSSGALGYFQH |
| PR004260 | 183 | 131 | 6 | GYTFTSH | 26 | FPGDGS | 56 | NSFYSMDY |
| PR004261 | 184 | 133 | 7 | GFTFSDY | 28 | WYDGSD | 58 | DRSSGAMGYFVY |
| PR004262 | 185 | 138 | 9 | GFTFSNY | 27 | WYDGSE | 63 | DLISGIYYVDH |
| PR004263 | 186 | 143 | 12 | GGTFSSY | 34 | IPLIGT | 66 | DTMVRGVRALD Y |
| PR004479 | 187 | 146 | 7 | GFTFSDY | 37 | WYEGSD | 58 | DRSSGAMGYFVY |
| PR004480 | 187 | 146 | 7 | GFTFSDY | 37 | WYEGSD | 58 | DRSSGAMGYFVY |
| PR004481 | 187 | 146 | 7 | GFTFSDY | 37 | WYEGSD | 58 | DRSSGAMGYFVY |

(continued)

| Antibody number | Heavy chain HC (SEQ ID NO:) | VH (SEQ ID NO:) | HCDR1 (SEQ ID NO:) | Amino acid sequence | HCDR2 (SEQ ID NO:) | Amino acid sequence | HCDR3 (SEQ ID NO:) | Amino acid sequence |
|---|---|---|---|---|---|---|---|---|
| PR004482 | 188 | 147 | 7 | GFTFSDY | 38 | WYDASD | 58 | DRSSGAMGYFVY |
| PR004483 | 188 | 147 | 7 | GFTFSDY | 38 | WYDASD | 58 | DRSSGAMGYFVY |
| PR004484 | 188 | 147 | 7 | GFTFSDY | 38 | WYDASD | 58 | DRSSGAMGYFVY |
| PR004485 | 184 | 133 | 7 | GFTFSDY | 28 | WYDGSD | 58 | DRSSGAMGYFVY |
| PR004486 | 184 | 133 | 7 | GFTFSDY | 28 | WYDGSD | 58 | DRSSGAMGYFVY |
| PR004487 | 189 | 148 | 9 | GFTFSNY | 39 | WYEGSE | 63 | DLISGIYYVDH |
| PR004488 | 189 | 148 | 9 | GFTFSNY | 39 | WYEGSE | 63 | DLISGIYYVDH |
| PR004489 | 189 | 148 | 9 | GFTFSNY | 39 | WYEGSE | 63 | DLISGIYYVDH |
| PR004490 | 190 | 149 | 9 | GFTFSNY | 40 | WYDASE | 63 | DLISGIYYVDH |
| PR004491 | 190 | 149 | 9 | GFTFSNY | 40 | WYDASE | 63 | DLISGIYYVDH |
| PR004492 | 190 | 149 | 9 | GFTFSNY | 40 | WYDASE | 63 | DLISGIYYVDH |
| PR004493 | 185 | 138 | 9 | GFTFSNY | 27 | WYDGSE | 63 | DLISGIYYVDH |
| PR004494 | 185 | 138 | 9 | GFTFSNY | 27 | WYDGSE | 63 | DLISGIYYVDH |

Table 1-7-2 Sequence numbers of anti-B7-H7 antibodies (rules of Chothia definition)

| Antibody number | Light chain LC (SEQ ID NO:) | VL (SEQ ID NO:) | LCDR1 (SEQ ID NO:) | Amino acid sequence | LCDR2 (SEQ ID NO:) | Amino acid sequence | LCDR3 (SEQ ID NO:) | Amino acid sequence |
|---|---|---|---|---|---|---|---|---|
| PR003181 | 191 | 150 | 80 | KSSQSLLNSRTRKNQLA | 93 | WASTRES | 110 | KQSYN LRT |
| PR003248 | 192 | 151 | 81 | RASQSINNWLA | 94 | KASSLES | 111 | QQYNSYST |
| PR003249 | 193 | 152 | 82 | RASQSISSWLA | 94 | KASSLES | 112 | QQYNSYSYT |
| PR003250 | 193 | 152 | 82 | RASQSISSWLA | 94 | KASSLES | 112 | QQYNSYSYT |
| PR003251 | 194 | 153 | 83 | RASQSVSSNLA | 95 | GASTRAT | 113 | QQYNIWPYΓ |
| PR003252 | 195 | 154 | 82 | RASQSISSWLA | 96 | KASSLKS | 114 | LQYNSYYT |
| PR003254 | 196 | 155 | 84 | RASQSVSSYLA | 95 | GASTRAT | 115 | QQYNNWPL T |
| PR003259 | 197 | 156 | 85 | RASQGISNYLA | 97 | AASTLQS | 116 | QKYNSAPFT |
| PR003260 | 198 | 157 | 83 | RASQSVSSNLA | 98 | YASTRAP | 113 | QQYNIWPYT |

(continued)

| Antibody number | Light chain LC (SEQ ID NO:) | VL (SEQ ID NO:) | LCDR1 (SEQ ID NO:) | Amino acid sequence | LCDR2 (SEQ ID NO:) | Amino acid sequence | LCDR3 (SEQ ID NO:) | Amino acid sequence |
|---|---|---|---|---|---|---|---|---|
| PR003261 | 199 | 158 | 82 | RASQSISSW LA | 94 | KASSLES | 117 | QQYNSYSVT |
| PR003262 | 200 | 159 | 86 | RASQSFSNN LA | 95 | GASTRAT | 118 | HQYNIWPLT |
| PR003263 | 201 | 160 | 83 | RASQSVSSN LA | 99 | GASTGAT | 119 | QQYYKWPLT |
| PR003264 | 202 | 161 | 87 | RASQGISSYF A | 97 | AASTLQS | 120 | QHLNRYPW T |
| PR003265 | 203 | 162 | 88 | RASQSISYNL A | 100 | GASIRAT | 121 | QQYNNWPP WT |
| PR003266 | 204 | 163 | 82 | RASQSISSW LA | 94 | KASSLES | 111 | QQYNSYST |
| PR004260 | 191 | 150 | 80 | KSSQSLLNSR TRKNQLA | 93 | WASTRES | 110 | KQSYN LRT |
| PR004261 | 193 | 152 | 82 | RASQSISSW LA | 94 | KASSLES | 112 | QQYNSYSYT |
| PR004262 | 197 | 156 | 85 | RASQGISNY LA | 97 | AASTLQS | 116 | QKYNSAPFT |
| PR004263 | 202 | 161 | 87 | RASQGISSYF A | 97 | AASTLQS | 120 | QHLNRYPW T |
| PR004479 | 193 | 152 | 82 | RASQSISSW LA | 94 | KASSLES | 112 | QQYNSYSYT |
| PR004480 | 205 | 164 | 82 | RASQSISSW LA | 94 | KASSLES | 122 | QQYQSYSYT |
| PR004481 | 206 | 165 | 82 | RASQSISSW LA | 94 | KASSLES | 123 | QQYNAYSYT |
| PR004482 | 193 | 152 | 82 | RASQSISSW LA | 94 | KASSLES | 112 | QQYNSYSYT |
| PR004483 | 205 | 164 | 82 | RASQSISSW LA | 94 | KASSLES | 122 | QQYQSYSYT |
| PR004484 | 206 | 165 | 82 | RASQSISSW LA | 94 | KASSLES | 123 | QQYNAYSYT |
| PR004485 | 205 | 164 | 82 | RASQSISSW LA | 94 | KASSLES | 122 | QQYQSYSYT |
| PR004486 | 206 | 165 | 82 | RASQSISSW LA | 94 | KASSLES | 123 | QQYNAYSYT |
| PR004487 | 197 | 156 | 85 | RASQGISNY LA | 97 | AASTLQS | 116 | QKYNSAPFT |
| PR004488 | 207 | 166 | 85 | RASQGISNY LA | 97 | AASTLQS | 124 | QKYQSAPFT |
| PR004489 | 208 | 167 | 85 | RASQGISNY LA | 97 | AASTLQS | 125 | QKYNAAPFT |

(continued)

| Antibody number | Light chain LC (SEQ ID NO:) | VL (SEQ ID NO:) | LCDR1 (SEQ ID NO:) | Amino acid sequence | LCDR2 (SEQ ID NO:) | Amino acid sequence | LCDR3 (SEQ ID NO:) | Amino acid sequence |
|---|---|---|---|---|---|---|---|---|
| PR004490 | 197 | 156 | 85 | RASQGISNY LA | 97 | AASTLQS | 116 | QKYNSAPFT |
| PR004491 | 207 | 166 | 85 | RASQGISNY LA | 97 | AASTLQS | 124 | QKYQSAPFT |
| PR004492 | 208 | 167 | 85 | RASQGISNY LA | 97 | AASTLQS | 125 | QKYNAAPFT |
| PR004493 | 207 | 166 | 85 | RASQGISNY LA | 97 | AASTLQS | 124 | QKYQSAPFT |
| PR004494 | 208 | 167 | 85 | RASQGISNY LA | 97 | AASTLQS | 125 | QKYNAAPFT |

## Example 2. Determination of ability of anti-B7-H7 antibodies of binding to B7-H7 by FACS

This example was intended to study the *in vitro* activity of H2L2 monoclonal antibodies against human B7-H7 in binding to human/cynomolgus monkey B7-H7. Since homologous molecules of B7-H7 were not present in mice, the detection was not performed. A HEK-293 cell strain (HEK-293/hu B7-H7, Harbour BioMed US, Inc.) overexpressing human B7-H7, a HEK-293 cell strain (HEK-293/cyno B7-H7, Harbour BioMed US, Inc.) overexpressing cynomolgus monkey B7-H7, or cell lines HCC827 (*ATCC® CRL-2868*) and LS180 (CoBioer Biosciences Co., Ltd., CBP60034) highly expressing human B7-H7 were used for antibody binding experiments at the cellular level. Briefly, the HEK-293/hu B7-H7 cells, HEK-293/cyno B7-H7 cells, HCC827 cells or LS180 cells were digested and resuspended in PBS containing 2% BSA. The cells were adjusted to a cell density of $1 \times 10^6$ cells/mL, respectively. The cells were seeded in a 96-well V-bottom plate (Corning, # 3894) at 100 μL/well, and the antibody to be tested at 2 × final concentration with 3-fold concentration gradient dilution was then added at 100 μL/well. The cells were incubated at 4°C for 2 hours in the dark. After the incubation, the cells were rinsed twice by adding pre-chilled PBS containing 2% BSA at 100 μL/well, and centrifuged at 500 g at 4°C for 5 minutes, and the supernatant was discarded. A fluorescent secondary antibody (Alexa Fluor 488-conjugated AffiniPure Goat Anti-Human IgG, Fcy Fragment Specific, Jackson, #109-545-098, 1:500 dilution) was added at 100 μL/well, and the cells were incubated at 4°C for 1 hour in the dark. The cells were washed twice by adding pre-chilled PBS containing 2% BSA at 100 μL/well, and centrifuged at 500 g at 4°C for 5 minutes, and the supernatant was discarded. Finally, the cells were resuspended in pre-chilled PBS containing 2% BSA at 200 μL/well, and the fluorescence signal values were read using an ACEA Novocyte3000 flow cytometer.

The binding of the antibodies to human B7-H7 and cynomolgus monkey B7-H7 on the cell surface and to B7-H7 on the surface of tumor cells HCC827 and LS180 is summarized as follows (Table 2-1, Table 2-2, and Table 2-3).

Table 2-1 Binding of anti-B7-H7 antibodies (initial antibodies) to human B7-H7 and cynomolgus monkey B7-H7 on the cell surface and to B7-H7 on the surface of tumor cells HCC827

| | HEK293-hB7-H7 | | HEK293-cynoB7-H7 | | HCC827 | |
|---|---|---|---|---|---|---|
| Antibody | Maximum MFI | EC50 (nM) | Maximum MFI | EC50 (nM) | Maximum MFI | EC50 (nM) |
| PR003248 | 981234 | 1.812 | 698165 | 8.513 | 68608 | 0.03993 |
| PR003249 | 800166 | 0.8811 | 418560 | 0.4117 | 55425 | 0.01507 |
| PR003250 | 822751 | 0.9951 | 404835 | 0.3991 | 68820 | 0.002943 |
| PR003251 | 813378 | 1.068 | 430442 | 0.522 | 53281 | 0.03726 |
| PR003252 | 836597 | 1.043 | 414124 | 0.4294 | 69422 | ~ |
| PR003254 | 762388 | 1.95 | 389065 | 1.241 | 45934 | 0.192 |
| PR003259 | 807637 | 0.945 | 418922 | 0.4211 | 68157 | ~ |

(continued)

| Antibody | HEK293-hB7-H7 | | HEK293-cynoB7-H7 | | HCC827 | |
|---|---|---|---|---|---|---|
| | Maximum MFI | EC50 (nM) | Maximum MFI | EC50 (nM) | Maximum MFI | EC50 (nM) |
| PR003260 | 806680 | 1.132 | 446853 | 0.5884 | 53349 | 0.04389 |
| PR003261 | 723446 | 0.9412 | 274992 | 0.3259 | 60082 | 0.04449 |
| PR003262 | 821530 | 1.329 | 419256 | 0.59 | 53997 | 0.04101 |
| PR003263 | 762945 | 1.064 | 409860 | 0.5559 | 53208 | 0.07892 |
| PR003264 | 756222 | 1.682 | 289909 | 0.8289 | 56951 | 0.6181 |
| PR003265 | 824473 | 1.244 | 450978 | 0.711 | 51768 | 0.09029 |
| PR003266 | 531300 | 0.6631 | 170315 | 0.211 | 49312 | 0.04772 |
| PR003181 | ~ | ~ | ~ | ~ | 25116 | 2.553 |

~ indicating that weaker binding results in a curve that could not be fitted, so that maximum MFI and EC50 could not be calculated.

Table 2-2 Binding of anti-B7-H7 antibodies (variant molecules, LALA mutation) to B7-H7 on the surface of tumor cells LS180

| | LS180 | |
|---|---|---|
| Antibody | Maximum MFI | EC50 (nM) |
| PR003181 | ~ | ~ |
| PR003249 | 21042 | 0.035 |
| PR003259 | 20317 | 0.066 |
| PR003264 | 21767 | 2.198 |
| PR004260 | ~ | ~ |
| PR004261 | 19723 | 0.036 |
| PR004262 | 19225 | 0.048 |
| PR004263 | 23985 | 3.075 |

~ indicating that weaker binding results in a curve that could not be fitted, so that maximum MFI and EC50 could not be calculated.

Table 2-3 Binding of anti-B7-H7 antibodies (variant molecules, PTM removal) to B7-H7 on the surface of tumor cells LS180

| | LS180 | |
|---|---|---|
| Antibody | Maximum MFI | EC50 (nM) |
| PR004261 | 26913 | 0.034 |
| PR004262 | 25881 | 0.033 |
| PR004483 | 25060 | 0.044 |
| PR004484 | 24634 | 0.04 |
| PR004491 | 22835 | 0.066 |
| PR004492 | 22862 | 0.052 |

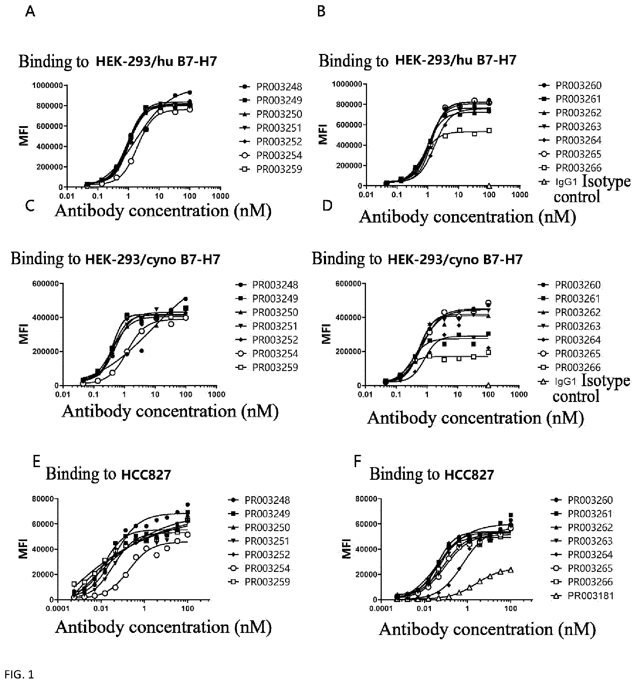

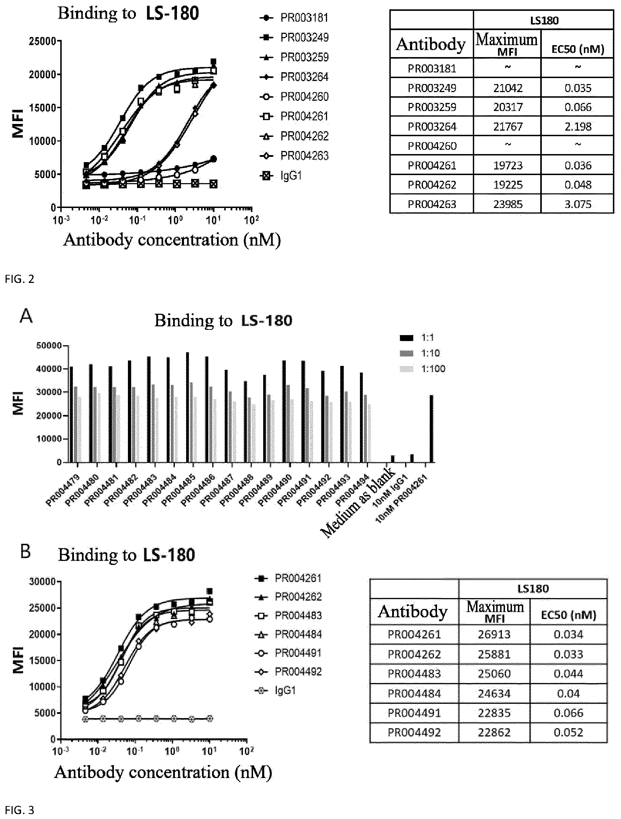

The results of the binding of the initial antibodies to human B7-H7 on the cell surface are as shown in FIG. 1 A-1F. The results show that these initial antibodies have good activity of binding to human and cynomolgus monkey B7-H7, and can bind to B7-H7 on the surface of tumor cells. The Fc termini of PR003181, PR003249, PR003259 and PR003264 were engineered with LALA mutations to remove ADCC effects. The results show that the engineered antibodies PR004260, PR004261, PR004262 and PR004263 are consistent with the initial antibodies in the binding activity to cells (FIG. 2). PTM removal was further performed on PR004261 and PR004262 to obtain antibodies PR004479-PR004494. The FACS results show that their binding activity is substantially consistent with that of the initial antibodies (FIG. 3).

**Example 3. Blocking effect of antibodies against the binding of human B7-H7 protein to HEK293 cells overexpressing human CD28H**

This example was intended to study the *in vitro* blocking activity of human anti-B7-H7 antibodies against the binding of human B7-H7 to its receptor CD28H. A HEK-293 cell strain (HEK-293/CD28H, Harbour BioMed US, Inc.) overexpressing human CD28H was used in experiments at the cellular level where human B7-H7/human CD28H binding was blocked. Briefly, the HEK-293/CD28H cells were digested and resuspended in PBS containing 2% BSA. The cells were adjusted to a cell density of $1 \times 10^6$ cells/mL. The cells were seeded in a 96-well V-bottom plate (Corning, Cat#: 3894) at 100 μL/well, and an antigen-binding protein to be tested at $2 \times$ final concentration with 3-fold concentration gradient dilution was then added at 100 μL/well, and uniformly mixed, with the maximum final concentration of the antigen-binding protein of 100 nM, a total of 8 concentrations, and hIgG as the control. The cells were incubated at 4°C for 1 hour in the dark. After the incubation, the cells were centrifuged at 4°C for 5 minutes, the supernatant was discarded, then a biotinylated human B7-H7 protein (Aero Biosystems, B77-H82F5) with a concentration of 1 μg/mL was added at 50 μL/well, and the cells were incubated at 4°C for 30 minutes in the dark. The cells were rinsed twice by adding pre-chilled PBS containing 2% BSA at 100 μl/well, and were centrifuged at 500 g at 4°C for 5 minutes, and the supernatant was discarded. A fluorescent secondary antibody (PE Streptavidin, BD, Cat#: 554061, 1:200) was added at 100 μL/well, and the cells were incubated at 4°C for 30 minutes in the dark. The cells were washed twice by adding pre-chilled PBS at 200 μL/well, and centrifuged at 500 g at 4°C for 5 minutes, and the supernatant was discarded. Finally, the cells were resuspended in pre-chilled PBS at 200 μL/well, and the fluorescence signal values were read using an ACEA Novocyte3000 flow cytometer. IC50 was calculated.

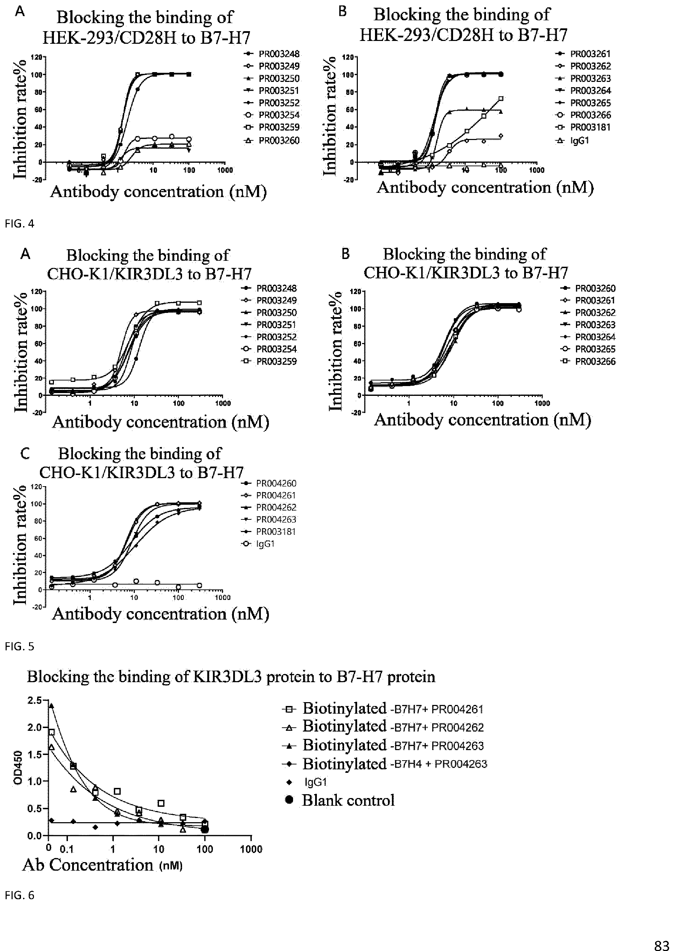

The results are as shown in FIG. 4 and Table 3, indicating that the blocking efficiency of PR003251, PR003254, PR003260, PR003262, PR003263 and PR003181 is acceptable, and the other antibodies can completely block the binding of B7-H7 protein to its receptor CD28H, all of which are superior to the positive control PR003181.

Table 3 Blocking effect of antibodies against the binding of human B7-H7 to human CD28H on the cell surface

| Antibody | Maximum inhibition rate (%) | IC50 (nM) |
|---|---|---|
| PR003248 | 100.8 | 1.87 |
| PR003249 | 101.1 | 1.377 |
| PR003250 | 100.6 | 1.405 |
| PR003251 | 16.23 | 1.241 |
| PR003252 | 100.7 | 1.434 |
| PR003254 | 27.44 | 1.589 |
| PR003259 | 101.2 | 1.428 |
| PR003260 | 20.44 | 2.474 |
| PR003261 | 100.4 | 1.493 |
| PR003262 | 26.36 | 3.372 |
| PR003263 | 59.29 | 1.7 |
| PR003264 | 101 | 1.516 |
| PR003265 | 102.1 | 1.388 |
| PR003266 | 100.6 | 1.566 |
| PR003181 | ~ | 71.67 |

**Example 4. Blocking effect of antibodies against the binding of human B7-H7 protein to human KIR3DL3 protein and to CHO-K1 cells overexpressing KIR3DL3**

**Example 4.1. Blocking effect of antibodies against the binding of human B7-H7 protein to KIR3DL3 expressed on the cell surface**

This example was intended to study the *in vitro* blocking activity of human anti-B7-H7 antibodies against the binding of human B7-H7 to its receptor KIR3DL3. A CHO-K1 cell (CHO-K1/KIR3DL3, Harbour BioMed US, Inc.) overexpressing human KIR3DL3 was used in experiments at the cellular level where human B7-H7/human KIR3DL3 binding was blocked. Briefly, the CHO-K1/KIR3DL3 cells were digested and resuspended in PBS containing 2% BSA. The cells were adjusted to a cell density of $1 \times 10^6$ cells/mL. The cells were seeded in a 96-well V-bottom plate (Corning, Cat#: 3894) at 100 µl/well, and an antigen-binding protein to be tested at $2 \times$ final concentration with 3-fold concentration gradient dilution was then added at 100 µL/well, and uniformly mixed, with the maximum final concentration of the antigen-binding protein of 100 nM, a total of 8 concentrations, and hIgG as the control. The cells were incubated at 4°C for 1 hour in the dark. After the incubation, the cells were centrifuged at 4°C for 5 minutes, the supernatant was discarded, then a biotinylated human B7-H7 protein (Acro Biosystems, B77-H82F5) with a concentration of 1 µg/mL was added at 50 µL/well, and the cells were incubated at 4°C for 30 minutes in the dark. The cells were rinsed twice by adding pre-chilled PBS containing 2% BSA at 100 µL/well, and were centrifuged at 500 g at 4°C for 5 minutes, and the supernatant was discarded. A fluorescent secondary antibody (PE Streptavidin, BD, Cat#: 554061, 1 : 200) was added at 100 µL/well, and the cells were incubated at 4°C for 30 minutes in the dark. The cells were washed twice by adding pre-chilled PBS at 200 µL/well, and centrifuged at 500 g at 4°C for 5 minutes, and the supernatant was discarded. Finally, the cells were resuspended in pre-chilled PBS at 200 µL/well, and the fluorescence signal values were read using an ACEA Novocyte3000 flow cytometer. IC50 was calculated.

The results are as shown in FIG. 5 and Table 4, indicating that all antibodies, except for the control IgG, are shown to completely block the binding of B7-H7 protein to its receptor KIR3DL3.

Table 4 Blocking effect of antibodies against the binding of human B7-H7 to human KIR3DL3 on the cell surface

| Antibody | Maximum inhibition rate (%) | IC50 (nM) |
|---|---|---|
| PR003248 | 99.32 | 12.91 |
| PR003249 | 97.52 | 5.068 |
| PR003250 | 97.24 | 8.487 |
| PR003251 | 96.38 | 6.341 |
| PR003252 | 97.13 | 7.594 |
| PR003254 | 97.24 | 7.18 |
| PR003259 | 107.9 | 7.809 |
| PR003260 | 106 | 6.464 |
| PR003261 | 105.3 | 7.497 |
| PR003262 | 105 | 9.928 |
| PR003263 | 102.9 | 6.23 |
| PR003264 | 104.1 | 8.563 |
| PR003265 | 100.6 | 7.045 |
| PR003266 | 101.7 | 8.548 |
| PR003181 | 96.7 | 10.36 |
| PR004260 | 96.23 | 8.507 |
| PR004261 | 101.2 | 6.969 |
| PR004262 | 100.5 | 6.537 |
| PR004263 | 99.93 | 8.767 |

**Example 4.2. Blocking effect of antibodies against the binding of human B7-H7 protein to human KIR3DL3 protein**

Human KIR3DL3 (Sino Biological, Cat# 16055-H08H) was diluted with PBS to 5 µg/ml, added to a 96-well plate (Corning, cat# 9018) at 100 µl per well, and incubated at 4ºC overnight. After the liquid was discarded, the plate was washed 3 times with a PBS buffer, 200 µl of PBS blocking solution containing 1% BSA was added, and the plate was incubated for 1 hour at room temperature. The blocking solution was discarded, and the plate was washed 3 times with a PBS buffer. Different concentrations of the antibodies to be tested were added along with 6 µg/ml biotin-B7H7 protein (Acro Biosystems, Cat# B77-H82F5) or Biotin-B7H4 protein (Acro Biosystems, Cat# B74-H82E2). The Biotin-B7H4 protein was a negative control protein that did not bind to KIR3DL3. The antibodies to be tested were subjected to 3-fold concentration dilution from 100 nM, with a total of 8 gradient concentrations. 100 μ! of the diluted antibody was added to each well, and the plate was incubated at 4°C overnight. After the plate was washed 3 times with a PBS buffer, StrepTactin-HRP Conjugate (Bio-rad, Cat# 161-0380) diluted 500 times was added, and the plate was incubated at 37°C for 1 hour in the dark. After the plate was washed 4 times with a PBS buffer, TMB (KPL SureBlue, Cat# 5120-0077) was added at 100 μl/well, and the plate was placed at room temperature for about 10 minutes in the dark; and a stop solution (BBI life sciences, Cat# E661006-0200) was added to each well at 100 μl/well to stop the reaction, and the absorbance at 450 nm was determined in a microplate reader (PerkinElemer # Enspire).

The results are as shown in FIG. 6, indicating that B7-H7 protein can bind to KIR3DL3 protein in the absence of the antibodies, whereas B7-H4 protein does not bind to KIR3DL3 protein; and the antibodies PR004261, PR004262 and PR004263 to be tested can dosedependently block the binding of B7-H7 protein to KIR3DL3 protein.

## Example 5. Determination of T cell activation activity

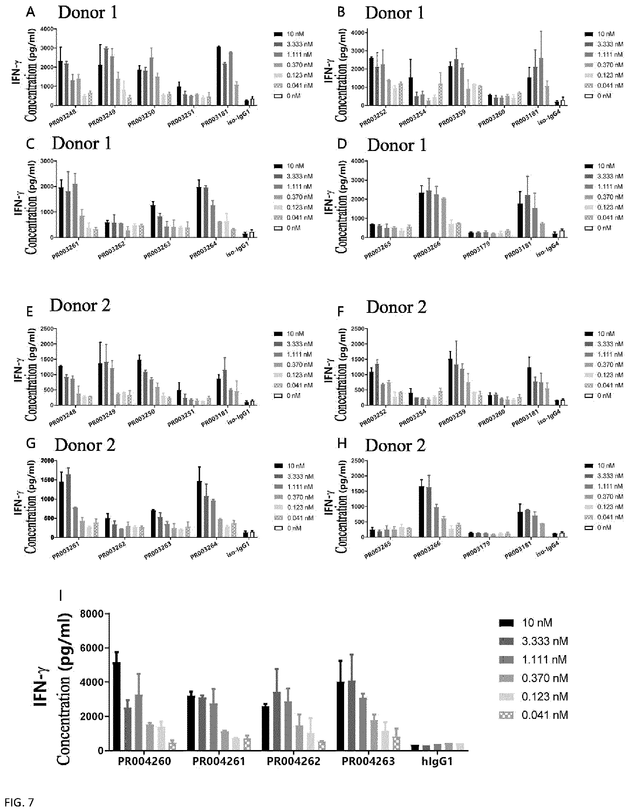

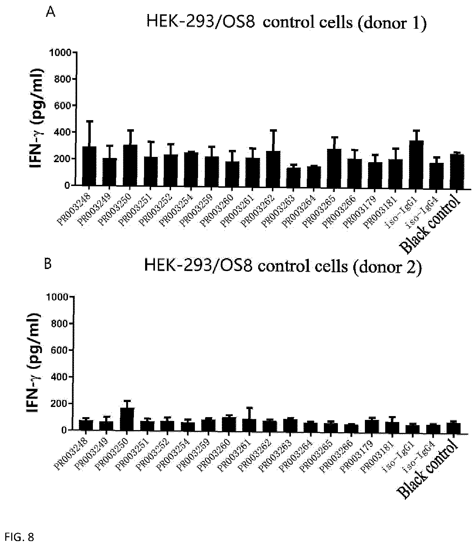

To detect the function of the anti-B7-H7 antibody in blocking T cell immune checkpoints and thus activating T cells, in this experiment, CHO-K1 cells overexpressing full-length B7-H7 and anti-human CD3 antibody OKT3 in the form of scFv were used as artificial antigen-presenting cells (CHO-K1/OS8/hB7-H7, KYinno Biotechnology Co., Ltd.) and negative control cells (CHO-K1/OS8, KYinno Biotechnology Co., Ltd.), respectively, T cells were isolated using a human T cell isolation kit (Miltenyi, # 130-096-535) according to the method in the instructions, and the artificial antigen-presenting cells were co-cultured with the T cells, so as to detect the effect of the anti-B7-H7 antibody on T cell activation. Specifically, CHO-K1-OS8-hB7-H7 or control cells CHO-K1-OS8 were plated at a density of $1 \times 10^4$ cells/well and cultured overnight. Human primary T cells were isolated and added to CHO-K1-OS8-hB7-H7 cells at a density of $2 \times 10^5$ cells/100 μl/well. Subsequently, the antibody to be tested at 2 × final concentration with 3-fold concentration gradient dilution was added at 100 μL/well, with the maximum final concentration of the antibody of 10 nM, a total of 6 concentrations for each antibody, two replicates set for each concentration. After culturing for 3 days, the supernatant was collected, and the concentration of IFN-y was detected by ELISA. The results are as shown in FIG. 7. FIG. 7A-FIG. 7H show that the antibodies PR003248, PR003249, PR003250, PR003252, PR003259, PR003261, PR003264 and PR003266 and the control antibody have a strong activity of promoting T cell activation to secrete IFN-γ (where donor 1 in FIG. 7A-7D, and donor 2 in FIG. 7E-7H), and FIG. 7I shows that the antibodies PR004260, PR004261, PR004262 and PR004263 also have a strong activity of promoting T cells to secrete IFN-y. If the artificial antigen-presenting cells do not express B7-H7, the antibodies do not show the activity of promoting T cell activation, indicating that the effect of the antibodies is B7-H7-specific (FIG. 8 shows that the anti-B7-H7 antibodies do not act on B7-H7 negative cells and thus cannot activate T cells).

## Example 6. Determination of NK cell killing activity

To detect the function of the anti-B7-H7 antibody in activating NK cells, in this experiment, MDA-MB-231 cells overexpressing full-length B7-H7 were used as target cells (MDA-MB-231/hB7-H7, Harbour BioMed US, Inc.), NK cells were isolated using a human NK cell isolation kit (Miltenyi, # 130-092-657) according to the method in the instructions, and the isolated and purified NK cells were activated with 100 IU/ml interleukin-2 for 2 days. The target cells MDA-MB-231/hB7-H7 were digested with trypsin and stained with 1 uM cell tracer dye CFSE at room temperature for 10 minutes in the dark. The activated NK cells were co-cultured with the target cells, the anti-B7-H7 antibody to be tested was added, and the cells were incubated in an incubator at 37°C for 4 h. After the incubation was completed, 2 μ! of 7AAD dead cell dye was added to each well, and the cells were uniformly mixed using a multi-channel pipette, and analyzed within 10 minutes on a flow cytometry sorter BD Cantoll to detect the killing on the target cells. (Killed target cells (%)=cells positive for both 7AAD staining and CFSE staining/cells positive for CFSE staining)

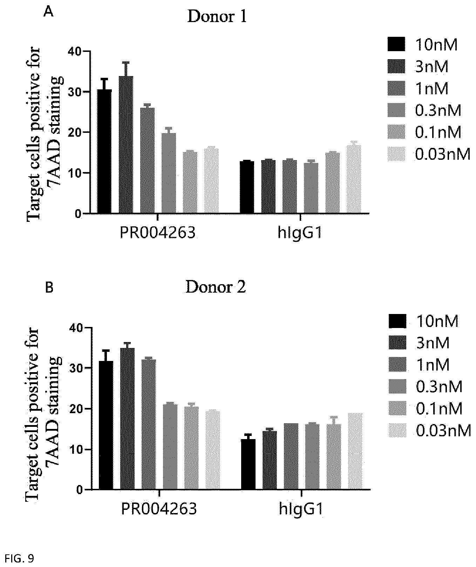

The results are as shown in FIG. 9, indicating that the antibody PR004263 significantly stimulates the NK cells to kill the target cells in a dose-dependent manner compared with the isotype control.

## Example 7. Determination of affinity of anti-B7-H7 antibodies for human B7-H7 recombinant protein

## Determination of affinity by BLI

10 × kinetics buffer (ForteBio, # 18-1105) was diluted to 1 × kinetics buffer for affinity testing and dilution of antigens and antibodies. The binding kinetics between antigen and antibody was analyzed using an Octet Red 96e (Fortebio) molecular interaction analyzer by biolayer interferometry (BLI) technology.

For determining the affinity of antibodies for antigens, the rotary speed of the sensor was set at 1000 rpm. AHC sensors (Fortebio, # 18-5060) placed in one column were first equilibrated in a test buffer for 10 minutes, and then used to capture the anti-B7-H7 antibodies at a capture height of 0.7 nm; and after being equilibrated in the buffer for 120 s, the AHC sensors bound to human B7-H7 (concentrations: 50-3.125 nM and 0 nM) with 2-fold gradient dilution for 180 s and dissociated for 300 s. The AHC sensors were finally regenerated by immersing in a 10 mM glycine-hydrochloric acid solution with pH 1.5 to elute the protein bound to the sensors.

For data analysis using Octet Data Analysis software (Fortebio, version 11.0), 0 nM well was used as the reference well, reference subtraction was performed, "1 : 1 Global fitting" method was selected for data fitting, the kinetic parameters of the binding of the antigen to the antigen-binding protein were calculated, and the $k_{on}$ (1/Ms) value, $k_{dis}$ (1/s) value and $K_D$ (M) value were obtained. The results are as shown in Table 5.

Table 5 Binding affinity of anti-B7-H7 antibodies for human B7-H7 protein (BLI)

| Antibody | Concentration (nM) | KD (M) | kon (1/Ms) | kdis (1/s) | Full R^2 |
|----------|--------------------|--------|-----------|-----------|----------|
| PR003248 | 100 | 1.60E-09 | 8.80E+04 | 1.40E-04 | 0.9986 |
| PR003249 | 100 | 1.83E-09 | 9.70E+04 | 1.78E-04 | 0.9989 |
| PR003250 | 100 | 2.91E-09 | 9.32E+04 | 2.71E-04 | 0.998 |
| PR003251 | 100 | 6.70E-09 | 9.43E+04 | 6.32E-04 | 0.9969 |
| PR003252 | 100 | 1.06E-09 | 1.25E+05 | 1.32E-04 | 0.9975 |
| PR003254 | 100 | 3.23E-08 | 7.56E+04 | 2.45E-03 | 0.9742 |
| PR003259 | 100 | 1.64E-09 | 7.53E+04 | 1.24E-04 | 0.9984 |
| PR003260 | 100 | 1.68E-09 | 6.11E+04 | 1.02E-04 | 0.9989 |
| PR003261 | 100 | 4.60E-09 | 7.30E+04 | 3.36E-04 | 0.998 |
| PR003262 | 100 | 1.55E-09 | 7.55E+04 | 1.17E-04 | 0.9985 |
| PR003263 | 100 | 5.44E-09 | 8.11E+04 | 4.41E-04 | 0.9982 |
| PR003264 | 100 | 2.94E-09 | 3.90E+04 | 1.15E-04 | 0.998 |
| PR003265 | 100 | 5.95E-10 | 4.93E+04 | 2.93E-05 | 0.9994 |
| PR003266 | 100 | 9.93E-09 | 6.52E+04 | 6.48E-04 | 0.9985 |
| PR003181 | 100 | 2.63E-08 | 7.81E+04 | 2.05E-03 | 0.9737 |

**Example 8. Determination of competition of anti-B7-H7 antibodies for binding to B7-H7 epitopes by BLI**

Epitope competition experiments were performed on the anti-B7-H7 antibodies using the ForteBio Octet Red96e platform with the same experimental buffer as described above. First step, acquisition of 100% signal of the antibody: B7-H7 (Aero Biosystems, B77-H82F5) was captured using SA sensors (Fortebio, #18-5019) at a capture height of 0.25 nm. After being equilibrated in a buffer for 120 s, the sensors were immersed in various antibodies diluted to 100 nM for 240 s, and the final signal of the antibody binding to B7-H7 was recorded as the 100% signal for that antibody. Second step, epitope competition experiment: B7-H7 was captured using SA sensors at a capture height of 0.25 nm. The sensors were immersed in a first antibody (concentration: 100 nM) for 240 s, then the SA sensors were immersed in a mixture of the first antibody and a second antibody (final concentration of both antibodies: 100 nM) for 240 s, and the difference in signals before and after immersing the sensors in the mixture of the two antibodies was recorded as the signal for that antibody as the second antibody. The inhibition rate was calculated by the following equation,

$$\text{Inhibition rate (\%)} = (A-B)/A * 100$$

A: 100% signal (obtained from the first step) of a certain antibody, B: signal (obtained from the second step) of this antibody as the second antibody.

If the resulting inhibition rate is greater than 85(%), it means that the epitopes to which the two antibodies bind completely overlap; and if the inhibition rate is less than 85(%), it means that the epitopes to which the two antibodies bind do not completely overlap.

First, PR003249 was used as the first antibody, and the other antibodies were each used as the second antibody to detect the competition between the first antibody and the second antibody (PR003249 in the second antibody included 3 replicates). The results in Table 6-1 show that the second antibodies PR003248, PR003250, PR003252, PR003259, PR003261, PR003264, PR003265 and PR003266 completely compete with the first antibody PR003249, indicating that the first binding epitope to which they bind is the same; and the epitopes to which the control antibody PR003181 binds partially overlap with the first binding epitope. For the remaining antibodies that bind to other different epitopes, PR003251 was used as the first antibody, and the other antibodies were each used as the second antibody to detect the competition between the first antibody and the second antibody. The results in Table 6-2 show that PR003251, PR003254, PR003260, PR003262 and PR003263 completely compete with the first antibody, indicating that the second binding epitope to which they bind is the same; and the epitopes to which the control antibody PR003181 bind do not overlap with the second binding epitope. B7-H7 epitopes to which the antibodies bind are summarized in Tables 6-3.

Table 6-1 Competitive analysis of antibodies for binding to B7-H7 in the first round

| | Competitive inhibition rate (%) | First antibody |
|---|---|---|
| | | **PR003249** |
| Second antibody | PR003249 | 106.54481 |
| | PR003248 | 107.97038 |
| | PR003250 | 103.47287 |
| | PR003251 | -6.5446 |
| | PR003252 | 107.27535 |
| | PR003254 | -5.9583 |
| | PR003249 | 105.63873 |
| | PR003259 | 109.54106 |
| | PR003260 | -3.1776 |
| | PR003261 | 110.5473 |
| | PR003262 | -2.5972 |
| | PR003263 | -0.3244 |
| | PR003264 | 108.85338 |
| | PR003249 | 107.16221 |
| | PR003265 | 106.20329 |
| | PR003266 | 107.39825 |
| | PR003181 | 77.01487 |

Table 6-2 Competitive analysis of antibodies for binding to B7-H7 in the second round

| | Competitive inhibition rate (%) | First antibody |
|---|---|---|
| | | PR003251 |
| Second antibody | PR003251 | 97.890716 |
| | PR003254 | 99.09022 |
| | PR003260 | 100.4908 |
| | PR003262 | 96.392817 |
| | PR003263 | 96.938776 |
| | PR003180 | 3.21033 |
| | PR003181 | 5.3217 |

Table 6-3 Summary of B7-H7 epitopes to which antibodies bind

| | |
|---|---|
| PR003249 PR003248 PR003250 PR003252 PR003259 PR003261 PR003264 PR003265 PR003266 | Epitope 1 |
| PR003181 | Epitope 1 (partially overlapped) |
| PR003251 PR003254 PR003260 PR003262 PR003263 | Epitope 2 |

## Example 9. Cross-reactivity to other members of the B7 family

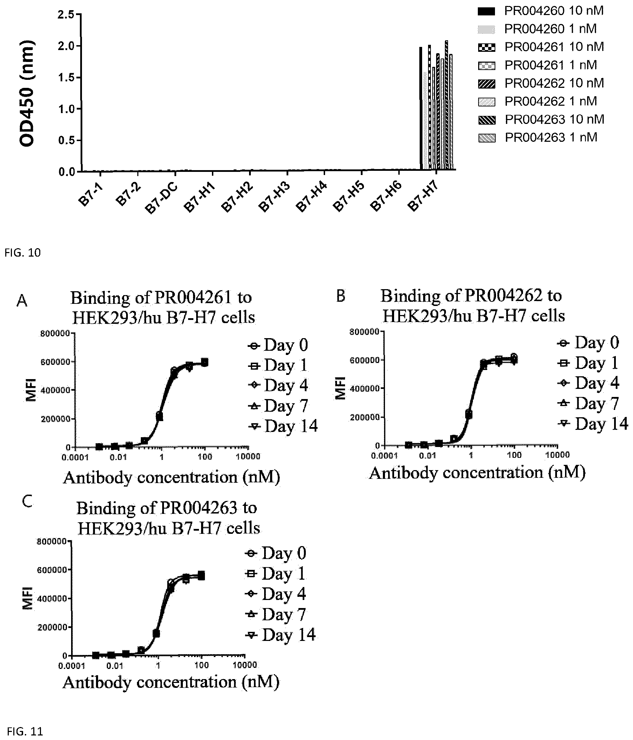

B7 family proteins (see Table 7 for details) were respectively diluted to 1 μg/mL with PBS, added into a 96-well plate (Corning, # 9018) at 100 μl/well, and incubated at 4°C overnight. After the liquid was discarded, the plate was washed 3 times with a PBST buffer (pH 7.4, containing 0.05% tween-20), 250 μl of blocking solution containing 2% BSA was added, and the plate was incubated for 1 hour at 37°C. The blocking solution was discarded, the plate was washed 3 times with PBST buffer (pH 7.4, containing 0.05% Tween-20), the antigen-binding protein to be tested was diluted to 2 concentrations of 10 nM and 1 nM, 100 μl of the diluted antigen-binding protein was added to each well, and the plate was incubated for 1 hour at 37°C, with the isotype antibody as the control. After the plate was washed 3 times with PBST buffer (pH 7.4, containing 0.05% Tween-20), goat anti-human HRP secondary antibody (Invitrogen, # A18805) diluted 5000 times was added, and the plate was incubated at 37°C for 1 hour in the dark. After the plate was washed 3 times with PBST buffer (pH 7.4, containing 0.05% tween-20), TMB (Biopanda, # TMB-S-003) was added at 100 μl/well, and the plate was placed at room temperature for about 30 minutes in the dark; and a stop solution (BBI life sciences, #E661006-0200) was added to each well at 50 μl/well to stop the reaction, and the absorbance at 450 nm (OD450) was determined in a microplate reader (PerkinElemer, #Enspire). FIG. 10 shows that the antibodies of the present invention only specifically binds to B7-H7 protein without cross-reacting with other member proteins of the B7 family.

Table 7 Material information of B7 family proteins used in this example

| Other members of the B7 family | Supplier | Catalog No. |
|---|---|---|
| Human B7-1/CD80 protein, Fc tag (HPLC validated) | Aero | B71-H5259 |
| Human B7-2/CD86 protein, Fc tag | Acro | CD6-H5257 |
| Human B7-DC/PD-L2/CD273 protein, recombinant (Fc tag) | Sino Biological | H10292-H02H |
| Human PD-L1/B7-H1 protein, His tag (HPLC validated) | Aero | PD1-H5229 |
| CD275/ICOS ligand protein, human, recombinant (Fc tag) | Sino Biological | 11559-H02H |
| B7-H3/CD276 protein, human, recombinant (ECD, Fc tag) | Sino Biological | 11188-H02H |
| Recombinant human B7H4 Fc chimera | R&D | 8870-B7-050 |
| Recombinant human VISTA/B7H5/PD-1H Fc chimera | R&D | 7126-B7-050 |
| B7-H6 /NCR3LG1 protein, human, recombinant (Fc tag) | Sino Biological | 16140-H02H |
| Recombinant human B7-H7/HHLA2 Fc chimera | R&D | 8084-B7-050 |

## Example 10. Analysis of stability in sera

30 μl of the antibody was taken and diluted with 270 μl of normal human sera (concentration in serum: 90%), the diluted antibody was divided into 5 parts, the 5 parts were incubated at 37°C for 0, 1, 4, 7 and 14 days, respectively, then taken out, snap-frozen with liquid nitrogen, and stored at -80°C. The binding of the antibody to B7-H7 on HEK-293/hB7-H7 cells was detected by flow cytometry. The HEK-293/hB7-H7 cells were digested and resuspended in PBS containing 2% BSA. The cells were adjusted to a cell density of $1 \times 10^6$ cells/mL, respectively. The cells were seeded in a 96-well V-bottom plate (Corning, # 3894) at 100 μL/well, and the antibody to be tested at $2 \times$ final concentration with 3-fold concentration gradient dilution was then added at 100 μL/well. The cells were incubated at 4°C for 2 hours in the dark. After the incubation, the cells were rinsed twice by adding pre-chilled PBS containing 2% BSA at 100 μL/well, and centrifuged at 500 g at 4°C for 5 minutes, and the supernatant was discarded. A fluorescent secondary antibody (Alexa Fluor 488-conjugated AffiniPure Goat Anti-Human IgG, Fcγ Fragment Specific, Jackson, #109-545-098, 1 : 500 dilution) was added at 100 μL/well, and the cells were incubated at 4°C for 60 minutes in the dark. The cells were washed twice by adding pre-chilled PBS containing 2% BSA at 100 μL/well, and centrifuged at 500 g at 4°C for 5 minutes, and the supernatant was discarded. Finally, the cells were resuspended in pre-chilled PBS containing 2% BSA at 200 μL/well, and the fluorescence signal values were read using an ACEA Novocyte3000 flow cytometer. The results in FIG. 11 show that PR004261, PR004262 and PR004263 have good stability in human sera at 37°C for 14 days.

## Example 11. Anti-tumor efficacy of anti-B7-H7 antibodies NCG mouse MDA-MB-231 tumor model having reconstituted human PBMC immune system

On the day of cell inoculation, each NCG mouse was subcutaneously inoculated with $5 \times 10^6$ MDA-MB-231 tumor cells (MDA-MB-231/hB7-H7 cells) overexpressing human B7-H7, wherein the cells were resuspended in a PBS and Matrigel (1:1) mixed solution (0.1 mL/mouse), and subcutaneous inoculation was performed. The mice were grouped when the mean tumor volume was about 90 mm³, 24 mice were divided into 4 groups, and each mouse was intravenously inoculated with $5 \times 10^6$ human PBMCs, wherein the cells were resuspended in 200 μl of PBS. The administration was started on the next day, the administration cycle was 4 weeks in total with an administration frequency of twice a week, tail vein administration was used, and the efficacy of the antibodies PR004261, PR004262 and PR004263 at a dose of 20 MPK was tested. After the administration was started, the body weight was weighed and the tumor volume was measured twice a week. The tumor volume was calculated as follows: Tumor volume ($mm^3$) = $0.5 \times$ tumor long diameter $\times$ tumor short diameter$^2$. Experimental observations were ended on day 36 after administration, and all mice were subsequently euthanized.

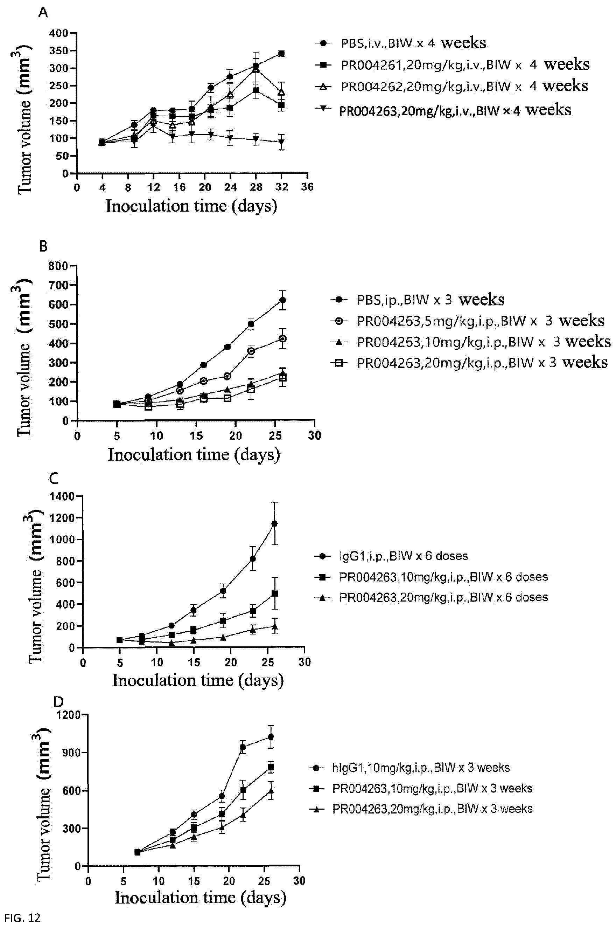

For the *in vivo* anti-tumor effects in the NCG mouse MDA-MB-231/hB7-H7 tumor model having reconstituted human PBMC immune system, see FIG. 12A, and specifically, the mean tumor volume of the mice in the vehicle control group on day 32 after administration is 339 $mm^3$. The mean tumor volume in the treatment group using the test drug PR004261 (20 mg/kg) on day 32 after administration is 192 $mm^3$, which is significantly different (p value < 0.0001) from that in the vehicle control group, with the tumor inhibition rate TGI (%) of 43.43%. The mean tumor volume in the treatment group using the test drug PR004262 (20 mg/kg) on day 32 after administration is 229 $mm^3$, which is significantly different (p value of 0.0049) from that in the vehicle control group, with the tumor inhibition rate TGI (%) of 32.52%. The mean tumor volume in the treatment group using the test drug PR004263 (20 mg/kg) on day 32 after administration is 86 $mm^3$, which is significantly different (p value < 0.0001) from that in the vehicle control group, with the tumor inhibition rate TGI (%) of 74.57%.

In the second experiment, a similar tumor inoculation regimen was performed, the administration cycle was a 3 weeks in total with an administration frequency of twice a week, intraperitoneal administration was used, and the efficacy of the antibody PR004263 at 3 doses of 5 MPK, 10 MPK and 20 MPK was tested. After the administration was started, the body weight was weighed and the tumor volume was measured twice a week. The tumor volume was calculated as follows: Tumor volume ($mm^3$) = $0.5 \times$ tumor long diameter $\times$ tumor short diameter$^2$. Experimental observations were ended on day 36 after administration, and all mice were subsequently euthanized.

For the in vivo anti-tumor effect in the tumor models, see FIG. 12B, and specifically, the mean tumor volume of the mice in the vehicle control group on day 26 after inoculation is 618 $mm^3$. The mean tumor volume in the treatment group using the test drug PR004263 (5 mg/kg) on day 26 after inoculation is 420 $mm^3$, which is significantly different (p value < 0.0198) from that in the vehicle control group, with the tumor inhibition rate TGI (%) of 32.1%. The mean tumor volume in the treatment group using the test drug PR004263 (10 mg/kg) on day 26 after inoculation is 243 $mm^3$, which is significantly different (p value of 0.0001) from that in the vehicle control group, with the tumor inhibition rate TGI (%) of 60.65%. The mean tumor volume in the treatment group using the test drug PR004263 (20 mg/kg) on day 26 after inoculation is 219 $mm^3$, which is significantly different (p value < 0.0001) from that in the vehicle control group, with the tumor inhibition rate TGI (%) of 64.52%.

### NCG mouse NUGC-4 tumor model having reconstituted human PBMC immune system and NCG mouse HCC-827 tumor model having reconstituted human PBMC immune system

Both NUGC-4 tumor cells and HCC-827 tumor cells express B7-H7 endogenously. A NCG mouse NUGC-4 tumor model having reconstituted human PBMC immune system was used for *in vivo* pharmacodynamic studies. The specific method was as follows: on the day of cell inoculation, each NCG mouse was subcutaneously co-inoculated with 5 $\times 10^6$ NUGC-4 tumor cells and 1 $\times 10^6$ human PBMCs, i.e., subcutaneous inoculation of mixed cells. When the mean tumor volume of the mice in each group was 70 $mm^3$, grouping and administration were performed, with a total of 6 administrations via intraperitoneal injection. After the administration was started, the body weight was weighed and the tumor volume was measured twice a week. The tumor volume was calculated as follows: Tumor volume ($mm^3$) = $0.5 \times$ tumor long diameter $\times$ tumor short diameter$^2$. Data analysis was performed using t-test. For the *in vivo* anti-tumor effects in the NCG mouse NUGC-4 tumor model having reconstituted human PBMC immune system, see FIG. 12C, and specifically, the mean tumor volume of the mice in the antibody control group on day 26 after administration is 1140.62 $mm^3$. The mean tumor volume in the treatment group using the test drug PR004263 (10 mg/kg) on day 26 after administration is 491.38 $mm^3$, which is significantly different (p value < 0.05) from that in the vehicle control group, with the tumor inhibition rate TGI (%) of 56.92%. The mean tumor volume in the treatment group at 20 mg/kg on day 26 after administration is 191.99 $mm^3$, which is significantly different (p value < 0.01) from that in the vehicle control group, with the tumor inhibition rate TGI (%) of 83.17%.

A NCG mouse HCC-827 tumor model having reconstituted human PBMC immune system was used for *in vivo* pharmacodynamic studies. The specific method was as follows: on the day of cell inoculation, each NCG mouse was subcutaneously inoculated with 1 $\times 10^7$ HCC-827 cells. The mice were grouped when the mean tumor volume was about 110 $mm^3$, each mouse was intravenously inoculated with 5 $\times 10^6$ human PBMCs, and the administration was started on the next day. The administration cycle was 5 administrations with an administration frequency of twice a week, and the mode of administration was intraperitoneal injection. After the administration was started, the body weight was weighed and the tumor volume was measured twice a week. The tumor volume was calculated as follows: Tumor volume ($mm^3$) = $0.5 \times$ tumor long diameter $\times$ tumor short diameter$^2$. Experimental observations were ended on day 26 after administration, and all mice were subsequently euthanized. For the *in vivo* anti-tumor effects in the NCG mouse HCC-827 tumor model having reconstituted human PBMC immune system, see FIG. 12D, and specifically, the mean tumor volume of the mice in the antibody control group on day 26 after administration is 1020.74 mm$^3$. The mean tumor volume in the treatment group using the test drug PR004263 (10 mg/kg) on day 26 after administration is 778.35 mm$^3$, which is significantly different (p value < 0.05) from that in the vehicle control group, with the tumor inhibition rate TGI (%) of 23.75%. The mean tumor volume in the treatment group at 20 mg/kg on day 26 after administration is 597.69 mm$^3$, which is significantly different (p value < 0.01) from that in the vehicle control group, with the tumor inhibition rate TGI (%) of 41.45%.

**Claims**

1. An anti-B7-H7 antibody or an antigen-binding fragment thereof, wherein the antibody or the antigen-binding fragment thereof comprises a light chain variable region (VL) and a heavy chain variable region (VH); wherein

   the VL comprises complementarity determining regions (CDRs) as follows or mutations thereof: LCDR1 set forth in an amino acid sequence of SEQ ID NO: 82, LCDR2 set forth in any one of amino acid sequences of SEQ ID NOs: 94-100, and LCDR3 set forth in any one of amino acid sequences of SEQ ID NOs: 111, 112, 114-116, 120 and 121;
   the VH comprises CDRs as follows or mutations thereof: HCDR1 set forth in an amino acid sequence of SEQ ID NO: 7, HCDR2 set forth in an amino acid sequence of SEQ ID NO: 34 or 36, and HCDR3 set forth in any one of amino acid sequences of SEQ ID NOs: 57-68;
   wherein the mutation is 3, 2 or 1 amino acid insertion, deletion or substitution in the amino acid sequence of the CDR.

2. The anti-B7-H7 antibody or the antigen-binding fragment thereof of claim 1, wherein the mutation in LCDR1 is 3, 2 or 1 amino acid substitution of SSG, 16V/F, S7N, S8N/Y, W9N/Y and L10F in the amino acid sequence set forth in SEQ ID NO: 82; the amino acid sequence is preferably set forth in any one of SEQ ID NOs: 81 and 83-88; and/or

   the mutation in LCDR3 is 1 amino acid substitution of N4Q, S5A and Y8V in the amino acid sequence set forth in SEQ ID NO: 112, or 2 or 1 amino acid substitution of Q1H, N4Y, N5I/K and L8Y in the amino acid sequence set forth in SEQ ID NO: 115, or 1 amino acid substitution of N4Q and S5A in the amino acid sequence set forth in SEQ ID NO: 116; the amino acid sequence is preferably set forth in any one of SEQ ID NOs: 113, 117-119 and 122-125; and/or
   the mutation in HCDR1 is 2 or 1 amino acid substitution of F2G and D6R/N/S/Y/T in the amino acid sequence set forth in SEQ ID NO: 7; the amino acid sequence is preferably set forth in any one of SEQ ID NOs: 8-13; and/or
   the mutation in HCDR2 is 2 or 1 amino acid substitution of Y2F, D3N/E, G4E/A, S5N/T/R and N6E/D/K in the amino acid sequence set forth in SEQ ID NO: 36; the amino acid sequence is preferably set forth in any one of SEQ ID NOs: 27-33, 35 and 37-40.

3. The anti-B7-H7 antibody or the antigen-binding fragment thereof of claim 1 or 2, wherein

   the LCDR1 comprises an amino acid sequence set forth in SEQ ID NO: 87, the LCDR2 comprises an amino acid sequence set forth in SEQ ID NO: 97, and the LCDR3 comprises an amino acid sequence set forth in SEQ ID NO: 120; or
   the LCDR1 comprises an amino acid sequence set forth in SEQ ID NO: 82, the LCDR2 comprises an amino acid sequence set forth in SEQ ID NO: 94, and the LCDR3 comprises an amino acid sequence set forth in SEQ ID NO: 112; or
   the LCDR1 comprises an amino acid sequence set forth in SEQ ID NO: 82, the LCDR2 comprises an amino acid sequence set forth in SEQ ID NO: 94, and the LCDR3 comprises an amino acid sequence set forth in SEQ ID NO: 122; or
   the LCDR1 comprises an amino acid sequence set forth in SEQ ID NO: 82, the LCDR2 comprises an amino acid sequence set forth in SEQ ID NO: 94, and the LCDR3 comprises an amino acid sequence set forth in SEQ ID NO: 123; or
   the LCDR1 comprises an amino acid sequence set forth in SEQ ID NO: 85, the LCDR2 comprises an amino acid sequence set forth in SEQ ID NO: 97, and the LCDR3 comprises an amino acid sequence set forth in SEQ ID NO: 116; or
   the LCDR1 comprises an amino acid sequence set forth in SEQ ID NO: 85, the LCDR2 comprises an amino acid sequence set forth in SEQ ID NO: 97, and the LCDR3 comprises an amino acid sequence set forth in SEQ ID NO:

124; or

the LCDR1 comprises an amino acid sequence set forth in SEQ ID NO: 85, the LCDR2 comprises an amino acid sequence set forth in SEQ ID NO: 97, and the LCDR3 comprises an amino acid sequence set forth in SEQ ID NO: 125; or

the LCDR1 comprises an amino acid sequence set forth in SEQ ID NO: 81, the LCDR2 comprises an amino acid sequence set forth in SEQ ID NO: 94, and the LCDR3 comprises an amino acid sequence set forth in SEQ ID NO: 111; or

the LCDR1 comprises an amino acid sequence set forth in SEQ ID NO: 82, the LCDR2 comprises an amino acid sequence set forth in SEQ ID NO: 94, and the LCDR3 comprises an amino acid sequence set forth in SEQ ID NO: 111; or

the LCDR1 comprises an amino acid sequence set forth in SEQ ID NO: 82, the LCDR2 comprises an amino acid sequence set forth in SEQ ID NO: 94, and the LCDR3 comprises an amino acid sequence set forth in SEQ ID NO: 117; or

the LCDR1 comprises an amino acid sequence set forth in SEQ ID NO: 82, the LCDR2 comprises an amino acid sequence set forth in SEQ ID NO: 96, and the LCDR3 comprises an amino acid sequence set forth in SEQ ID NO: 114; or

the LCDR1 comprises an amino acid sequence set forth in SEQ ID NO: 83, the LCDR2 comprises an amino acid sequence set forth in SEQ ID NO: 95, and the LCDR3 comprises an amino acid sequence set forth in SEQ ID NO: 113; or

the LCDR1 comprises an amino acid sequence set forth in SEQ ID NO: 83, the LCDR2 comprises an amino acid sequence set forth in SEQ ID NO: 98, and the LCDR3 comprises an amino acid sequence set forth in SEQ ID NO: 113; or

the LCDR1 comprises an amino acid sequence set forth in SEQ ID NO: 83, the LCDR2 comprises an amino acid sequence set forth in SEQ ID NO: 99, and the LCDR3 comprises an amino acid sequence set forth in SEQ ID NO: 119; or

the LCDR1 comprises an amino acid sequence set forth in SEQ ID NO: 84, the LCDR2 comprises an amino acid sequence set forth in SEQ ID NO: 95, and the LCDR3 comprises an amino acid sequence set forth in SEQ ID NO: 115; or

the LCDR1 comprises an amino acid sequence set forth in SEQ ID NO: 86, the LCDR2 comprises an amino acid sequence set forth in SEQ ID NO: 95, and the LCDR3 comprises an amino acid sequence set forth in SEQ ID NO: 118; or

the LCDR1 comprises an amino acid sequence set forth in SEQ ID NO: 88, the LCDR2 comprises an amino acid sequence set forth in SEQ ID NO: 100, and the LCDR3 comprises an amino acid sequence set forth in SEQ ID NO: 121;

and/or

the HCDR1 comprises an amino acid sequence set forth in SEQ ID NO: 12, the HCDR2 comprises an amino acid sequence set forth in SEQ ID NO: 34, and the HCDR3 comprises an amino acid sequence set forth in SEQ ID NO: 66; or

the HCDR1 comprises an amino acid sequence set forth in SEQ ID NO: 7, the HCDR2 comprises an amino acid sequence set forth in SEQ ID NO: 28, and the HCDR3 comprises an amino acid sequence set forth in SEQ ID NO: 58; or

the HCDR1 comprises an amino acid sequence set forth in SEQ ID NO: 7, the HCDR2 comprises an amino acid sequence set forth in SEQ ID NO: 37, and the HCDR3 comprises an amino acid sequence set forth in SEQ ID NO: 58; or

the HCDR1 comprises an amino acid sequence set forth in SEQ ID NO: 7, the HCDR2 comprises an amino acid sequence set forth in SEQ ID NO: 38, and the HCDR3 comprises an amino acid sequence set forth in SEQ ID NO: 58; or

the HCDR1 comprises an amino acid sequence set forth in SEQ ID NO: 9, the HCDR2 comprises an amino acid sequence set forth in SEQ ID NO: 27, and the HCDR3 comprises an amino acid sequence set forth in SEQ ID NO: 63; or

the HCDR1 comprises an amino acid sequence set forth in SEQ ID NO: 9, the HCDR2 comprises an amino acid sequence set forth in SEQ ID NO: 39, and the HCDR3 comprises an amino acid sequence set forth in SEQ ID NO: 63; or

the HCDR1 comprises an amino acid sequence set forth in SEQ ID NO: 9, the HCDR2 comprises an amino acid sequence set forth in SEQ ID NO: 40, and the HCDR3 comprises an amino acid sequence set forth in SEQ ID NO: 63; or

the HCDR1 comprises an amino acid sequence set forth in SEQ ID NO: 7, the HCDR2 comprises an amino acid sequence set forth in SEQ ID NO: 27, and the HCDR3 comprises an amino acid sequence set forth in SEQ ID NO:

57; or

the HCDR1 comprises an amino acid sequence set forth in SEQ ID NO: 7, the HCDR2 comprises an amino acid sequence set forth in SEQ ID NO: 28, and the HCDR3 comprises an amino acid sequence set forth in SEQ ID NO: 59; or

the HCDR1 comprises an amino acid sequence set forth in SEQ ID NO: 7, the HCDR2 comprises an amino acid sequence set forth in SEQ ID NO: 36, and the HCDR3 comprises an amino acid sequence set forth in SEQ ID NO: 68; or

the HCDR1 comprises an amino acid sequence set forth in SEQ ID NO: 8, the HCDR2 comprises an amino acid sequence set forth in SEQ ID NO: 29, and the HCDR3 comprises an amino acid sequence set forth in SEQ ID NO: 60; or

the HCDR1 comprises an amino acid sequence set forth in SEQ ID NO: 8, the HCDR2 comprises an amino acid sequence set forth in SEQ ID NO: 30, and the HCDR3 comprises an amino acid sequence set forth in SEQ ID NO: 62; or

the HCDR1 comprises an amino acid sequence set forth in SEQ ID NO: 8, the HCDR2 comprises an amino acid sequence set forth in SEQ ID NO: 32, and the HCDR3 comprises an amino acid sequence set forth in SEQ ID NO: 62; or

the HCDR1 comprises an amino acid sequence set forth in SEQ ID NO: 9, the HCDR2 comprises an amino acid sequence set forth in SEQ ID NO: 27, and the HCDR3 comprises an amino acid sequence set forth in SEQ ID NO: 61; or

the HCDR1 comprises an amino acid sequence set forth in SEQ ID NO: 9, the HCDR2 comprises an amino acid sequence set forth in SEQ ID NO: 31, and the HCDR3 comprises an amino acid sequence set forth in SEQ ID NO: 64; or

the HCDR1 comprises an amino acid sequence set forth in SEQ ID NO: 10, the HCDR2 comprises an amino acid sequence set forth in SEQ ID NO: 29, and the HCDR3 comprises an amino acid sequence set forth in SEQ ID NO: 62; or

the HCDR1 comprises an amino acid sequence set forth in SEQ ID NO: 11, the HCDR2 comprises an amino acid sequence set forth in SEQ ID NO: 33, and the HCDR3 comprises an amino acid sequence set forth in SEQ ID NO: 65; or

the HCDR1 comprises an amino acid sequence set forth in SEQ ID NO: 13, the HCDR2 comprises an amino acid sequence set forth in SEQ ID NO: 35, and the HCDR3 comprises an amino acid sequence set forth in SEQ ID NO: 67;

preferably, in the anti-B7-H7 antibody or the antigen-binding fragment thereof,

the VL comprises LCDR1, LCDR2 and LCDR3 having amino acid sequences set forth in SEQ ID NOs: 87, 97 and 120, respectively, and the VH comprises HCDR1, HCDR2 and HCDR3 having amino acid sequences set forth in SEQ ID NOs: 12, 34 and 66, respectively; or

the VL comprises LCDR1, LCDR2 and LCDR3 having amino acid sequences set forth in SEQ ID NOs: 82, 94 and 112, respectively, and the VH comprises HCDR1, HCDR2 and HCDR3 having amino acid sequences set forth in SEQ ID NOs: 7, 28 and 58, respectively; or

the VL comprises LCDR1, LCDR2 and LCDR3 having amino acid sequences set forth in SEQ ID NOs: 82, 94 and 112, respectively, and the VH comprises HCDR1, HCDR2 and HCDR3 having amino acid sequences set forth in SEQ ID NOs: 7, 37 and 58, respectively; or

the VL comprises LCDR1, LCDR2 and LCDR3 having amino acid sequences set forth in SEQ ID NOs: 82, 94 and 122, respectively, and the VH comprises HCDR1, HCDR2 and HCDR3 having amino acid sequences set forth in SEQ ID NOs: 7, 37 and 58, respectively; or

the VL comprises LCDR1, LCDR2 and LCDR3 having amino acid sequences set forth in SEQ ID NOs: 82, 94 and 123, respectively, and the VH comprises HCDR1, HCDR2 and HCDR3 having amino acid sequences set forth in SEQ ID NOs: 7, 37 and 58, respectively; or

the VL comprises LCDR1, LCDR2 and LCDR3 having amino acid sequences set forth in SEQ ID NOs: 82, 94 and 112, respectively, and the VH comprises HCDR1, HCDR2 and HCDR3 having amino acid sequences set forth in SEQ ID NOs: 7, 38 and 58, respectively; or

the VL comprises LCDR1, LCDR2 and LCDR3 having amino acid sequences set forth in SEQ ID NOs: 82, 94 and 122, respectively, and the VH comprises HCDR1, HCDR2 and HCDR3 having amino acid sequences set forth in SEQ ID NOs: 7, 38 and 58, respectively; or

the VL comprises LCDR1, LCDR2 and LCDR3 having amino acid sequences set forth in SEQ ID NOs: 82, 94 and 123, respectively, and the VH comprises HCDR1, HCDR2 and HCDR3 having amino acid sequences set forth in SEQ ID NOs: 7, 38 and 58, respectively; or

the VL comprises LCDR1, LCDR2 and LCDR3 having amino acid sequences set forth in SEQ ID NOs: 82, 94 and 122, respectively, and the VH comprises HCDR1, HCDR2 and HCDR3 having amino acid sequences set forth in SEQ ID NOs: 7, 28 and 58, respectively; or

the VL comprises LCDR1, LCDR2 and LCDR3 having amino acid sequences set forth in SEQ ID NOs: 82, 94 and 123, respectively, and the VH comprises HCDR1, HCDR2 and HCDR3 having amino acid sequences set forth in SEQ ID NOs: 7, 28 and 58, respectively; or

the VL comprises LCDR1, LCDR2 and LCDR3 having amino acid sequences set forth in SEQ ID NOs: 85, 97 and 116, respectively, and the VH comprises HCDR1, HCDR2 and HCDR3 having amino acid sequences set forth in SEQ ID NOs: 9, 27 and 63, respectively; or

the VL comprises LCDR1, LCDR2 and LCDR3 having amino acid sequences set forth in SEQ ID NOs: 85, 97 and 116, respectively, and the VH comprises HCDR1, HCDR2 and HCDR3 having amino acid sequences set forth in SEQ ID NOs: 9, 39 and 63, respectively; or

the VL comprises LCDR1, LCDR2 and LCDR3 having amino acid sequences set forth in SEQ ID NOs: 85, 97 and 124, respectively, and the VH comprises HCDR1, HCDR2 and HCDR3 having amino acid sequences set forth in SEQ ID NOs: 9, 39 and 63, respectively; or

the VL comprises LCDR1, LCDR2 and LCDR3 having amino acid sequences set forth in SEQ ID NOs: 85, 97 and 125, respectively, and the VH comprises HCDR1, HCDR2 and HCDR3 having amino acid sequences set forth in SEQ ID NOs: 9, 39 and 63, respectively; or

the VL comprises LCDR1, LCDR2 and LCDR3 having amino acid sequences set forth in SEQ ID NOs: 85, 97 and 116, respectively, and the VH comprises HCDR1, HCDR2 and HCDR3 having amino acid sequences set forth in SEQ ID NOs: 9, 40 and 63, respectively; or

the VL comprises LCDR1, LCDR2 and LCDR3 having amino acid sequences set forth in SEQ ID NOs: 85, 97 and 124, respectively, and the VH comprises HCDR1, HCDR2 and HCDR3 having amino acid sequences set forth in SEQ ID NOs: 9, 40 and 63, respectively; or

the VL comprises LCDR1, LCDR2 and LCDR3 having amino acid sequences set forth in SEQ ID NOs: 85, 97 and 125, respectively, and the VH comprises HCDR1, HCDR2 and HCDR3 having amino acid sequences set forth in SEQ ID NOs: 9, 40 and 63, respectively; or

the VL comprises LCDR1, LCDR2 and LCDR3 having amino acid sequences set forth in SEQ ID NOs: 85, 97 and 124, respectively, and the VH comprises HCDR1, HCDR2 and HCDR3 having amino acid sequences set forth in SEQ ID NOs: 9, 27 and 63, respectively; or

the VL comprises LCDR1, LCDR2 and LCDR3 having amino acid sequences set forth in SEQ ID NOs: 85, 97 and 125, respectively, and the VH comprises HCDR1, HCDR2 and HCDR3 having amino acid sequences set forth in SEQ ID NOs: 9, 27 and 63, respectively; or

the VL comprises LCDR1, LCDR2 and LCDR3 having amino acid sequences set forth in SEQ ID NOs: 81, 94 and 111, respectively, and the VH comprises HCDR1, HCDR2 and HCDR3 having amino acid sequences set forth in SEQ ID NOs: 7, 27 and 57, respectively; or

the VL comprises LCDR1, LCDR2 and LCDR3 having amino acid sequences set forth in SEQ ID NOs: 82, 94 and 112, respectively, and the VH comprises HCDR1, HCDR2 and HCDR3 having amino acid sequences set forth in SEQ ID NOs: 7, 28 and 59, respectively; or

the VL comprises LCDR1, LCDR2 and LCDR3 having amino acid sequences set forth in SEQ ID NOs: 83, 95 and 113, respectively, and the VH comprises HCDR1, HCDR2 and HCDR3 having amino acid sequences set forth in SEQ ID NOs: 8, 29 and 60, respectively; or

the VL comprises LCDR1, LCDR2 and LCDR3 having amino acid sequences set forth in SEQ ID NOs: 82, 96 and 114, respectively, and the VH comprises HCDR1, HCDR2 and HCDR3 having amino acid sequences set forth in SEQ ID NOs: 9, 27 and 61, respectively; or

the VL comprises LCDR1, LCDR2 and LCDR3 having amino acid sequences set forth in SEQ ID NOs: 84, 95 and 115, respectively, and the VH comprises HCDR1, HCDR2 and HCDR3 having amino acid sequences set forth in SEQ ID NOs: 10, 29 and 62, respectively; or

the VL comprises LCDR1, LCDR2 and LCDR3 having amino acid sequences set forth in SEQ ID NOs: 83, 98 and 113, respectively, and the VH comprises HCDR1, HCDR2 and HCDR3 having amino acid sequences set forth in SEQ ID NOs: 8, 30 and 62, respectively; or

the VL comprises LCDR1, LCDR2 and LCDR3 having amino acid sequences set forth in SEQ ID NOs: 82, 94 and 117, respectively, and the VH comprises HCDR1, HCDR2 and HCDR3 having amino acid sequences set forth in SEQ ID NOs: 9, 31 and 64, respectively; or

the VL comprises LCDR1, LCDR2 and LCDR3 having amino acid sequences set forth in SEQ ID NOs: 86, 95 and 118, respectively, and the VH comprises HCDR1, HCDR2 and HCDR3 having amino acid sequences set forth in SEQ ID NOs: 8, 32 and 62, respectively; or

the VL comprises LCDR1, LCDR2 and LCDR3 having amino acid sequences set forth in SEQ ID NOs: 83, 99 and 119, respectively, and the VH comprises HCDR1, HCDR2 and HCDR3 having amino acid sequences set forth in SEQ ID NOs: 11, 33 and 65, respectively; or

the VL comprises LCDR1, LCDR2 and LCDR3 having amino acid sequences set forth in SEQ ID NOs: 88, 100 and 121, respectively, and the VH comprises HCDR1, HCDR2 and HCDR3 having amino acid sequences set forth in SEQ ID NOs: 13, 35 and 67, respectively; or

the VL comprises LCDR1, LCDR2 and LCDR3 having amino acid sequences set forth in SEQ ID NOs: 82, 94 and 111, respectively, and the VH comprises HCDR1, HCDR2 and HCDR3 having amino acid sequences set forth in SEQ ID NOs: 7, 36 and 68, respectively.

4. The anti-B7-H7 antibody or the antigen-binding fragment thereof of any one of claims 1-3, wherein the light chain variable region (VL) further comprises a light chain variable region framework region (VL FWR), and/or the heavy chain variable region (VH) further comprises a heavy chain variable region framework region (VH FWR); wherein

the VL FWR is a light chain variable region framework region of a human antibody; the VH FWR is a heavy chain variable region framework region of a human antibody;
preferably,
the VL FWR comprises VL FWR1 having an amino acid sequence set forth in any one of SEQ ID NOs: 74-79, VL FWR2 having an amino acid sequence set forth in any one of SEQ ID NOs: 90-92, VL FWR3 having an amino acid sequence set forth in any one of SEQ ID NOs: 102-109 and VL FWR4 having an amino acid sequence set forth in any one of SEQ ID NOs: 127-130, and/or the VH FWR comprises VH FWR1 having an amino acid sequence set forth in any one of SEQ ID NOs: 2-5, VH FWR2 having an amino acid sequence set forth in any one of SEQ ID NOs: 15-25, VH FWR3 having an amino acid sequence set forth in any one of SEQ ID NOs: 42-55 and VH FWR4 having an amino acid sequence set forth in any one of SEQ ID NOs: 70-72;
more preferably,
the VL comprises an amino acid sequence set forth in any one of SEQ ID NOs: 151-167; and/or the VH comprises an amino acid sequence set forth in any one of SEQ ID NOs: 132-149; further more preferably,
the VL comprises an amino acid sequence set forth in SEQ ID NO: 161, and the VH comprises an amino acid sequence set forth in SEQ ID NO: 143; or
the VL comprises an amino acid sequence set forth in SEQ ID NO: 152, and the VH comprises an amino acid sequence set forth in SEQ ID NO: 133; or
the VL comprises an amino acid sequence set forth in SEQ ID NO: 152, and the VH comprises an amino acid sequence set forth in SEQ ID NO: 146; or
the VL comprises an amino acid sequence set forth in SEQ ID NO: 164, and the VH comprises an amino acid sequence set forth in SEQ ID NO: 146; or
the VL comprises an amino acid sequence set forth in SEQ ID NO: 165, and the VH comprises an amino acid sequence set forth in SEQ ID NO: 146; or
the VL comprises an amino acid sequence set forth in SEQ ID NO: 152, and the VH comprises an amino acid sequence set forth in SEQ ID NO: 147; or
the VL comprises an amino acid sequence set forth in SEQ ID NO: 164, and the VH comprises an amino acid sequence set forth in SEQ ID NO: 147; or
the VL comprises an amino acid sequence set forth in SEQ ID NO: 165, and the VH comprises an amino acid sequence set forth in SEQ ID NO: 147; or
the VL comprises an amino acid sequence set forth in SEQ ID NO: 164, and the VH comprises an amino acid sequence set forth in SEQ ID NO: 133; or
the VL comprises an amino acid sequence set forth in SEQ ID NO: 165, and the VH comprises an amino acid sequence set forth in SEQ ID NO: 133; or
the VL comprises an amino acid sequence set forth in SEQ ID NO: 156, and the VH comprises an amino acid sequence set forth in SEQ ID NO: 138; or
the VL comprises an amino acid sequence set forth in SEQ ID NO: 156, and the VH comprises an amino acid sequence set forth in SEQ ID NO: 148; or
the VL comprises an amino acid sequence set forth in SEQ ID NO: 166, and the VH comprises an amino acid sequence set forth in SEQ ID NO: 148; or
the VL comprises an amino acid sequence set forth in SEQ ID NO: 167, and the VH comprises an amino acid sequence set forth in SEQ ID NO: 148; or
the VL comprises an amino acid sequence set forth in SEQ ID NO: 156, and the VH comprises an amino acid sequence set forth in SEQ ID NO: 149; or
the VL comprises an amino acid sequence set forth in SEQ ID NO: 166, and the VH comprises an amino acid sequence set forth in SEQ ID NO: 149; or
the VL comprises an amino acid sequence set forth in SEQ ID NO: 167, and the VH comprises an amino acid sequence set forth in SEQ ID NO: 149; or

the VL comprises an amino acid sequence set forth in SEQ ID NO: 166, and the VH comprises an amino acid sequence set forth in SEQ ID NO: 138; or

the VL comprises an amino acid sequence set forth in SEQ ID NO: 167, and the VH comprises an amino acid sequence set forth in SEQ ID NO: 138; or

the VL comprises an amino acid sequence set forth in SEQ ID NO: 151, and the VH comprises an amino acid sequence set forth in SEQ ID NO: 132; or

the VL comprises an amino acid sequence set forth in SEQ ID NO: 152, and the VH comprises an amino acid sequence set forth in SEQ ID NO: 134; or

the VL comprises an amino acid sequence set forth in SEQ ID NO: 153, and the VH comprises an amino acid sequence set forth in SEQ ID NO: 135; or

the VL comprises an amino acid sequence set forth in SEQ ID NO: 154, and the VH comprises an amino acid sequence set forth in SEQ ID NO: 136; or

the VL comprises an amino acid sequence set forth in SEQ ID NO: 155, and the VH comprises an amino acid sequence set forth in SEQ ID NO: 137; or

the VL comprises an amino acid sequence set forth in SEQ ID NO: 157, and the VH comprises an amino acid sequence set forth in SEQ ID NO: 139; or

the VL comprises an amino acid sequence set forth in SEQ ID NO: 158, and the VH comprises an amino acid sequence set forth in SEQ ID NO: 140; or

the VL comprises an amino acid sequence set forth in SEQ ID NO: 159, and the VH comprises an amino acid sequence set forth in SEQ ID NO: 141; or

the VL comprises an amino acid sequence set forth in SEQ ID NO: 160, and the VH comprises an amino acid sequence set forth in SEQ ID NO: 142; or

the VL comprises an amino acid sequence set forth in SEQ ID NO: 162, and the VH comprises an amino acid sequence set forth in SEQ ID NO: 144; or

the VL comprises an amino acid sequence set forth in SEQ ID NO: 163, and the VH comprises an amino acid sequence set forth in SEQ ID NO: 145.

5. The anti-B7-H7 antibody or the antigen-binding fragment thereof of any one of claims 1-4, wherein the anti-B7-H7 antibody or the antigen-binding fragment thereof is a full-length antibody, Fab, Fab', F(ab')$_2$, Fv, preferably scFv, a bispecific antibody, a multispecific antibody, a heavy chain antibody or a single domain antibody, or a monoclonal antibody or a polyclonal antibody prepared from the above-mentioned antibodies.

6. The anti-B7-H7 antibody or the antigen-binding fragment thereof of claim 5, wherein the anti-B7-H7 antibody or the antigen-binding fragment thereof is a full-length antibody comprising an antibody heavy chain constant region and an antibody light chain constant region;

preferably, the heavy chain constant region is selected from hIgG1, hIgG2, hIgG3 or hIgG4, and the light chain constant region is selected from a kappa chain or a lamda chain of a human antibody;
more preferably, the heavy chain constant region is hIgG1, and the light chain constant region is a kappa chain of a human antibody.

7. The anti-B7-H7 antibody or the antigen-binding fragment thereof of claim 6, wherein the full-length antibody comprises a heavy chain and a light chain; wherein the heavy chain comprises an amino acid sequence set forth in any one of SEQ ID NOs: 169-190; and/or the light chain comprises an amino acid sequence set forth in any one of SEQ ID NOs: 192-208;

preferably,
the heavy chain comprises an amino acid sequence set forth in SEQ ID NO: 186, and the light chain comprises an amino acid sequence set forth in SEQ ID NO: 202; or
the heavy chain comprises an amino acid sequence set forth in SEQ ID NO: 180, and the light chain comprises an amino acid sequence set forth in SEQ ID NO: 202; or
the heavy chain comprises an amino acid sequence set forth in SEQ ID NO: 184, and the light chain comprises an amino acid sequence set forth in SEQ ID NO: 193; or
the heavy chain comprises an amino acid sequence set forth in SEQ ID NO: 170, and the light chain comprises an amino acid sequence set forth in SEQ ID NO: 193; or
the heavy chain comprises an amino acid sequence set forth in SEQ ID NO: 187, and the light chain comprises an amino acid sequence set forth in SEQ ID NO: 193; or
the heavy chain comprises an amino acid sequence set forth in SEQ ID NO: 187, and the light chain comprises an amino acid sequence set forth in SEQ ID NO: 205; or

the heavy chain comprises an amino acid sequence set forth in SEQ ID NO: 187, and the light chain comprises an amino acid sequence set forth in SEQ ID NO: 206; or

the heavy chain comprises an amino acid sequence set forth in SEQ ID NO: 188, and the light chain comprises an amino acid sequence set forth in SEQ ID NO: 193; or

the heavy chain comprises an amino acid sequence set forth in SEQ ID NO: 188, and the light chain comprises an amino acid sequence set forth in SEQ ID NO: 205; or

the heavy chain comprises an amino acid sequence set forth in SEQ ID NO: 188, and the light chain comprises an amino acid sequence set forth in SEQ ID NO: 206; or

the heavy chain comprises an amino acid sequence set forth in SEQ ID NO: 184, and the light chain comprises an amino acid sequence set forth in SEQ ID NO: 205; or

the heavy chain comprises an amino acid sequence set forth in SEQ ID NO: 184, and the light chain comprises an amino acid sequence set forth in SEQ ID NO: 206; or

the heavy chain comprises an amino acid sequence set forth in SEQ ID NO: 185, and the light chain comprises an amino acid sequence set forth in SEQ ID NO: 197; or

the heavy chain comprises an amino acid sequence set forth in SEQ ID NO: 175, and the light chain comprises an amino acid sequence set forth in SEQ ID NO: 197; or

the heavy chain comprises an amino acid sequence set forth in SEQ ID NO: 189, and the light chain comprises an amino acid sequence set forth in SEQ ID NO: 197; or

the heavy chain comprises an amino acid sequence set forth in SEQ ID NO: 189, and the light chain comprises an amino acid sequence set forth in SEQ ID NO: 207; or

the heavy chain comprises an amino acid sequence set forth in SEQ ID NO: 189, and the light chain comprises an amino acid sequence set forth in SEQ ID NO: 208; or

the heavy chain comprises an amino acid sequence set forth in SEQ ID NO: 190, and the light chain comprises an amino acid sequence set forth in SEQ ID NO: 197; or

the heavy chain comprises an amino acid sequence set forth in SEQ ID NO: 190, and the light chain comprises an amino acid sequence set forth in SEQ ID NO: 207; or

the heavy chain comprises an amino acid sequence set forth in SEQ ID NO: 190, and the light chain comprises an amino acid sequence set forth in SEQ ID NO: 208; or

the heavy chain comprises an amino acid sequence set forth in SEQ ID NO: 185, and the light chain comprises an amino acid sequence set forth in SEQ ID NO: 207; or

the heavy chain comprises an amino acid sequence set forth in SEQ ID NO: 185, and the light chain comprises an amino acid sequence set forth in SEQ ID NO: 208; or

the heavy chain comprises an amino acid sequence set forth in SEQ ID NO: 169, and the light chain comprises an amino acid sequence set forth in SEQ ID NO: 192; or

the heavy chain comprises an amino acid sequence set forth in SEQ ID NO: 171, and the light chain comprises an amino acid sequence set forth in SEQ ID NO: 193; or

the heavy chain comprises an amino acid sequence set forth in SEQ ID NO: 172, and the light chain comprises an amino acid sequence set forth in SEQ ID NO: 194; or

the heavy chain comprises an amino acid sequence set forth in SEQ ID NO: 173, and the light chain comprises an amino acid sequence set forth in SEQ ID NO: 195; or

the heavy chain comprises an amino acid sequence set forth in SEQ ID NO: 174, and the light chain comprises an amino acid sequence set forth in SEQ ID NO: 196; or

the heavy chain comprises an amino acid sequence set forth in SEQ ID NO: 176, and the light chain comprises an amino acid sequence set forth in SEQ ID NO: 198; or

the heavy chain comprises an amino acid sequence set forth in SEQ ID NO: 177, and the light chain comprises an amino acid sequence set forth in SEQ ID NO: 199; or

the heavy chain comprises an amino acid sequence set forth in SEQ ID NO: 178, and the light chain comprises an amino acid sequence set forth in SEQ ID NO: 200; or

the heavy chain comprises an amino acid sequence set forth in SEQ ID NO: 179, and the light chain comprises an amino acid sequence set forth in SEQ ID NO: 201; or

the heavy chain comprises an amino acid sequence set forth in SEQ ID NO: 181, and the light chain comprises an amino acid sequence set forth in SEQ ID NO: 203; or

the heavy chain comprises an amino acid sequence set forth in SEQ ID NO: 182, and the light chain comprises an amino acid sequence set forth in SEQ ID NO: 204.

8. An isolated nucleic acid encoding the anti-B7-H7 antibody or the antigen-binding fragment thereof of any one of claims 1-7.

9. A recombinant expression vector, comprising the isolated nucleic acid of claim 8;
wherein preferably, the expression vector comprises a eukaryotic cell expression vector and/or a prokaryotic cell expression vector.

10. A transformant, comprising the recombinant expression vector of claim 9;
wherein preferably, the host cell of the transformant is a prokaryotic cell and/or a eukaryotic cell, the prokaryotic cell is preferably an *E. coli* cell such as a TG1 cell or a BL21 cell, and the eukaryotic cell is preferably a HEK293 cell or a CHO cell.

11. A chimeric antigen receptor, comprising the anti-B7-H7 antibody or the antigen-binding fragment thereof of any one of claims 1-7.

12. A genetically modified cell, wherein the genetically modified cell comprises the chimeric antigen receptor of claim 11; preferably, the genetically modified cell is a eukaryotic cell, preferably an isolated human cell; more preferably, the genetically modified cell is an immune cell such as a T cell, or an NK cell.

13. A method for preparing an anti-B7-H7 antibody or an antigen-binding fragment thereof, wherein the method comprises the following steps: culturing the transformant of claim 10, and obtaining the anti-B7-H7 antibody or the antigen-binding fragment thereof from the culture.

14. An antibody-drug conjugate, comprising an antibody moiety and a conjugated moiety, wherein the antibody moiety comprises the anti-B7-H7 antibody or the antigen-binding fragment thereof of any one of claims 1-7, the conjugated moiety comprises a detectable marker, a drug, a toxin, a cytokine, a radionuclide, an enzyme, or a combination thereof, and the antibody moiety is conjugated to the conjugated moiety via a chemical bond or a linker.

15. A pharmaceutical composition, comprising the anti-B7-H7 antibody or the antigen-binding fragment thereof of any one of claims 1-7, the chimeric antigen receptor of claim 11, the genetically modified cell of claim 12, and/or the antibody-drug conjugate of claim 14;

wherein preferably,
the pharmaceutical composition is in a liquid dosage form, a gaseous dosage form, a solid dosage form and a semi-solid dosage form, and/or the pharmaceutical composition can be administered via oral administration, injection administration, nasal administration, transdermal administration or mucosal administration;
further preferably,
the pharmaceutical composition further comprises a combined therapeutic agent, and the combined therapeutic agent comprises a chemotherapeutic agent, a radiotherapeutic agent, an immunosuppressant, and/or a cytotoxic drug.

16. A kit, comprising the antibody or the antigen-binding fragment thereof of any one of claims 1-7, the chimeric antigen receptor of claim 11, the genetically modified cell of claim 12, the antibody-drug conjugate of claim 14, and/or the pharmaceutical composition of claim 15; wherein preferably, the kit further comprises (i) a device for administering the antibody or the antigen-binding fragment thereof, or the chimeric antigen receptor, or the genetically modified cell, or the antibody-drug conjugate, or the pharmaceutical composition; and/or (ii) instructions for use.

17. Use of the anti-B7-H7 antibody or the antigen-binding fragment thereof of any one of claims 1-7, the chimeric antigen receptor of claim 11, the genetically modified cell of claim 12, the antibody-drug conjugate of claim 14, the pharmaceutical composition of claim 15, and/or the kit of claim 16 in the preparation of a drug for the treatment and/or prevention of a cancer; wherein preferably, the cancer is selected from the group consisting of lung cancer, breast cancer, gastric cancer, small intestine cancer, colon cancer, rectal cancer, pancreatic cancer, kidney cancer, bladder cancer and osteosarcoma.

18. An administration device, wherein the administration device comprises the anti-B7-H7 antibody or the antigen-binding fragment thereof of any one of claims 1-7, the chimeric antigen receptor of claim 11, the genetically modified cell of claim 12, the antibody-drug conjugate of claim 14, the pharmaceutical composition of claim 15, and/or the kit of claim 16;
preferably, the administration device further comprises a component, such as a syringe, an infusion device or an implantable administration device, that accommodates the anti-B7-H7 antibody or the antigen-binding fragment thereof, the chimeric antigen receptor, the genetically modified cell, the antibody-drug conjugate, the pharmaceutical composition, and/or the kit, or that administers the anti-B7-H7 antibody or the antigen-binding fragment thereof, the chimeric antigen receptor, the genetically modified cell, the antibody-drug conjugate, the pharmaceutical composition, and/or the kit to a subject.

19. A method for detecting B7-H7, wherein the method comprises the step of performing detection using the anti-B7-H7 antibody or the antigen-binding fragment thereof of any one of claims 1-7, the chimeric antigen receptor of claim 11, the genetically modified cell of claim 12, the antibody-drug conjugate of claim 14, the pharmaceutical composition of claim 15, and/or the kit of claim 16; preferably, the detection is for non-diagnostic and/or therapeutic purposes.

20. A method for preventing and/or treating a B7-H7-related disease or disorder in a subject in need thereof, wherein the method comprises the step of performing detection by administering to the subject the anti-B7-H7 antibody or the antigen-binding fragment thereof of any one of claims 1-7, the chimeric antigen receptor of claim 11, the genetically modified cell of claim 12, the antibody-drug conjugate of claim 14, the pharmaceutical composition of claim 15, and/or the kit of claim 16.

21. The method of claim 20, wherein the disease or disorder is a cancer; preferably, the cancer is selected from one or more of the group consisting of lung cancer, breast cancer, gastric cancer, small intestine cancer, colon cancer, rectal cancer, pancreatic cancer, kidney cancer, bladder cancer, osteosarcoma, etc.

A

B

Binding to HEK-293/hu B7-H7

Binding to HEK-293/hu B7-H7

C

Binding to HEK-293/cyno B7-H7

D

Binding to HEK-293/cyno B7-H7

E

Binding to HCC827

F

Binding to HCC827

FIG. 1

## Binding to LS-180

| Antibody | LS180 | |
| --- | --- | --- |
| | Maximum MFI | EC50 (nM) |
| PR003181 | ~ | ~ |
| PR003249 | 21042 | 0.035 |
| PR003259 | 20317 | 0.066 |
| PR003264 | 21767 | 2.198 |
| PR004260 | ~ | ~ |
| PR004261 | 19723 | 0.036 |
| PR004262 | 19225 | 0.048 |
| PR004263 | 23985 | 3.075 |

FIG. 2

### A  Binding to LS-180

### B  Binding to LS-180

| Antibody | LS180 | |
| --- | --- | --- |
| | Maximum MFI | EC50 (nM) |
| PR004261 | 26913 | 0.034 |
| PR004262 | 25881 | 0.033 |
| PR004483 | 25060 | 0.044 |
| PR004484 | 24634 | 0.04 |
| PR004491 | 22835 | 0.066 |
| PR004492 | 22862 | 0.052 |

FIG. 3

44

A  Blocking the binding of
   HEK-293/CD28H to B7-H7

B  Blocking the binding of
   HEK-293/CD28H to B7-H7

FIG. 4

A  Blocking the binding of
   CHO-K1/KIR3DL3 to B7-H7

B  Blocking the binding of
   CHO-K1/KIR3DL3 to B7-H7

C  Blocking the binding of
   CHO-K1/KIR3DL3 to B7-H7

FIG. 5

Blocking the binding of KIR3DL3 protein to B7-H7 protein

FIG. 6

83

FIG. 7

A

## HEK-293/OS8 control cells (donor 1)

B

## HEK-293/OS8 control cells (donor 2)

FIG. 8

A          Donor 1

B          Donor 2

FIG. 9

FIG. 10

A  Binding of PR004261 to
   HEK293/hu B7-H7 cells

B  Binding of PR004262 to
   HEK293/hu B7-H7 cells

C  Binding of PR004263 to
   HEK293/hu B7-H7 cells

FIG. 11

FIG. 12

## INTERNATIONAL SEARCH REPORT

| | International application No. |
|---|---|
| | **PCT/CN2023/072622** |

**A. CLASSIFICATION OF SUBJECT MATTER**

C07K16/28(2006.01)i;C07K16/46(2006.01)i;C07K19/00(2006.01)i;C12N5/10(2006.01)i;C12N15/13(2006.01)i;C12N15/63(2006.01)i;A61K39/395(2006.01)i;A61P35/00(2006.01)i;G01N33/50(2006.01)i

According to International Patent Classification (IPC) or to both national classification and IPC

**B. FIELDS SEARCHED**

Minimum documentation searched (classification system followed by classification symbols)

IPC: C07K C12N A61K A61P G01N

Documentation searched other than minimum documentation to the extent that such documents are included in the fields searched

Electronic data base consulted during the international search (name of data base and, where practicable, search terms used)

CNABS, CNKI, CNTXT, DWPI, SIPOABS, EPTXT, USTXT, WOTXT, JPTXT, NCBI, 中国专利生物序列检索系统, China Patent Biological Sequence Search System, Genbank, EMBL, STN and Search terms: B7-H7, HHLA2, 抗体, antibody, B7-H?, SEQ ID Nos: 12, 34, 66, 87, 97, 120, 143, 161, 180 and 202

**C. DOCUMENTS CONSIDERED TO BE RELEVANT**

| Category* | Citation of document, with indication, where appropriate, of the relevant passages | Relevant to claim No. |
|---|---|---|
| A | CN 106414489 A (AMPLIMMUNE INC.; UNIV. JOHNS HOPKINS) 15 February 2017 (2017-02-15) claims and embodiments | 1-19 (in part) |
| A | CN 112153977 A (DANA-FARBER CANCER INSTITUTE) 29 December 2020 (2020-12-29) claims and embodiments | 1-19 (in part) |
| A | CN 112930357 A (NGM BIOPHARMACEUTICALS, INC.) 08 June 2021 (2021-06-08) claims, description, paragraphs 252-261, embodiments | 1-19 (in part) |
| A | US 2016002337 A1 (ALBERT EINSTEIN COLLEGE OF MEDICINE OF YESHIVA UNIVERSITY) 07 January 2016 (2016-01-07) claims, description, paragraphs 47-57 | 1-19 (in part) |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **13 April 2023** | **28 April 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

**INTERNATIONAL SEARCH REPORT**

| International application No. |
| --- |
| **PCT/CN2023/072622** |

Box No. I      **Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

     a.    ☑   forming part of the international application as filed.

     b.    ☐   furnished subsequent to the international filing date for the purposes of international search (Rule 13*ter*.1(a)),

            ☐   accompanied by a statement to the effect that the sequence listing does not go beyond the disclosure in the international application as filed.

2.   ☐   With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

| International application No. |
| --- |
| **PCT/CN2023/072622** |

---

**Box No. II    Observations where certain claims were found unsearchable (Continuation of item 2 of first sheet)**

This international search report has not been established in respect of certain claims under Article 17(2)(a) for the following reasons:

1. ☑ Claims Nos.: **20-21**
   because they relate to subject matter not required to be searched by this Authority, namely:

   PCT Rule 39.1(iv) – methods for treatment of the human or animal body by surgery or therapy.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

---

**Box No. III    Observations where unity of invention is lacking (Continuation of item 3 of first sheet)**

This International Searching Authority found multiple inventions in this international application, as follows:

Invention 1: claims 1-19 (in part)

relate to an anti-B7-H7 antibody or an antigen-binding fragment thereof, containing amino acid sequences of LCDRs 1-3 which are respectively as shown in SEQ ID NOs: 87, 97 and 120, and amino acid sequences of HCDR1, HCDR2 and HCDR3 which are respectively as shown in SEQ ID NOs: 12, 34 and 66, and corresponding heavy and light chain variable regions, a heavy chain and light chain, a nucleic acid, a vector, a transformant, a chimeric antigen receptor, cells, a method for preparing an antibody, an antibody conjugate, a pharmaceutical composition, a kit, a pharmaceutical use, a drug administration device, and a method for detecting B7-H7.

Inventions 2-30: claims 1-19 (in part), wherein the subject matter of the inventions is the same as the subject matter of invention 1; however, amino acid sequences of three LCDRs and three HCDRs involved in the inventions are as follows:

SEQ ID NOs: 82, 94 and 112, and SEQ ID NOs: 7, 28 and 58;

SEQ ID NOs: 82, 94 and 112, and SEQ ID NOs: 7, 37 and 58;

SEQ ID NOs: 82, 94 and 122, and SEQ ID NOs: 7, 37 and 58;

SEQ ID NOs: 82, 94 and 123, and SEQ ID NOs: 7, 37 and 58;

SEQ ID NOs: 82, 94 and 112, and SEQ ID NOs: 7, 38 and 58;

SEQ ID NOs: 82, 94 and 122, and SEQ ID NOs: 7, 38 and 58;

SEQ ID NOs: 82, 94 and 123, and SEQ ID NOs: 7, 38 and 58;

SEQ ID NOs: 82, 94 and 122, and SEQ ID NOs: 7, 28 and 58;

SEQ ID NOs: 82, 94 and 123, and SEQ ID NOs: 7, 28 and 58;

SEQ ID NOs: 85, 97 and 116, and SEQ ID NOs: 9, 27 and 63;

SEQ ID NOs: 85, 97 and 116, and SEQ ID NOs: 9, 39 and 63;

SEQ ID NOs: 85, 97 and 124, and SEQ ID NOs: 9, 39 and 63;

SEQ ID NOs: 85, 97 and 125, and SEQ ID NOs: 9, 39 and 63;

SEQ ID NOs: 85, 97 and 116, and SEQ ID NOs: 9, 40 and 63;

SEQ ID NOs: 85, 97 and 124, and SEQ ID NOs: 9, 40 and 63;

SEQ ID NOs: 85, 97 and 125, and SEQ ID NOs: 9, 40 and 63;

Form PCT/ISA/210 (continuation of first sheet) (July 2022)

**INTERNATIONAL SEARCH REPORT**

| International application No. |
|---|
| **PCT/CN2023/072622** |

**Box No. III     Observations where unity of invention is lacking (Continuation of item 3 of first sheet)**

SEQ ID NOs: 85, 97 and 124, and SEQ ID NOs: 9, 27 and 63;

SEQ ID NOs: 85, 97 and 125, and SEQ ID NOs: 9, 27 and 63;

SEQ ID NOs: 81, 94 and 111, and SEQ ID NOs: 7, 27 and 57;

SEQ ID NOs: 82, 94 and 112, and SEQ ID NOs: 7, 28 and 59;

SEQ ID NOs: 83, 95 and 113, and SEQ ID NOs: 8, 29 and 60;

SEQ ID NOs: 82, 96 and 114, and SEQ ID NOs: 9, 27 and 61;

SEQ ID NOs: 84, 95 and 115, and SEQ ID NOs: 10, 29 and 62;

SEQ ID NOs: 83, 98 and 113, and SEQ ID NOs: 8, 30 and 62;

SEQ ID NOs: 82, 94 and 117, and SEQ ID NOs: 9, 31 and 64;

SEQ ID NOs: 86, 95 and 118, and SEQ ID NOs: 8, 32 and 62;

SEQ ID NOs: 83, 99 and 119, and SEQ ID NOs: 11, 33 and 65;

SEQ ID NOs: 88, 100 and 121, and SEQ ID NOs: 13, 35 and 67; and

SEQ ID NOs: 82, 94 and 111, and SEQ ID NOs: 7, 36 and 68.

Groups 1-30 relate to different anti-B7-H7 antibodies. However, the prior art document CN 112930357 A (date of publication: 08 June 2021) discloses an anti-B7-H7 antibody, and it is known in the prior art that apart from a single-domain antibody, identification and binding of other antibodies and antigens generally need to depend on the combined action of three CDRs of a heavy chain and three CDRs of a light chain, and the six CDRs together form a functional unit of an antibody. The different antibodies involved in groups 1-30 do not have six identical CDR sequences therebetween. Therefore, the 30 groups of inventions do not have specific technical features contributing to the prior art, are not so linked as to form a single general inventive concept, and therefore do not comply with PCT Rule 13.1 and 13.2.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **1-19 (in part, relating to SEQ ID NOs: 12, 34, 66, 87, 97, 120, 143, 161, 180 and 202)**

**Remark on Protest**     ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

INTERNATIONAL SEARCH REPORT
Information on patent family members

International application No.

**PCT/CN2023/072622**

| Patent document cited in search report | | | Publication date (day/month/year) | Patent family member(s) | | | Publication date (day/month/year) |
|---|---|---|---|---|---|---|---|
| CN | 106414489 | A | 15 February 2017 | US | 2016096891 | A1 | 07 April 2016 |
| | | | | US | 10316092 | B2 | 11 June 2019 |
| | | | | MX | 2015016111 | A | 26 October 2016 |
| | | | | KR | 20160129698 | A | 09 November 2016 |
| | | | | WO | 2014190356 | A2 | 27 November 2014 |
| | | | | WO | 2014190356 | A3 | 23 April 2015 |
| | | | | EP | 3004158 | A2 | 13 April 2016 |
| | | | | SG | 10201709715 | RA | 28 December 2017 |
| | | | | HK | 1232243 | A1 | 05 January 2018 |
| | | | | CA | 2913312 | A1 | 27 November 2014 |
| | | | | AU | 2014268298 | A1 | 21 January 2016 |
| | | | | AU | 2014268298 | B2 | 17 January 2019 |
| | | | | JP | 2016527187 | A | 08 September 2016 |
| | | | | JP | 6682426 | B2 | 15 April 2020 |
| | | | | BR | 112015029395 | A2 | 19 September 2017 |
| | | | | SG | 11201509618 | QA | 30 December 2015 |
| CN | 112153977 | A | 29 December 2020 | US | 2021115144 | A1 | 22 April 2021 |
| | | | | BR | 112020015999 | A2 | 15 December 2020 |
| | | | | AU | 2019257249 | A1 | 15 October 2020 |
| | | | | JP | 2021520208 | A | 19 August 2021 |
| | | | | RU | 2020136055 | A3 | 06 May 2022 |
| | | | | SG | 11202007898 | XA | 29 October 2020 |
| | | | | EP | 3773658 | A1 | 17 February 2021 |
| | | | | EP | 3773658 | A4 | 27 April 2022 |
| | | | | MX | 2020010094 | A | 15 January 2021 |
| | | | | KR | 20210007959 | A | 20 January 2021 |
| | | | | IL | 276746 | A | 29 October 2020 |
| | | | | WO | 2019204057 | A1 | 24 October 2019 |
| | | | | CA | 3091859 | A1 | 24 October 2019 |
| CN | 112930357 | A | 08 June 2021 | JP | 2021533796 | A | 09 December 2021 |
| | | | | AU | 2019326438 | A1 | 04 March 2021 |
| | | | | US | 2021198366 | A1 | 01 July 2021 |
| | | | | WO | 2020041300 | A1 | 27 February 2020 |
| | | | | CA | 3108905 | A1 | 27 February 2020 |
| | | | | EP | 3850009 | A1 | 21 July 2021 |
| US | 2016002337 | A1 | 07 January 2016 | US | 2019010234 | A1 | 10 January 2019 |
| | | | | US | 11261253 | B2 | 01 March 2022 |
| | | | | US | 2023076027 | A1 | 09 March 2023 |
| | | | | US | 10093737 | B2 | 09 October 2018 |
| | | | | US | 2023002491 | A1 | 05 January 2023 |
| | | | | WO | 2014133728 | A2 | 04 September 2014 |
| | | | | WO | 2014133728 | A3 | 16 October 2014 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210071861

- WO 2014190356 A

**Non-patent literature cited in the description**

- *Nat Commun.*, 2013, vol. 4, 2043
- *Cancer Immunol Res.*, 2019, vol. 7 (6), 939-951
- *Cancer Immunol Res.*, 23 November 2020
- *Clin Cancer Res.*, 15 May 2015, vol. 21 (10), 2359-2366
- *Onko Targets Ther.*, 2018, vol. 11, 1563-1570
- **KABAT et al.** Sequences of Proteins of Immunological Interest. National Institutes of Health, 1991
- *JMol Biol*, 1997, vol. 273, 927-48
- **BIRD et al.** *Science*, 1988, vol. 242, 423-426

- **HUSTON et al.** *Proc. Natl. Acad. Sci. USA*, 1988, vol. 85, 5879-5883
- **BERKOW et al.** The Merck Manual of Medical Information. Merck&Co.Inc., 2000
- **EBADI**. *CRC Desk Reference of Clinical Pharmacology*, 1998
- *J. Biol. Chem*, 1968, vol. 243, 3558
- **HOLLIGER ; HUDSON**. *Nat. Biotechnol.*, 2005, vol. 23, 1126-1136
- Remington's Pharmaceutical Sciences. Mack Publishing Company, 1995